# EUROPEAN PATENT APPLICATION

(11) **EP 2 532 755 A1**
(43) Date of publication of application: **12.12.2012**
(21) Application number: 11305731.9
(22) Date of filing: 10.06.2011
(51) Int. Cl.: C12Q 1/68, G01N 33/50

(54) **Methods and uses based on Slfn2 expression and relating to the identification and profiling of compounds for use in the treatment or prevention of pain**

(71) Applicant: Sanofi-Aventis, 75013 Paris (FR)
(72) Inventor: The designation of the inventor has not yet been filed
(74) Representative: Schneider, Rudolf

(57) **Abstract**

A method of detecting the effect of a compound on a pain-related tissue status or a pain-related disease, uses of Slfn2, uses of a cell overexpressing Slfn2 or of a Slfn2 knock-out cell, methods for determining a pain-modulating effect of a compound, a method for determining or adapting the dosage of a compound for the prevention or treatment of a pain-related tissue status or disease, a method of determining the beneficial or adverse effect of a substance, a kit for detecting the effect of a compound on a pain-related tissue status or disease, a compound able to lower Slfn2 activity for use in the prevention or treatment of a pain-related tissue status or disease and a method of preventing or treating a pain-related tissue status or disease.

## Description

Present invention relates to a method of detecting the effect of a compound on a pain-related tissue status or a pain-related disease, uses of Slfn2, uses of a cell overexpressing Slfn2 or of a Slfn2 knock-out cell, methods for determining a pain-modulating effect of a compound, a method for determining or adapting the dosage of a compound for the prevention or treatment of a pain-related tissue status or disease, a method of determining the beneficial or adverse effect of a substance, a kit for detecting the effect of a compound on a pain-related tissue status or disease, a compound able to lower Slfn2 activity for use in the prevention or treatment of a pain-related tissue status or disease and a method of preventing or treating a pain-related tissue status or disease.

Physical pain is a typical sensory experience that may be described as the unpleasant awareness of a noxious stimulus or bodily harm. Individuals experience pain by various daily hurts and aches, and sometimes through more serious injuries or illnesses. For scientific and clinical purposes, pain is defined by the International Association for the Study of Pain (IASP) as "an unpleasant sensory and emotional experience associated with actual or potential tissue damage, or described in terms of such damage".

Pain of any type is the most common reason for physician consultation in the United States, prompting half of all Americans to seek medical care annually. It is a major symptom in many medical conditions, significantly interfering with a person's quality of life and general functioning. Diagnosis is based on characterizing pain in various ways, according to duration, intensity, type (dull, burning, throbbing or stabbing), source, or location in body. Usually pain stops without treatment or responds to simple measures such as resting or taking an analgesic, and it is then called 'acute' pain. But it may also become intractable and develop into a condition called chronic pain, in which pain is no longer considered a symptom but an illness by itself. In recent years, the study of pain has attracted many different fields such as pharmacology, neurobiology, nursing, dentistry, physiotherapy, and psychology.

Pain is part of the body's defense system, triggering a reflex reaction to retract from a painful stimulus, and helps adjust behavior to increase avoidance of that particular harmful situation in the future.

Medical management of pain has given rise to a distinction between acute pain and chronic pain. Acute pain is 'normal' pain, it is felt when hurting a toe, breaking a bone, having a toothache, or walking after an extensive surgical operation. Chronic pain is a 'pain illness', it is felt day after day, month after month, and seems impossible to heal.

In general, physicians are more comfortable treating acute pain, which usually is caused by soft tissue damage, infection and/or inflammation among other causes. It is usually treated simultaneously with pharmaceuticals, commonly analgesics, or appropriate techniques for removing the cause and for controlling the pain sensation. The failure to treat acute pain properly may lead to chronic pain in some cases.

A series of pharmaceuticals is known for the treatment of pain. However, side-effects and resistance are common problems associated with known analgesics. Accordingly, it is no surprise that a survey of American adults found pain was the most common reason that people use complementary and alternative medicine. In addition, the identification of novel compounds and thus the development of novel pharmaceutical treatments of pain are compromised by the lack of objective and quantifiable parameters and methods for determination of drug action.

### THIS PROVES THAT NEW APPROACHES AND TARGETS FOR PAIN THERAPY ARE STILL NEEDED

A common feature of many clinical syndromes where chronic pain develops is the existence of previous nerve damage, affecting peripheral nerves, the spinal cord or (as in stroke) the brain. The concept of neuropathic pain (i.e. pain arising as a consequence of neuronal damage) has become accepted as the underlying cause of many different chronic pain conditions seen in the clinic. Several animal models of neuropathic pain are known in the art, which mimic many aspects of the clinical condition. These include lesions of the sciatic nerve (constriction or partial section), section of spinal nerves, ischemic lesions of the spinal cord, diabetic neuropathy, etc. Such models have been subjected to detailed study of the anatomical, biochemical and physiological changes that accompany the development of the pain state.

A number of experimental models for neuropathic pain have been developed (Bennett and Xie (1988), Pain 33: 87-107; Seltzer et al. (1990), Pain 43: 205-218; Kim and Chung (1992), Pain 50: 355-363; DeLeo et al. (1994) Pain 56: 9-16 ; Na et al. (1994), Neurosci. Lett. 177: 50- 52). The availability of these different models provides an opportunity to investigate mechanisms of neuropathic pain.

Further animal models for pain are considered in an article of Walker et al. (1999), Molecular Medicine Today 5: 319-321, comparing models for different types of pain, which are acute pain, chronic/inflammatory pain and chronic/neuropathic pain, on the basis of behavioural signs.

Present invention is based on studies of the inventors using three different inbred strains mouse strains differing in their pain sensitivity in a murine "Kim and Chung" model system of chronic neuropathic pain.

One object of the present invention is thus to provide a target for examination and development of a medicament for the treatment and prevention of pain.

Surprisingly, the inventors found that Slfn2 is involved in pain, which, in light of the then existing knowledge of possible functions of Slfn2 that rather hinted to completely different functions (e.g. in cell cycle control, see below) was not to be expected: In a screening assay for the identification of genes involved in pain, three different inbred mouse strains differing in their pain sensitivity were examined. The expression of various genes was correlated with the pain sensitivity of the mouse strains. Among the genes showing the best correlation between pain sensitivity and expression was Slfn2 (see examples). Therefore, Slfn2 is an interesting target for the identification and profiling of compounds concerning their analgesic effects.

The above overview does not necessarily describe all problems solved by present invention.

Accordingly, a first aspect of the invention relates to a method of detecting the effect of a compound on a pain-related tissue status or a pain-related disease, the method comprising the steps of:
a) providing a test system) comprising Slfn2,
b) contacting the test system with a test compound, and
c) determining the effect of the test compound on the test system,
wherein the test compound is identified as being active in modulating a pain-related tissue status or disease, if a significant effect of the test compound on the test system relative to a control is detected.

In a second aspect, the invention relates to the use of Slfn2 for detecting the effect of a compound on a pain-related tissue status or a pain-related disease.

In a third aspect, the invention relates to the use of Slfn2 for determining the dosage of a compound for the prevention or treatment of a pain-related tissue status or disease.

In a fourth aspect, the invention relates to the use of a cell overexpressing Slfn2 or of a non-human transgenic animal heterologously expressing Slfn2 for determining the effect of a compound on a pain-related tissue status or disease.

In a fifth aspect, the invention relates to the use of a cell overexpressing Slfn2 or of a non-human transgenic animal heterologously expressing Slfn2 as a model system for enhanced pain sensitivity.

In a sixth aspect, the invention relates to the use of a Slfn2 knock-out cell or a non-human Slfn2 knock-out animal as a model system for lowered pain sensitivity.

In a seventh aspect, the invention relates to the use of a method for determining a pain-modulating effect of a compound comprising
(i) administering a test compound to a subject
(ii) detecting whether said compound increases or decreases the Slfn2 expression level in said subject in comparison to a control animal not having been administered said compound,
wherein a decrease in Slfn2 expression level after treatment with the compound in comparison to the level in the control animal is indicative of a pain reducing effect of the compound and an increase is indicative of pain as (adverse) effect of the compound.

In a eighth aspect, the invention relates to the a method of for determining a pain-modulating effect of a compound comprising
(i) determining the expression level of SIfn2 in a subject
(ii) applying a compound to the subject, and
(iii) determining the expression level of Slfn2 after application of the compound, wherein
the compound is displaying pain as adverse effect, if the expression level of Slfn2 is increased in one or more of the samples obtained after application of the compound in comparison to the level obtained before application of the compound and a decrease in Slfn2 expression level after treatment with the compound in comparison with the level before treatment is indicative of a pain reducing effect of the compound.

In a ninth aspect, the invention relates to a method for determining the dosage of a compound for the prevention or treatment of a pain-related tissue status or disease, comprising the steps of
(i) determining the level of Slfn2 in an individual, and optionally determining the level of Slfn2 in a reference, and
(ii) determining the dosage of a pharmaceutical depending on the level of Slfn2 in the first individual optionally in comparison with that of the reference.
In a tenth aspect, the invention relates to a method for adapting the dosage of a compound for the prevention or treatment of a pain-related tissue status or disease, comprising the steps of
(i) determining the level of Slfn2 in an individual
(ii) determining the level of Slfn2 in one or more,
(iii) examining the tested sample as to whether the level of Slfn2 present in said sample is different from the level in the one or more references, and (d) adapting the dosage of a pharmaceutical depending on whether the level of Slfn2 in the tested sample is different from the level in the one or more reference samples.

In an eleventh aspect, the invention relates to a method of determining the beneficial and/or adverse effects of a substance on a pain-related tissue status or disease, comprising the steps of
(i) determining the level of Slfn2 in a test sample,
(ii) determining the level of Slfn2 in one or more reference samples, and
(iii) examining the tested sample as to whether the level of Slfn2 present in said sample is different from the level in the one or more reference samples,
wherein the test sample was exposed differently to said substance than the one or more reference samples.

In a twelfth aspect, the invention relates to the use of Slfn2 as a target molecule for the discovery of a compound preventing or reducing a pain related tissue status or disease.

In a thirteenth aspect, the invention relates to a kit for detecting the effect of a compound on a pain-related tissue status or a pain-related disease comprising
a. a means for detecting Slfn2 and optionally
b. a data carrier comprising instructions for one of the above methods and optionally
c. a container.

In a fourteenth aspect, the invention relates to a compound able to lower Slfn2 activity and/or expression for the prevention or treatment of a pain-related tissue status or disease.

In a fifteenth aspect, the invention relates to a method of preventing or treating a pain-related tissue status or disease, wherein a therapeutically effective amount of the compound according to the fifteenth aspect is administered to an individual at risk of developing a pain-related tissue status or disease or suffering from a pain-related tissue status or disease.

This summary of the invention does not necessarily describe all features of the present invention. Other embodiments will become apparent from a review of the ensuing detailed description.

### DEFINITIONS AND METHODS:

In the following, definitions, methods and technologies applicable in the context of present invention and its different aspects and embodiments are listed.

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents (for example: patents, patent applications, scientific publications, manufacturer's specifications, instructions, GenBank Accession Number sequence submissions etc.) are cited throughout the text of this specification. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

Sequences: All sequences referred to herein are disclosed in the attached sequence listing that, with its whole content and disclosure, is a part of this specification.

The term "about" when used in connection with a numerical value is meant to encompass numerical values within a range having a lower limit that is 5% smaller than the indicated numerical value and having an upper limit that is 5% larger than the indicated numerical value.

The term "Schlafen 2" or Slfn2, as used herein, refers to murine Slfn2 comprising the nucleic acid sequence shown in SEQ ID NO: 1 and the amino acid sequence shown in SEQ ID NO: 2 or to a functional variant thereof as defined below. As will be detailed below, the term "functional variant of Slfn2" encompasses naturally occurring variants, e.g. the below listed homologs, especially the human and/or rat protein and/or nucleic acid as identified below.

Cloning and characterization of Slfn: Identification and cloning of the Slfn family of proteins is described by Schwarz, Katayama and Hedrick, 1998). Schlafen 2 (in the following referred to as Slfn2) is a member of the (Schlafen) Slfn gene family comprising 10 family members in mouse and 7 family members in human (Schwarz et al., 1998; Berger et al., 2010). Slfn2 is sometimes also referred to as schlafen family member 12 like (see e.g. accession number NP_035538 in the NCBI database that corresponds to the murine Slfn2 protein and coding sequence). Slfn 2 has strongest homology to human SLFN12L, rat slfn 2, orang utan SLFN12L and chimpanzee SLFN12L (see below). The gene of murine Slfn 2 is located on chromosome 4 (Ferguson et al., 2005).

The reference sequence of the murine Slfn2 coding sequence is known and publicly available under the accession number: AF099973 from the NCBI (National Centre for Biotechnology Information; National Library of Medicine 38A, Bethesda, MD20894, USA; www.ncbi.nih.gov). Version AF099973.1 of the coding sequence (SEQ ID NO: 1, positions 1 to 1684) is given in the following:

1 gctttaatgc agcaaggaac aaagaactca catgggctta gaggtgaaca cacaaccagg

61 gctgcagtta gaactcttcc aagtgccaaa gcccaccggc gaagacatct gctggccagg
121 acttgttgca gacagagggt ctttctcagg tgacactagc ctgctttacc aagactgctg
181 acatgggtac tagacttgag gcaactgagc aaagcaacca cagaactcag aggaatgaca
241 ttcagctgga aaatgcaaag gctgctggga aaatgggcat cagtgttgat ctggaagcca
301 aatatgctaa gctgggtcta aatctcggag caatcacttt tggagagaag gataggaaga
361 aaatgaagaa ttctcacctc agaaaacagg agaatgcaaa catctctcta gctgtatgtg
421 ctctcctgaa ttcgggaggt ggagcaatca aggttaaaat tgaaaatgaa aattatagtc
481 tcactaggga tggcctggga ctagatttgg aagcctctct ttgtaaatgt ctgccctttg
541 tccagtggca cctggacttc acggagagcg aaggctacat ttatatctac gtgaaatcgt
601 ggagccaaga aatctttggg ctgcctattg gcaccctaag aaccaatttg tatgtaagga
661 gcatgtcatc ttctgtacaa gtgagcgccg ctgctgccct ggaatttctc caggacctgg
721 aggaaactgg agggagaccc tgtgtcagac cagagttgcc tgcaagcata gccttccctg
781 aagtggaagg agaatggcac ctggaggatt tggctgctgc attgtttaac aggacagaat
841 ttcagtacga ggaaactttc ccctttacca gatccagata tgttgaagtt acattgcttt
901 cagcgaaacg cctgcgaaaa cgcatcaaag agctcctccc tcaaactgtt tctgcttttg
961 caaacacgga tgggggattt ttgttcattg gtttggatgg caaaacccag caaattattg
1021 gttttgaagc agagaagagc gatctcgtgc ttctagagag tgaaatagaa aagcacatcc
1081 ggcagctgcc tgtcactcac ttctgtgagg agaaggagaa gatcaagtac acgtgcaaat
1141 tcatcgaagt gcacaaatcc ggagctgtgt gtgcatatgt gtgtgcgctc agagtggaga
1201 gattctgctg tgcagtattc gctgcagagc ccgagtcctg gcatgtggaa ggcggctgtg
1261 tgaagaggtt taccacagag gaatgggtga aactccagat gaatgcccca tcaggttgaa
1321 gggggattaa taatctgtat gagggcggca gagatggctc agtggctaag agcactgact
1381 gctcttccag agatctgagc tgtaaccccc cccccccagc ctgaaacctg gttgctcagg
1441 tttcactgtt agcaagaaga gagcatggga gagggggagg gagcctgccg ccctatcgtt
1501 catcctaccc tttgtccacc cgagactaga ggggtgtgtc cgttccacgc agacaaaggg
1561 gcccagaggg tctgtggcag tggtcacctg aaactggact ccaggatgat ttggcgggaa
1621 tgggcccctt ccccctcctt cataaaattg agctttgctc agaaaaaaaa aaaaaaaaaa
1681 aaaa (SEQ ID NO:1)

It should be noted that the coding sequences comprised herein are indicated as cDNA sequences corresponding to the respective mRNA sequences.

The above coding sequence (SEQ ID NO: 1) encodes a 378 amino acid protein the reference sequence of which is publicly available at the NCBI database under accession number AAC83826. The translated region starts at position 183 of SEQ ID NO: 1 and ends at position 1319. Version AAC83826.1 (SEQ ID NO: 2) is given in the following:

1 mgtrleateq snhrtqrndi qlenakaagk mgisvdleak yaklglnlga
51 Itfgekdrkk mknshlrkqe nanislavca llnsgggaik vkienenysl
101 trdglgldle aslckclpfv qwhldftese gyiyiyvksw sqeifglpig
151 tlrtnlyvrs msssvqvsaa aaleflqdle etggrpcvrp elpasiafpe
201 vegewhledl aaalfnrtef qyeetfpftr sryvevtlls akrlrkrike
251 llpqtvsafa ntdggflfig ldgktqqiig feaeksdlvl leseiekhir
301 qlpvthfcee kekikytckf ievhksgavc ayvcalrver fccavfaaep
351 eswhveggcv krftteewvk lqmnapsg (SEQ ID NO:2)

The divergent AAA domain (from position 231 to position 355), that is common to Slfn-family proteins is shown underlined. Position 135, the mutation of which gives rise to the "electra" phenotype in mice (see below) is marked boldand underlined.

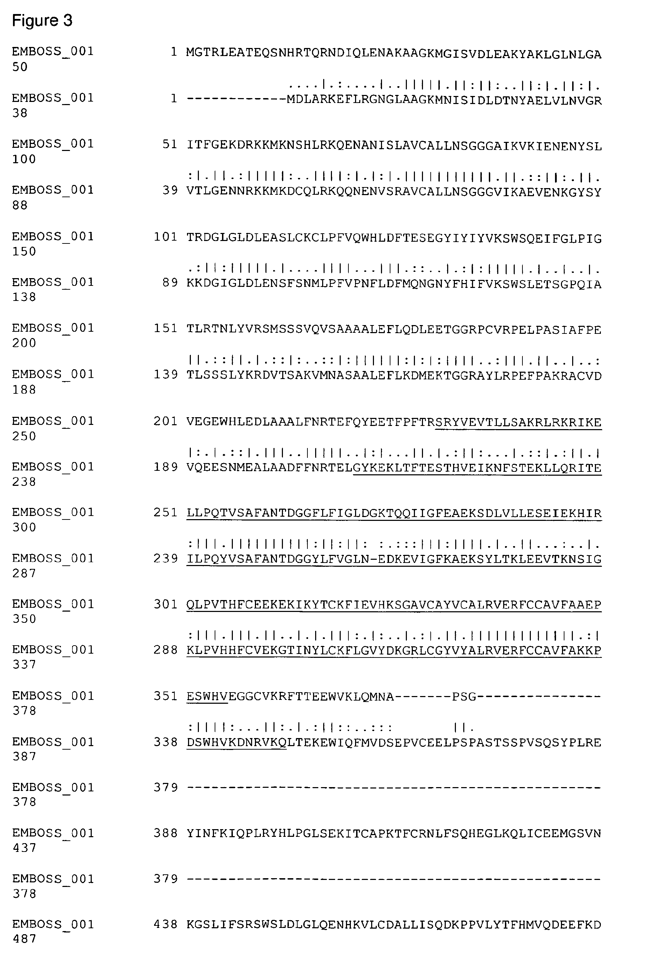

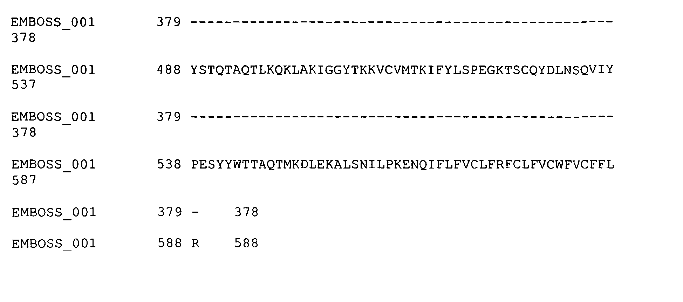

The promoter region of murine Slfn2 has been cloned and analysed (see Wern-Joo et al., 2007). According to Wern-Joo et al., 2007 (the content of which is included herein in full as reference, see especially also page 3278, e.g. figure 3 of that article), the transcription start site is located 12 nucleotides upstream of the first nucleotide according to SEQ ID NO: 1. In the sequence according to SEQ ID NO: 3 as given below, the nucleotides according to Wern-Joo (capital letters) are attached to the first part of SEQ ID NO: 1 (small letters):

+1
AGTCTGTGCA ATgctttaat gcagcaagga acaaagaact cacatgggct tagaggtgaa
cacacaacca gggctgcagt tagaactctt ccaagtgcca aagcccaccg gcgaagacat
ctgctggcca ggacttgttg cagacagagg gtctttctca ggtgacacta gcctgcttta
ccaagactgc tgacatgggt ac…[see SEQ ID NO:1]…aaaa (pos. 1684)
(SEQ ID NO:3)

The translation start (methionin/atg) is underlined and typed bold.

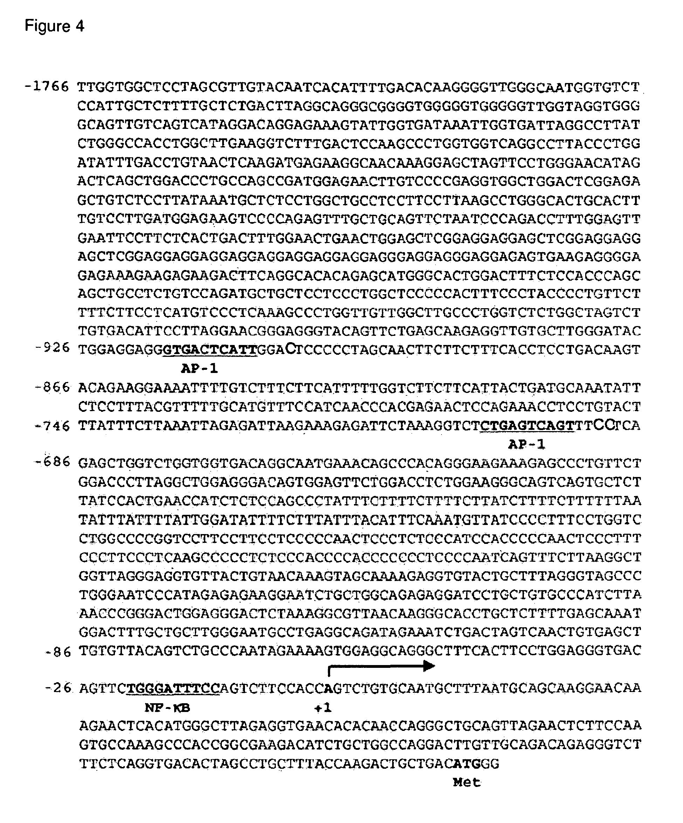

The promoter region of murine Slfn2 has been cloned and analysed (see Wern-Joo et al., 2007). The promoter of murine Slfn2 plus some downstream sequences according to Wern-Joo et al., 2004 is given in figure 4 (the sequence according to figure 4 includes some downstream positions incl. the translation start - Met). The upstream regulatory region, i.e. the promoter from position -1766 to +1 (+1 is underlined) is given in the following as SEQ ID NO: 4:

TTGGTGGCTCCTAGCGTTGTACAATCACATTTTGACACAAGGGGTTGGGCAATGGTGTCT
CCATTGCTCTTTTGCTCTGACTTAGGCAGGGCGGGGTGGGGGTGGGGGTTGGTAGGTGGG
GCAGTTGTCAGTCATAGGACAGGAGAAAGTATTGGTGATAAATTGGTGATTAGGCCTTAT
CTGGGCCACCTGGCTTGAAGGTCTTTGACTCCAAGCCCTGGTGGTCAGGCCTTACCCTGG
ATATTTGACCTGTAACTCAAGATGAGAAGGCAACAAAGGAGCTAGTTCCTGGGAACATAG
ACTCAGCTGGACCCTGCCAGCCGATGGAGAACTTGTCCCCGAGGTGGCTGGACTCGGAGA
GCTGTCTCCTTATAAATGCTCTCCTGGCTGCCTCCTTCCTTAAGCCTGGGCACTGCACTT
TGTCCTTGATGGAGAAGTCCCCAGAGTTTGCTGCAGTTCTAATCCCAGACCTTTGGAGTT
GAATTCCTTCTCACTGACTTTGGAACTGAACTGGAGCTCGGAGGAGGAGCTCGGAGGAGG
AGCTCGGAGGAGGAGGAGGAGGAGGAGGAGGGAGGAGGGAGGAGAGTGAAGAGGGGA
GAGAAAGAAGAGAAGACTTCAGGCACACAGAGCATGGGCACTGGACTTTCTCCACCCAGC
AGCTGCCTCTGTCCAGATGCTGCTCCTCCCTGGCTCCCCCACTTTCCCTACCCCTGTTCT
TTTCTTCCTCATGTCCCTCAAAGCCCTGGTTGTTGGCTTGCCCTGGTCTCTGGCTAGTCT
TGTGACATTCCTTAGGAACGGGAGGGTACAGTTCTGAGCAAGAGGTTGTGCTTGGGATAC
TGGAGGAGGGTGACTCATTGGACTCCCCCTAGCAACTTCTTCTTTCACCTCCTGACAAGT
ACAGAAGGAAAATTTGTCTTTCTTCATTTTGGTCTTCTTCATTACTGATGCAAATATT
CTCCTTTACGTTTTTGCATGTTTCCATCAACCCACGAGAACTCCAGAAACCTCCTGTACT
TTATTTCTTAAATTAGAGATTAAGAAAGAGATTCTAAAGGTCTCTGAGTCAGTTTCCTCA
GAGCTGGTCTGGTGGTGACAGGCAATGAAACAGCCCACAGGGAAGAAAGAGCCCTGTTCT
GGACCCTTAGGCTGGAGGGACAGTGGAGTTCTGGACCTCTGGAAGGGCAGTCAGTGCTCT
TATCCACTGAACCATCTCTCCAGCCCTATTTCTTTTCTTTTCTTATCTTTTCTTTTTTAA
TATTTATTTTATTGGATATTTTCTTTATTTACATTTCAAATGTTATCCCCTTTCCTGGTC
CTGGCCCCGGTCCTTCCTTCCTCCCCCAACTCCCTCTCCCATCCACCCCCAACTCCCTTT
CCCTTCCCTCAAGCCCCCTCTCCCACCCCACCCCCCCTCCCCAATCAGTTTCTTAAGGCT
GGTTAGGGAGGTGTTACTGTAACAAAGTAGCAAAAGAGGTGTACTGCTTTAGGGTAGCCC
TGGGAATCCCATAGAGAGAAGGAATCTGCTGGCAGAGAGGATCCTGCTGTGCCCATCTTA
AACCCGGGACTGGAGGGACTCTAAAGGCGTTAACAAGGGCACCTGCTCTTTTGAGCAAAT
GGACTTTGCTGCTTGGGAATGCCTGAGGCAGATAGAAATCTGACTAGTCAACTGTGAGCT
TGTGTTACAGTCTGCCCAATAGAAAAGTGGAGGCAGGGCTTTCACTTCCTGGAGGGTGAC
AGTTCTGGGATTTCCAGTCTTCCACCA

(SEQ ID NO: 4)

The Slfn2 sequence containing the upstream regulatory sequence according to Wern-Joo et al., 2004 (capital letters) and the complete coding sequence according to SEQ ID NO: 1 (small letters) comprises positions -1766 to +1696 and is given in the following:

TTGGTGGCTCCTAGCGTTGTACAATCACATTTTGACACAAGGGGTTGGGCAATGGTGTCT
CCATTGCTCTTTTGCTCTGACTTAGGCAGGGCGGGGTGGGGGTGGGGGTTGGTAGGTGGG
GCAGTTGTCAGTCATAGGACAGGAGAAAGTATTGGTGATAAATTGGTGATTAGGCCTTAT
CTGGGCCACCTGGCTTGAAGGTCTTTGACTCCAAGCCCTGGTGGTCAGGCCTTACCCTGG
ATATTTGACCTGTAACTCAAGATGAGAAGGCAACAAAGGAGCTAGTTCCTGGGAACATAG
ACTCAGCTGGACCCTGCCAGCCGATGGAGAACTTGTCCCCGAGGTGGCTGGACTCGGAGA
GCTGTCTCCTTATAAATGCTCTCCTGGCTGCCTCCTTCCTTAAGCCTGGGCACTGCACTT
TGTCCTTGATGGAGAAGTCCCCAGAGTTTGCTGCAGTTCTAATCCCAGACCTTTGGAGTT
GAATTCCTTCTCACTGACTTTGGAACTGAACTGGAGCTCGGAGGAGGAGCTCGGAGGAGG
AGCTCGGAGGAGGAGGAGGAGGAGGAGGAGGAGGGAGGAGGGAGGAGAGTGAAGAGGGGA
GAGAAAGAAGAGAAGACTTCAGGCACACAGAGCATGGGCACTGGACTTTCTCCACCCAGC
AGCTGCCTCTGTCCAGATGCTGCTCCTCCCTGGCTCCCCCACTTTCCCTACCCCTGTTCT
TTTCTTCCTCATGTCCCTCAAAGCCCTGGTTGTTGGCTTGCCCTGGTCTCTGGCTAGTCT
TGTGACATTCCTTAGGAACGGGAGGGTACAGTTCTGAGCAAGAGGTTGTGCTTGGGATAC
TGGAGGAGGGTGACTCATTGGACTCCCCCTAGCAACTTCTTCTTTCACCTCCTGACAAGT
ACAGAAGGAAAATTTGTCTTTCTTCATTTTTGGTCTTCTTCATTACTGATGCAAATATT
CTCCTTTACGTTTTTGCATGTTTCCATCAACCCACGAGAACTCCAGAAACCTCCTGTACT
TTATTTCTTAAATTAGAGATTAAGAAAGAGATTCTAAAGGTCTCTGAGTCAGTTTCCTCA
GAGCTGGTCTGGTGGTGACAGGCAATGAAACAGCCCACAGGGAAGAAAGAGCCCTGTTCT
GGACCCTTAGGCTGGAGGGACAGTGGAGTTCTGGACCTCTGGAAGGGCAGTCAGTGCTCT
TATCCACTGAACCATCTCTCCAGCCCTATTTCTTTTCTTTTCTTATCTTTTCTTTTTTAA
TATTTATTTTATTGGATATTTTCTTTATTTACATTTCAAATGTTATCCCCTTTCCTGGTC
CTGGCCCCGGTCCTTCCTTCCTCCCCAACTCCCTCTCCCATCCACCCCCAACTCCCTTT
CCCTTCCCTCAAGCCCCCTCTCCCACCCCACCCCCCCTCCCCAATCAGTTTCTTAAGGCT
GGTTAGGGAGGTGTTACTGTAACAAAGTAGCAAAAGAGGTGTACTGCTTTAGGGTAGCCC
TGGGAATCCCATAGAGAGAAGGAATCTGCTGGCAGAGAGGATCCTGCTGTGCCCATCTTA
AACCCGGGACTGGAGGGACTCTAAAGGCGTTAACAAGGGCACCTGCTCTTTTGAGCAAAT
GGACTTTGCTGCTTGGGAATGCCTGAGGCAGATAGAAATCTGACTAGTCAACTGTGAGCT
TGTGTTACAGTCTGCCCAATAGAAAAGTGGAGGCAGGGCTTTCACTTCCTGGAGGGTGAC
AGTTCTGGGATTTCCAGTCTTCCACCAGTCTGTGCAATgctttaatgcagcaaggaacaa
agaactcacatgggcttagaggtgaacacacaaccagggctgcagttagaactcttccaa
gtgccaaagcccaccggcgaagacatctgctggccaggacttgttgcagacagagggtct
ttctcagtgacactagcctgctttaccaagactgctgacatgggtactagacttgaggca
actgagcaaagcaaccacagaactcagaggaatgacattcagctggaaaatgcaaaggct
gctgggaaaatgggcatcagtgttgatctggaagccaaatatgctaagctgggtctaaat
ctcggagcaatcacttttggagagaaggataggaagaaaatgaagaattctcacctcaga
aaacaggagaatgcaaacatctctctagctgtatgtgctctcctgaattcgggaggtgga
gcaatcaaggttaaaattgaaaatgaaaattatagtctcactagggatggcctgggacta
gatttggaagcctctctttgtaaatgtctgccctttgtccagtggcacctggacttcacg
gagagcgaaggctacatttatatctacgtgaaatcgtggagccaagaaatctttgggctg
cctattggcaccctaagaaccaatttgtatgtaaggagcatgtcatcttctgtacaagtg
agcgccgctgctgccctggaatttctccaggacctggaggaaactggaggagaccctgt
gtcagaccagagttgcctgcaagcatagccttccctgaagtggaaggagaatggcacctg
gaggatttggctgctgcattgtttaacaggacagaatttcagtacgaggaaactttcccc
tttaccagatccagatatgttgaagttacattgctttcagcgaaacgcctgcgaaaacgc
atcaaagagctcctccctcaaactgtttctgcttttgcaaacacggatgggggatttttg

ttcattggtttggatggcaaaacccagcaaattattggttttgaagcagagaagagcgat
ctcgtgcttctagagagtgaaatagaaaagcacatccggcagctgcctgtcactcacttc
tgtgaggagaaggagaagatcaagtacacgtgcaaattcatcgaagtgcacaaatccgga
gctgtgtgtgcatatgtgtgtgcgctcagagtggagagattctgctgtgcagtattcgct
gcagagcccgagtcctggcatgtggaaggcggctgtgtgaagaggtttaccacagaggaa
tgggtgaaactccagatgaatgccccatcaggttgaaggggattaataatctgtatgag
ggcggcagagatggctcagtggctaagagcactgactgctcttccagagatctgagctgt
aacccccccccccccagcctgaaacctggttgctcaggtttcactgttagcaagaagagag
catgggagagggggaggagcctgccgccctatcgttcatcctaccctttgtccacccga
gactagaggggtgtgtccgttccacgcagacaaagggcccagagggtctgtggcagtgg
tcacctgaaactggactccaggatgatttggcgggaatgggccccttccccctccttcat
aaaattgagctttgctcagaaaaaaaaaaaaaaaaaaaa

(SEQ ID NO: 5)

The upstream sequence comprises two AP-1 sites (-917 to -908 and -703 to -695) and one NF-kappa B binding site (-21 to -12). According to Wern-Joo et al., 2007 both AP-1 sites are important for maximal promoter activity upon LPS or CpG-DNA stimulation (8-9 fold induction), but activity is also exhibited by a fragment comprising only the second AP-1 site (-703 to -695) and the NF kappa B site (2 fold induction). Possible promoter fragments able to drive expression of downstream coding regions and the construction of fragments, point mutants and various luciferase constructs are disclosed in Wern-Joo et al., 2007. Examples of Slfn-2 promoter fragments usable for cloning of reporter-gene constructs are nucleic acids comprising or having the following positions according to SEQ ID NO:5: from -1766/-1265 to +1/+50/ +100/+150/+198.

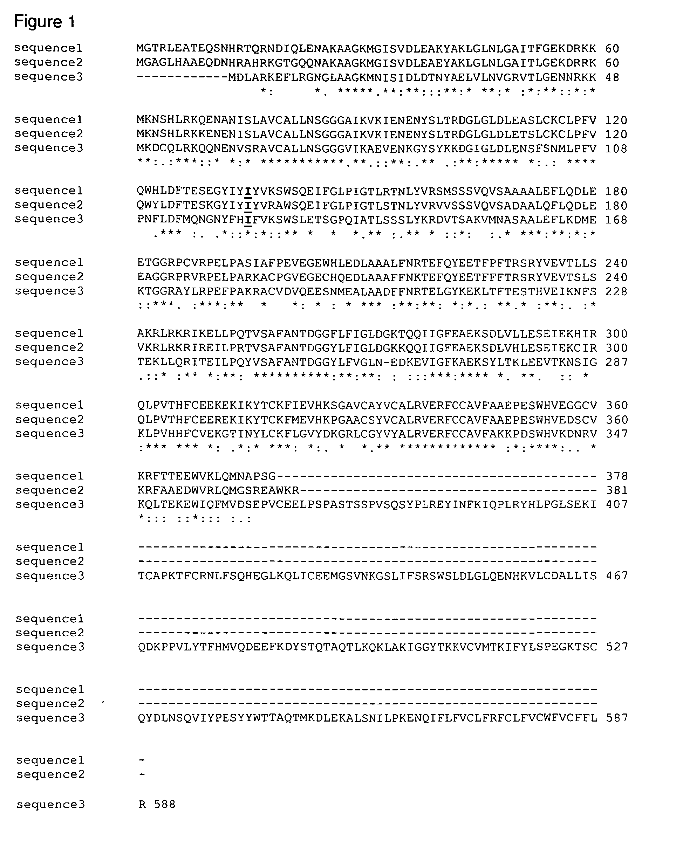

Slfn2 has been reported to be implicated in the regulation of quiescence in T-cells and monocytes (Berger et al., 2010) and in interferon alpha-induced growth arrest in fibroblasts (Katsoulidis et al., 2009). A point mutation on position 135 of the 278 amino acid protein caused by a thymidine-to-adenosine transversion in the Slfn2 gene and leading to an isoleucine to asparagine substitution leads to a loss-of-function mutation causing lymphoid and myeloid immunodeficiency due to loss of quiescence of T-cells and monocytes in mice, and causes the "electra" phenotype, leaving natural killer cells or B cells unaffected (Berger et al., 2010). Markedly NFAT and NF-kB and Erk and Akt appear to be normally activated in electra mice, the mitogen-activated protein kinases p38 and Jnk are constitutively phosphorylated. Moreover, expression levels of the anti-apoptotic Bcl-2 is reported to be lower in T-cells derived from electra mice, wherein transgenic expression of Bcl-2 rescues CD4+ and CD8+ T-cells derived from electra mice from apoptotic cell death (all Berger et al., 2010, see also Horton and Powell, 2010). Thus, the reported mutation of Slfn2 appears to affect T-cell antigen receptor-induced signaling pathways, wherein other pathways are not affected. As can be gained from sequence comparison of murine, rat and human proteins (see Figure 1) using ClustalW, the isoleucine at position 135 of murine Slfn2 according to SEQ ID NO:2 (and corresponding to position 135 in rat Slfn2 according to SEQ ID nO:7 and position 123 in hsSLFN12L according to SEQ ID NO:9) is conserved among murine Slfn2, rat Slfn2 and homo sapiens SLFN12L hinting to an equal importance of said amino acid or surrounding protein region within the respective rat and homo sapiens proteins.

The following species variants are exemplified:

| **Organism** | **Murine protein Identity* (%)** | **NCBI accession numbers** | **Symbol** | **Chromosome Location** |
|---|---|---|---|---|
| Mus | 100 % | AAC83826.1 | SLFN2 | 11 (50.3 cM) |
| musculus | | AF099973.1 | | 82878614-82884180(+) |
| | | | | *^{NCBI Mouse Build 37}* |
| Rattus | 83% | NM_001107031.1 | SLFN2 | 10(126) |
| norvegicus | | NP_001100501.1 | | |
| | | XP 220779.4 | | |
| Human | 50% | NP_001182719.1 | SLFN12L | 17 (q12) |
| | | NM_001195790.1 | | |

| | | | | |
|---|---|---|---|---|
| *Percentual identity has been determined using the EMBL ClustalW program. | | | | |

The rat homolog of Slfn2 is schlafen family member 12-like (Slfn12L). The protein consists of 381 amino acids and the protein sequence is available under accession number NP_001100501.1 (SEQ ID NO: 7) and is encoded by the coding sequence NM_001107031.1 (from positions 193-1338).

The coding sequence according to NM_001107031.1 is given in the following (SEQ ID NO: 6):

1 cttcaatgca gcaaagaaca gagaactcaa gtgagcttag aggtgagctc acagccaggg

61 ctgcagatag aactctccca agtgccaaca accagcctcg aagacgtccc cggccaggac
121 ttgttgcaga caacactagt tgtctttctc aagtgacact agcctgcctc accaaaggag
181 ctgactgctg gcatgggcgc tggacttcat gcagctgagc aagacaacca cagagctcac
241 aggaagggca ctgggcagca aaatgcaaaa gctgctggga aaatgggcat cagcgttgac
301 ctggaagctg agtatgctaa gctgggtcta aatctaggag caatcactct tggagagaag
361 gataggagga aaatgaagaa ctctcacctc aggaaaaagg agaatgagaa catctctctg
421 gctgtgtgtg ctctcctgaa ttcaggaggt ggagcaatca aggttaaaat tgaaaatgaa
481 aattatagtc tcactaggga tggcctggga ctagatttgg aaacctctct ttgtaaatgt
541 ctcccgtttg tccagtggta cctggacttc actgagagca aaggctacat ttatatctac
601 gtgagagcat ggagtcagga aatctttggg ctgccgattg gcaccctgag caccaatttg
661 tacgtaaggg tcgtttcatc ctccgtacaa gtgagcgctg atgctgcgct acaatttctc
721 caggacctgg aggaagctgg agggagaccc cgtgtcagac cagagttgcc tgcaaggaaa
781 gcctgccctg gagtggaagg agaatgtcac caggaggatt tggctgctgc atttttaac
841 aagaccgaat ttcagtatga ggaaactttc ttctttacca ggtccaggta tgttgaagtt
901 acatcacttt cagtgaaacg cctgcgaaaa cgcatcagag agatcctccc tcgaactgtt
961 tctgcattcg caaacacgga tgggggatat ttgttcattg gcttggatgg gaaaaaacag
1021 caaataattg gctttgaagc agagaagagc gaccttgtgc atctagagag tgaaatagaa
1081 aagtgcatcc gacagctgcc tgtcactcac ttctgtgagg agagggagaa gatcaagtac
1141 acgtgcaaat tcatggaagt gcacaaaccc ggagccgcgt gttcatacgt gtgtgcgctc
1201 cgagtggaga gattctgctg tgcagtgttc gctgcagagc ccgaatcctg gcacgtggaa
1261 gacagctgtg tgaagaggtt tgccgcagag gactgggtta ggctccagat gggttccagg
1321 gaagcttgga aaaggtgact aataatcagt tagtgaaaaa taaagatttt taagttcccc
1381 acttgaccct gctgtgagga caagtgcacc tcccaaaaga atagcatcac atgttttggt
1441 gaggtagcct ggctatagcc accacaaacc atctggcctc gataaggcct gacacaacag
1501 ttctgaggaa aacaagaact agggtcagct tgccccagaa gcagggtggc ctagagttga
1561 actgaggcca tgtagttttc cctataaatc ccttccccta attgggctcg ggttgactc
1621 ctctgtctcc tgtgtgagat acgtgtcatc cccagagctc tggcttctca aatatactta
1681 ttatatcaag accggtttct cgtgagttct tgggggttgc gtcatcttga gatttgagtg
1741 ggggtcttcc ccaccttggt ggtctttcat tacaaccatc tgtaatgata tctgatgccc
1801 tcttctaact attttccagc atccacatgg tgattcacaa ccacctagaa ccccagttta
1861 tttatttttt ttggctgttt tttttttttt tttggtttta catggagtgg tttaatgaga
1921 gaagaagggt agaagaggag aggaacaaag gaggctggcc atgggcacgt gggggaagg
1981 ggggatgggg agagaaggga cagggacaaa gaggacacca gaagaggaga gcaagagaga

2041 ggagtaagag caaactgatg agcttccagg gactaagc
(SEQ ID NO:6)

The protein sequence of rat Slfn2 according to NP_001100501.1 (SEQ ID NO:7) is given in the following:

1 mgaglhaaeq dnhrahrkgt gqqnakaagk mgisvdleae yaklglnlga itlgekdrrk

61 mknshlrkke nenislavca llnsgggaik vkienenysl trdglgldle tslckclpfv

121 qwyldftesk gyiyiyvraw sqeifglpig tlstnlyvrv vsssvqvsad aalqflqdle

181 eaggrprvrp elparkacpg vegechqedl aaaffnktef qyeetffftr sryvevtsls

241 vkrlrkrire ilprtvsafa ntdggylfig ldgkkqqiig feaeksdlvh leseiekcir

301 qlpvthfcee rekikytckf mevhkpgaac syvcalrver fccavfaaep eswhvedscv

361 krfaaedwvr lqmgsreawk r

(SEQ ID NO:7)

The divergent AAA domain spans positions 231 to 361 of the rat Slfn2 protein.

The Human homolog of SIfn2 is schlafen family member 12-like (SLFN12L). The coding sequence can be retrieved from the NCBI database under accession number NM_001195790.1 (SEQ ID NO: 8), coding for a 588 amino acid protein with sequence according to NCBI accession number NP_001182719.1 (SEQ ID NO:9):

Human SLFN12L coding sequence according to NM_001195790.1 (SEQ ID NO:8):

1 atggacctcg ccagaaaaga atttctgcgt ggaaatggct tagctgctgg gaaaatgaac

61 atcagtattg atttagacac aaactatgct gagctggttc taaatgtggg aagagtcact

121 cttggagaga acaatagaaa aaaaatgaag gattgtcaac tgagaaaaca gcagaatgaa

181 aatgtctcac gagctgtgtg tgctctgctg aattctggag ggggagtgat caaggctgaa

241 gttgagaata aaggctatag ttataaaaaa gatggaatag ggctagattt ggaaaattct

301 tttagtaaca tgctgccatt tgttcctaat ttcctggact tcatgcagaa tggtaactac

361 tttcacattt ttgtgaaatc atggagcttg gaaacctctg gtccgcagat tgccacgttg

421 agctccagtt tgtacaagag agatgtaacg tctgcaaaag tcatgaatgc ttctgctgca

481 ctggagttcc tcaaagacat ggaaaaaact ggagggagag catatttaag accagaattc

541 cctgcaaaaa gggcctgtgt tgatgtacaa gaagaaagta acatggaagc cttggctgct

601 gattttttta acagaacaga acttggttat aaagaaaaat tgacctttac tgaatccaca

661 cacgttgaaa taaaaaactt ctcgactgaa aagttgttac aacgaattac agagattctc

721 cctcaatatg tttctgcatt tgcaaatact gatggaggat atttattcgt tggtctaaat

781 gaagataaag aagtaattgg ctttaaagca gagaagagtt atcttactaa gttagaagaa

841 gtaacaaaaa attccattgg gaaactgcct gtgcatcact tctgtgtgga gaaggggacg

901 ataaattact tatgcaaatt ccttggagta tatgataaag gaaggctttg tggatatgtg

961 tatgcactca gagtggaacg cttctgctgt gcagtgtttg ctaaaaagcc tgattcctgg

1021 cacgtgaaag ataacagagt taagcagttg accgagaagg aatggatcca gttcatggtg

1081 gattcagaac cagtatgtga ggaactgccc tctccagcaa gtacatcatc acctgtctcc

1141 cagagttatc ctcttcgtga atatattaac ttcaaaattc agccactgag atatcacctt

1201 ccagggctat cagaaaagat aacttgtgct ccaaaaacct tctgcagaaa tctgttctca

1261 caacatgaag gacttaagca attaatatgt gaagaaatgg gctctgtcaa taagggctca

1321 ctgatcttct ctaggagctg gtctttggat ctgggcttgc aagagaacca caaagtcctc

1381 tgtgatgctc ttctgatttc ccaggacaag cctccagtcc tatacacctt ccacatggta

1441 caggatgagg agtttaaaga ctattctaca caaactgccc aaactttaaa acagaagctg

1501 gcaaaaattg gtggttacac taaaaaagtg tgtgtcatga caaagatctt ctacttgagc

1561 cctgaaggca agacaagctg ccagtatgat ttaaactcgc aagtaattta ccctgaatcc

1621 tactattgga caacagctca aacaatgaaa gacttggaaa aggccctttc aaatatctta

1681 cctaaggaga atcaaatctt tttgtttgtt tgtttgtttc gtttttgttt gtttgtttgt

1741 tggtttgttt gtttttctt gagatga

(SEQ ID NO:8)

Human SLFN12L protein sequence according to NP_001182719.1 (SEQ ID NO:9):

1 mdlarkeflr gnglaagkmn isidldtnya elvlnvgrvt lgennrkkmk dcqlrkqqne

61 nvsravcall nsgggvikae venkgysykk dgigldlens fsnmlpfvpn fldfmqngny

121 fhifvkswsl etsgpqiatl ssslykrdvt sakvmnasaa leflkdmekt ggraylrpef

181 pakracvdvq eesnmealaa dffnrtelgy kekltftest hveiknfste kllqriteil

241 pqyvsafant dggylfvgln edkevigfka eksyltklee vtknsigklp vhhfcvekgt

301 inylckflgv ydkgrlcgyv yalrverfcc avfakkpdsw hvkdnrvkql tekewiqfmv

361 dsepvceelp spastsspvs qsyplreyin fkiqplryhl pglsekitca pktfcrnlfs

421 qheglkqlic eemgsvnkgs lifsrswsld lglqenhkvl cdallisqdk ppvlytfhmv

481 qdeefkdyst qtaqtlkqkl akiggytkkv cvmtkifyls pegktscqyd lnsqviypes

541 yywttaqtmk dlekalsnil pkenqiflfv clfrfclfvc wfvcfflr

(SEQ ID NO:9)

The divergent AAA domain spans positions 218 to 349 of human SLFN12L.

Further SLFN2 homologs (SLFN12L) are known in Orang Utan (Pongo abelii, see Gene ID: 100190853 of NCBI database) and Chimpanzee (Pan troglodytes, see Gene ID: 454585 of NCBI database).

The term Slfn2 protein as used herein refers e.g to a protein having or comprising
i. an amino acid sequence according to SEQ ID NO: 2, 7 or 9,
ii. an amino acid sequence that is a fragment of the amino acid sequence according to (i) and/ or
iii. an amino acid sequence that has at least 80%, at least 90%, at least 95% or at least 99% sequence identity to the amino acid sequence according to (i) or (ii) and/or
iv. an amino acid sequence with at least 95% or at least 99% or 100% identity to at least 10, 11, 12, 13, 14 or 15 consecutive amino acids of the Slfn2 protein according to SEQ ID NO:2, and/or
v. an amino acid sequence with at least 50% identity with the amino acid sequence according to SEQ ID NO:2 and/or
vi. an amino acid sequence with one of the following sequence motifs: YxxlxV, YxxlxVxxW, YxxIxVxxWS or YxxlxVxxWSxS with "x" standing for any amino acid and/or
vii. carries a variant AAA domain, preferably with 50, 60, 70, 80, 90, 95 or more sequence identity with the variant AAA domain of the Slfn2 protein according to SEQ ID NO:2.

This means that the Slfn2 protein or the functionally active variant can comprise, consist or or have an amino acid sequence with the following features: An amino acid sequence having (1), (2), (3), (4), (5), (6) and/or (7). This means that the Slfn2 protein at least has (1) or (2) or (3) or (4) or (5) or (6) or (7); the protein can also have (1) and (2) or (1) and (3) or (1) and (4) or (1) and (5) or (1) and (6) or (1) and (7) or (2) and (3) or (2) and (6) or (2) and (5) or (2) and (6) or (2) and (7) or (3) and (4) or (3) and (5) or (3) and (6) or (3) and (7) or (6) and (5) or (4) and (6) or (4) and (7) or (5) and (6) or (5) and (7) or (6) and (7); the data carrier can also comprise (1) and (2) and (3) or (1) and (2) and (4) or (1) and (2) and (5) or (1) and (2) and (6) or (1) and (2) and (7) or (1) and (3) and (4) or (1) and (3) and (5) or (1) and (3) and (6) or (1) and (3) and (7) or (1) and (4) and (5) or (1) and (4) and (6) or (1) and (4) and (7) or (1) and (5) and (6) or (1) and (5) and (7) or (1) and (6) and (7) or (2) and (3) and (4) or (2) and (3) and (5) or (2) and (3) and (6) or (2) and (3) and (7) or (2) and (4) and (5) or (2) and (4) and (6) or (2) and (4) and (7) or (2) and (5) and (6) or (2) and (5) and (7) or or (2) and (6) and (7) or (3) and (4) and (5) or (3) and (4) and (6) or (3) and (4) and (7) or (4) and (5) and (6) or (4) and (5) and (7) or (5) and (6) and (7); the data carrier can also comprise (1) and (2) and (3) and (4) or (1) and (2) and (3) and (5) or (1) and (2) and (3) and (6) or (1) and (2) and (3) and (7) or (2) and (3) and (4) and (5) or (2) and (3) and (4) and (6) or (2) and (3) and (4) and (7) or (3) and (4) and (5) and (6) or (3) and (4) and (5) and (7) or (4) and (5) and (6) and (7); the data carrier can also comprise (1) and (2) and (3) and (4) and (5) or (1) and (2) and (3) and (4) and (6) or (1) and (2) and (3) and (4) and (7) or (2) and (3) and (4) and (5) and (6) or (2) and (3) and (4) and (5) and (7) or (3) and (4) and (5) and (6) and (7).

The term "pain", as used in the context of the present invention, refers to a complex subjective sensation reflecting real or potential tissue damage and the affective response to it. The term "pain" encompasses acute pain, subacute pain and chronic pain. It further encompasses disease-associated pain, algesia and migraine.

Acute pain is a physiological signal indicating a possible or actual injury. Acute pain starts abruptly and is generally sharp in quality. It serves as a warning of disease or a threat to the body. Acute pain might be mild and last just a moment, or it might be severe and last for weeks. In most cases, acute pain disappears when the underlying cause of pain has been treated or healed. Acute pain might be somatogenetic (organic). Organic pain might be any pain resulting from a disorder, abnormality or chemical imbalance in an organ system, for example, the human or animal body. Thus, organic pain is a term covering pain causes that range in diversity from heartburn to multiples sclerosis. Almost any system or body organ can be affected by organic pain including, but not limited to, the circulatory system, musculoskeletal system, or neurological system. Further examples of organic pain are constipation, diarrhoea, gastrointestinal tract infections, enteritis, colitis, or urinary tract infections.

Unrelieved acute pain, however, might lead to chronic pain. Subacute pain can be classified as a status between acute pain and chronic pain.

Chronic pain can either be somatogenetic (organic) (see above) or psychogenic (psychosomatic). Chronic pain is frequently accompanied or followed by vegetative signs, which often result in depression.

Traditionally, the discrimination between acute and chronic pain has relied upon an arbitrary interval of time from onset, the two most commonly used markers being 3 months and 6 months from the initiation of pain (Turk, D.C., Okifuji, A. (2001), "Pain terms and taxonomies". In Loeser, D., Butler, S. H., Chapman, J.J. et al. Bonica's management of pain (3 ed.), Lippincott Williams & Wilkins, pages 18 to 25). Some researchers have placed the transition from acute to chronic pain at 12 months (Main, C.J., Spanswick, C.C. (2001), Pain management: an interdisciplinary approach, Elsevier, page 93). For other researchers acute pain is pain that lasts less than 30 days, chronic pain is pain of more than six months duration, and subacute pain is pain that lasts from one to six months (Thienhaus, O., Cole, B.E. (2002), "Classification of pain", In Weiner, R.S. Pain management: A practical guide for clinicians (6 ed.), American Academy of Pain Management).

In the context of the present invention, acute pain may refer to pain that lasts less than 30 days (about 1 month), chronic pain may refer to pain that lasts more than six months, and subacute pain may refer to pain that lasts from one to six months. Alternatively, in the context of the present invention, acute pain may refer to pain that does not extend beyond the expected period of healing and chronic pain may refer to pain that extends beyond the expected period of healing.

Further, the term "pain", as used herein, means pain of nociceptive, inflammatory or neuropathic origin.

Nociceptive pain is part of a rapid warning relay instructing the motor neurons of the central nervous system to minimize detected physical harm. Nociceptive pain is caused by activation of nociceptors. These nociceptors are free nerve endings that terminate just below the skin, in tendons, joints, and in body organs. Nociceptive pain may be divided into "superficial somatic" pain and "deep" pain. "Deep" pain, in turn, may be divided into "deep somatic" pain and "visceral" pain. "Superficial somatic pain" is initiated by activation of nociceptors in the skin or superficial tissues, while "deep somatic" pain is initiated by stimulation of nociceptors in ligaments, tendons, bones, blood vessels, fasciae and muscles, and is dull, aching, poorly-localized pain. "Visceral" pain originates in the viscera (organs). "Visceral" pain may be well-localized, but often it is extremely difficult to locate, and several visceral regions produce "referred" pain when injured, where the sensation is located in an area distant from the site of pathology or injury. Examples of nociceptive pain include, but are not limited to, sprains, bone fractures, burns or obstructions. Further, nociceptive pain can be associated with nerve damage caused by trauma, diseases such as diabetes, shingles, irritable bowel syndrome, late-stage cancer or the toxic effects of chemotherapy. Nociceptive pain is generally time-limited. This means that when the tissue damage heals, the pain typically resolves. Thus, nociceptive pain is usually acute pain. Another characteristic of nociceptive pain is that it tends to respond well to treatment with opioids.

Neuropathic pain or neuralgia (both terms are used synonymously herein) can be defined as non-nociceptive pain or as pain that is not related to activation of pain receptor cells in any part of the body. Neuropathic pain or neuralgia is rather caused by damage to or malfunction of the nervous system. Unlike nociceptive pain, neuropathic pain exists with no continuous nociceptive input. Neuralgia or neuropathic pain can be defined as non-nociceptive pain, or in other words, pain that is not related to activation of pain receptor cells in any part of the body. It is believed that neuralgia is pain produced by a change in neurological structure or function. Unlike nociceptive pain, neuralgia exists with no continuous nociceptive input. Neuralgia falls into two categories: central neuralgia and peripheral neuralgia. This unusual pain is thought to be linked to four possible mechanisms: ion gate malfunctions; the nerve becomes mechanically sensitive and creates an ectopic signal; cross signals between large and small fibers; and malfunction due to damage in the central processor. Neuralgia falls into two categories: central neuropathic pain or neuralgia (originating in the brain or spinal cord) and peripheral neuropathic pain or neuralgia (originating in the peripheral nervous system). Peripheral neuropathic pain can be described as burning, tingling, stabbing or as pins and needles. Bumping the "funny bone", for example, elicits peripheral neuropathic pain. Neuropathic pain is often difficult to diagnose, and most treatments show little or no effectiveness. Diagnosis typically involves locating the damaged nerve by identifying missing sensory or motor function. This may involve tests such as an EMG test or a nerve conduction test. Neuralgia is more difficult to treat than other types of pain because it does not respond well to normal pain medications. Examples of neuropathic pain include, but are not limited to, reflex sympathetic dystrophy, nerve trauma, components of cancer pain, phantom limb pain, entrapment neuropathy (e.g. carpal tunnel syndrome) and peripheral neuropathy (widespread nerve damage), bony hyperostosis casts: crutches, prolonged cramped postures; haemorrhage into a nerve; exposure to cold or radiation; collagen-vascular disorders; infectious diseases such as Lyme disease and HIV; toxins such as emetine, hexobarbital, barbital, chlorobutanol, sulfonamides, phenytoin, nitrofurantoin, the vinca alkaloids, heavy metals, carbon monoxide, triorthocresylphosphate, orthodinitrophenol, and other solvents and industrial poisons; autoimmune reactions; nutritional deficiency, and vitamin B deficiency in particular ; and metabolic disorders such as hypothyroidism, porphyria, sarcoidosis, amyloidosis, uremia and diabetes (see e.g. The Merck Manual, 16th ed. 1518 (1992). Peripheral neuropathy may have several causes, for example, diabetes, chronic alcohol use, exposure to toxins (applied in chemotherapies), or vitamin deficiencies. Neuropathic pain is frequently chronic. It tends to have a less robust response to treatment with opioids. However, it may respond well to other drugs such as anti-seizure and anti-depressant drugs.

The pain may also be a mixed category pain. In some conditions the pain appears to be caused by a complex mixture of nociceptive pain and neuropathic pain. For example, an initial nervous system dysfunction o injury may trigger the neural release of inflammatory mediators and subsequent neurogenic inflammation. Migraine headaches, for example, probably represent a mixture of nociceptive pain and neuropathic pain.

Inflammatory pain is associated with tissue damage and the resulting inflammatory process. Chronic pain may involve a mix of both inflammatory and neuropathic components, whereas inflammation may cause damage to the neurons and produce neuropathic pain or neuronal injury may cause an inflammatory reaction that contributes to the inflammatory pain.

Algesia, from the Greek word algesis, is the sensitivity to pain. Hyperalgesia is an increased sensitivity to pain, which may be caused by damage to nociceptors or peripheral nerves. Hyperalgesia can be experienced in focal, discrete areas, or as a more diffuse, body-wide form. Conditioning studies have established that it is possible to experience a learned hyperalgesia of the latter, diffuse form. The focal form is typically associated with injury, and is divided into two subtypes: (i) primary hyperalgesia which describes pain sensitivity that occurs directly in the damaged tissues or (ii) secondary hyperalgesia which describes pain sensitivity that occurs in surrounding undamaged tissues.

For an overview of pain mechanisms, it is referred to Scholz and Woolf, 2002; Julius and Basbaum, 2001, Woolf and Mannion, 1999; Wood, J.D., 2000; Woolf and Salter, 2000.

Many diseases and diseased states are associated with pain (disease-associated pain or diseased state-associated pain). These pain types may also fall under the above pain classifications. Examples of disease-associated pain include, but are not limited to, pain associated with arthritis, back pain, cancer, epilepsy, lumbago, sciatica, lumbar spinal stenosis, cervical spinal stenosis, (clinical) depression, Fibromyalgia, Chronic Fatigue Syndrome, Complex Regional Pain Syndrome, Irritable Bowel Syndrome, Myofascial Pain Syndrome, Post-Vasectomy Pain Syndrome or Restless Leg Syndrome. Disease-associated pain may also be pain associated with diabetes, e.g. diabetic neuropathy or the diabetic foot, pain associated with Parkinson's Disease, pain associated with viral infections, e.g. influenza or HIV, or bacterial infections, pain associated with fever, pain associated with levodopa, e.g. in Parkinson's disease, pain associated with coronary heart disease, pain associated with shingles, pain associated with headache, pain associated with migraine, pain associated with muscular tension, especially chronic muscular tension, osteoarthritis as well as the pain-associated diseases listed under causes of neuropathic pain above.

Migraine (from the Greek words *hemi,* meaning half, and *kranion,* meaning skull) is a debilitating condition characterized by moderate to severe headaches. Migraine headache is typically associated with unilateral pain (affecting one half of the head) and pulsating in nature. It usually lasts between 2 to 70 hours. Symptoms of migraine include, but are not limited to, nausea, vomiting, photophobia (increased sensitivity to light), and phonophobia (increased sensitivity to sound). The cause of migraine headache is unknown. The most common theory is a disorder of the serotonergic control system. Migraine is usually treated with analgesics for the headache and antimetics for the nausea. In addition, triggering conditions such as noise or bright light should be avoided. Pericranial (jaw and neck) muscle tenderness is a most common finding in migraine. Thus, tender muscle trigger points can be at least part of the cause, and perpetuate most kinds of headaches.

As described above, migraine is a disease that is associated with pain but is a disease that comprises more and different symptoms than symptoms of pain. Said types of disease that are intimately connected with symptoms of pain but that have in addition symptoms outside of pain/algesia, are referred to, herein, as pain-associated diseases. Some pain associated diseases are listed above in connection with disease-associated pain states and display pain as one characteristic disease symptom (e.g. migraine). Other pain associated diseases (either psychogenic or somatogenic) may be increased, provoked or even caused by pain, such as depression or epilepsy.

The herein described definitions of pain, pain-stages, pain-states, pain-sensations and pain-sensitivities etc. also comprise equivalent states, stages, sensations and signalling cascades in lower animals or organisms, such as *c*. *elegans.* Moreover, the term pain also comprises pre-stages of pain.

The terms "tissue status", "status of a tissue", "tissue state" and "state of a tissue" are used interchangeably herein referring to the condition of a tissue. The state of a tissue may be characterized by a specific morphology of such tissue or may be characterised by the expression of one or more specific molecules such as but not limited to peptides, proteins, and nucleic acids, or combinations thereof. The status of a tissue may be regarded as "healthy" or "normal" in case it resembles the condition of such tissue when being free from illness or injury and efficiently fulfilling its specific function. The status of a tissue may be regarded as "degenerative", "diseased" or "abnormal" in case such tissue fails to fulfil its function due to an illness or injury. Additionally or alternatively, the status of a tissue may be regarded as "degenerative", "diseased" or "abnormal" in case the morphology of the tissue or its molecule expression pattern is "altered" or "changed" in comparison to normal tissue. Accordingly, the morphology of a tissue or the expression pattern of specific molecules in a tissue may be an indicator for the state of a tissue. Examples of a tissues status include but are not limited to tissue degradation such as cartilage degradation, bone degradation, and degradation of the synovium, tissue inflammation such as cartilage inflammation, or inflammation of the synovium, tissue remodelling such as bon remodelling or cartilage remodelling, sclerosis, liquid accumulation, or proliferative tissue such as proliferation in wound healing processes, cystic formations, or in cancer.

The term "pain-related or pain-associated tissue status" as used herein, refers to a status of a tissue typically associated with pain or a pain-associated disease, typically a diseased or abnormal tissue state such as but not limited to an infection of tissue, infarction, necrosis or other states of tissue associated with pain.

The term "tissue" as used herein, refers to an ensemble of cells of the same origin which concertedly fulfil a specific function. Examples of a tissue include but are not limited to bone, cartilage, connective tissue, muscle tissue, nervous tissue, and epithelial tissue. Multiple tissues together form an "organ" to carry out a specific function. Examples of an organ include but are not limited to joint, skeleton, muscle, blood, brain, heart, liver, kidney, stomach, and skin. For example, a joint is formed of many different tissues such as but not limited to bone, cartilage, synovium, muscle, ligament, and tendon.

The terms "disease" and "disorder" are used interchangeably herein, referring to an abnormal condition, especially an abnormal medical condition such as an illness or injury, wherein a tissue, an organ or an individual is not able to efficiently fulfil its function anymore. Typically, but not necessarily, a disease is associated with specific symptoms or signs indicating the presence of such disease. The presence of such symptoms or signs may thus, be indicative for a tissue, an organ or an individual suffering from a disease. An alteration of these symptoms or signs may be indicative for the progression of such a disease. A progression of a disease is typically characterized by an increase or decrease of such symptoms or signs which may indicate a "worsening" or "bettering" of the disease. The "worsening" of a disease is characterized by a decreasing ability of the tissue, organ or organism to fulfil its function efficiently, whereas the "bettering" of a disease is typically characterized by an increase in the ability of the tissue, an organ or an individual to fulfil its function efficiently. A tissue, an organ or an individual being at "risk of developing" a disease is in a healthy state but shows potential of a disease emerging. Typically, the risk of developing a disease is associated with early or weak signs or symptoms of such disease. In such case, the onset of the disease may still be prevented by treatment. Examples of a disease include but are not limited to traumatic diseases, inflammatory diseases, infectious diseases, cutaneous conditions, endocrine diseases, intestinal diseases, neurological disorders, joint diseases, genetic disorders, autoimmune diseases, and various types of cancer.

"Symptoms" of a disease are implication of the disease noticeable by the tissue, organ or organism having such disease and include but are not limited to pain, weakness, tenderness, strain, stiffness, and spasm of the tissues, an organ or an individual. "Signs" or "signals" of a disease include but are not limited to the change or alteration such as the presence, absence, increase or elevation, decrease or decline, of specific indicators such as biomarkers or molecular markers, or the development, presence, or worsening of symptoms. Symptoms of pain include, but are not limited to an unpleasant sensation that may be felt as a persistent or varying burning, throbbing, itching or stinging ache.

The term "biomarker" or "indicator" are used interchangeably herein. In the context of present invention, a biomarker can be defined as a substance within a biological system that is used as an indicator of a biological state of said system. In the art, the term "biomarker"ís sometimes also applied to means for the detection of said endogenous substances (e.g. antibodies, nucleic acid probes etc, imaging systems). In the context of present invention, however, the term "biomarker"shall only be applied for the substance, not for the detection means.

Thus biomarkers can be any kind of molecule present in a living organism, such as a nucleic acid (DNA, mRNA, miRNA, rRNA etc.), a protein (cell surface receptor, cytosolic protein etc.), a metabolite or hormone (blood sugar, insulin, estrogen, etc.), a molecule characteristic of a certain modification of another molecule (e.g. sugar moieties or phosphoryl residues on proteins, methyl-residues on genomic DNA) or a substance that has been internalized by the organism or a metabolite of such a substance.

As shown by the inventors, there is surprisingly a close correlation between pain and the expression/steady state level of Slfn2. Slfn2 can thus be used as an indicator of pain, i.e. qualifies as biomarker for pain.

The indicator or biomarker refers to a sign or signal for a condition or is used to monitor a condition. Such a "condition" refers to the biological status of a cell, tissue or organ or to the health and/or disease status of an individual. An indicator may be the presence or absence of a molecule, including but not limited to peptide, protein, and nucleic acid, or may be a change in the expression level or pattern of such molecule in a cell, or tissue, organ or individual. An indicator may be a sign for the onset, development or presence of a disease in an individual or for the further progression of such disease. An indicator may also be a sign for the risk of developing a disease in an individual. Known indicators of pain include but are not limited to: Cystatin C (Pain 102 (2003) p.251-256), Cathepsin H and Cathepsin C and the circulatory pain-indicators beta endorphin, serotonin, 5-hydroxy indole acetic acid, anandamide, N-palmitoylethanolamide (for an overview see e.g. Journal of the American Osteopathic Association (2007) 107; p.387-400).

As used herein, the term "variant" is to be understood as a polynucleotide or protein which differs in comparison to the polynucleotide or protein from which it is derived by one or more changes in the sequence and comprises fragments or derivatives. The polypeptide or polynucleotide from which a protein or nucleic acid variant is derived is also known as the parent polypeptide or nucleic acid. Typically, a variant is constructed artificially, preferably by gene-technological means. Typically, the parent polypeptide or polynucleotide is a wild-type protein or nucleic acid. Further, the variants usable in the present invention may also be derived from homologs, orthologs, or paralogs of the parent molecule or from artificially constructed variant, provided that the variant exhibits at least one biological activity of the parent molecule, i.e. is functionally active, if the variant is meant to be a functional variant.

Alternatively or additionally, a "variant" as used herein, can be characterized by a certain degree of sequence identity to the parent polypeptide or parent nucleic acid from which it is derived. More precisely, a protein variant in the context of the present invention exhibits 80% or more sequence identity to its parent polypeptide. A nucleic acid variant in the context of the present invention exhibits 80% or more sequence identity to its parent nucleic acid. The term "80% or more sequence identity" is used throughout the specification with regard to polypeptide and nucleic acid sequence comparisons. This expression preferably refers to a sequence identity of 80% or more, 81% or more, 82% or more, 83% or more, 84% or more, 85% or more, 86% or more, 87% or more, 88% or more, 89% or more, 90% or more, 91 % or more, 92% or more, 93% or more, 94% or more, 95% or more 96% or more, 97% or more, 98% or more, or 99% or more to the respective reference polypeptide or to the respective reference nucleic acid.

The similarity of nucleotide and amino acid sequences, i.e. the percentage of sequence identity, can be determined via sequence alignments. Such alignments can be carried out with several art-known algorithms, e.g. with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on http://www.ebi.ac.uk/Tools/clustalw/ or on http://www.ebi.ac.uk/Tools/clustalw2/index.html or on http://npsa-pbil.ibcp.fr/cgi-bin/npsa_automat.pl?page=/NPSA/npsa_clustalw.html. Preferred parameters used are the default parameters as they are set on http://www.ebi.ac.uk/Tools/clustalw/ or http://www.ebi.ac.uk/Tools/clustalw2/index.html. The grade of sequence identity (sequence matching) may be calculated using e.g. BLAST, BLAT or BlastZ (or BlastX). A similar algorithm is incorporated into the BLASTN and BLASTP programs of Altschul et al. (1990) J. Mol. Biol. 215: 403-410. BLAST nucleic acid searches are performed with the BLASTN program, score = 100, word length = 12, to obtain nucleic acid sequences that are homologous to those nucleic acids which encode Slfn2. BLAST protein searches are performed with the BLASTP program, score = 50, word length = 3, to obtain amino acid sequences homologous to Slfn2. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1:154-162) or Markov random fields. When percentages of sequence identity are referred to in the present application, these percentages are calculated in relation to the full length of the longer sequence, if not specifically indicated otherwise.

The term expression level (of a gene, here e.g. Slfn2) refers to the amount of gene product present in the body or a sample at a certain point of time. The expression level can e.g. be measured/quantified/detected by means of the protein or mRNA expressed from the gene. The expression level can for example be quantified by normalizing the amount of gene product of interest (e.g. Slfn2 mRNA or protein) present in a sample with the total amount of gene product of the same category (total protein or mRNA) in the same sample or a reference sample (e.g. a sample taken at the same time from the same individual or a part of identical size (weight, volume) of the same sample) or by identifying the amount of gene product of interest per defined sample size (weight, volume, etc.). The expression level can be measured or detected by means of any method as known in the art, e.g. methods for the direct detection and quantification of the gene product of interest (such as mass spectrometry) or methods for the indirect detection and measurement of the gene product of interest that usually work via binding of the gene product of interest with one or more different molecules or detection means (e.g. primer(s), probes, antibodies, protein scaffolds) specific for the gene product of interest, here Slfn2.

"Hybridization" can also be used as a measure of sequence identity or homology between two nucleic acid sequences. A nucleic acid sequence encoding miRNAs or a portion thereof can be used as a "hybridization probe" according to standard hybridization techniques. The hybridization of a Slfn2 probe to DNA or RNA from a test source is an indication of the presence of the Slfn2 DNA or RNA in the test source. Hybridization conditions are known to those skilled in the art and can be found, for example, in Current Protocols in Molecular Biology, John Wiley & Sons, N. Y., 6.3.1-6.3.6, 1991. "Moderate hybridization conditions" are defined as equivalent to hybridization in 2X sodium chloride/sodium citrate (SSC) at 30°C, followed by a wash in 1X SSC, 0.1% SDS at 50°C. "Highly stringent conditions" are defined as equivalent to hybridization in 6X sodium chloride/sodium citrate (SSC) at 45°C, followed by a wash in 0.2 X SSC, 0.1 % SDS at 65°C.

The terms "protein" and "polypeptide" are used interchangeably herein and refer to any peptide-linked chain of amino acids, regardless of length or post-translational modification. Proteins usable in the present invention (including protein derivatives, protein variants, protein fragments, protein segments, protein epitopes and protein domains) can be further modified by chemical or biological modification. This means such a biologically or chemically modified polypeptide comprises other chemical groups than the 20 naturally occurring amino acids. Examples of such other chemical groups include without limitation glycosylated amino acids, phosphorylated amino acids or covalent attachment of amino-acid chains e.g. for stabilization of the protein/polypeptide (such as attachment of, e.g. rPEG, XTEN or PAS). Modification of a polypeptide may provide advantageous properties as compared to the parent polypeptide, e.g. one or more of enhanced stability, increased biological half-life, or increased water solubility. Chemical modifications applicable to the variants usable in the present invention include without limitation: PEGylation, glycosylation of non-glycosylated parent polypeptides, or the modification of the glycosylation pattern present in the parent polypeptide, rPEGylation, XTENylation or PASylation.

In the context of the different aspects of present invention, the term "peptide" refers to a short polymer of amino acids linked by peptide bonds. It has the same chemical (peptide) bonds as proteins, but is commonly shorter in length. The shortest peptide is a dipeptide, consisting of two amino acids joined by a single peptide bond. There can also be a tripeptide, tetrapeptide, pentapeptide, etc. Preferably, the peptide has a length of up to 8, 10, 12, 15, 18 or 20 amino acids. A peptide has an amino end and a carboxyl end, unless it is a cyclic peptide.

In the context of the different aspects of present invention, the term "polypeptide" refers to a single linear chain of amino acids bonded together by peptide bonds and preferably comprises at least about 21 amino acids. A polypeptide can be one chain of a protein that is composed of more than one chain or it can be the protein itself if the protein is composed of one chain.

In the context of the different aspects of present invention, the term "protein" refers to a molecule comprising one or more polypeptides that resume a secondary and tertiary structure and additionally refers to a protein that is made up of several polypeptides, i.e. several subunits, forming quaternary structures. The protein has sometimes non-peptide groups attached, which can be called prosthetic groups or cofactors.

In the context of present invention, the primary structure of a protein or polypeptide is the sequence of amino acids in the polypeptide chain. The secondary structure in a protein is the general three-dimensional form of local segments of the protein. It does not, however, describe specific atomic positions in three-dimensional space, which are considered to be tertiary structure. In proteins, the secondary structure is defined by patterns of hydrogen bonds between backbone amide and carboxyl groups. The tertiary structure of a protein is the three-dimensional structure of the protein determined by the atomic coordinates. The quaternary structure is the arrangement of multiple folded or coiled protein or polypeptide molecules molecules in a multi-subunit complex. The terms "amino acid chain" and "polypeptide chain" are used synonymously in the context of present invention.

The term Slfn2 or Slfn2 protein also encompasses naturally occurring variants such as homologs and orthologs in same or different species, in particular in human. The different aspects of present invention also relate to functional variants of Slfn2 protein, either naturally occurring or non-naturally occurring. The term "variant" of a Slfn2 protein comprises fragments and derivatives of a protein. A fragment is a protein or polypeptide that carries one or more end-terminal (n- and/or c-terminal) or internal deletions of one, two or more amino acids, when compared to the full-length protein. A functional fragment of the protein is any fragment of this protein having at least one and preferably two or more of the functional characteristics of the full-length protein. The term derivative of a protein comprises any type of modification of the protein in comparison to the naturally-occurring form, and in the context of present invention especially in comparison to the Slfn2 protein according to SEQ IDs no. 2 and 9, which is not a deletion. A functional derivative of the protein is any derivative of this protein having at least one and preferably two or more of the functional characteristics of the unmodified protein. The variants of Slfn2 of present invention also comprise functional derivatives of fragments of Slfn2.

Non-naturally occurring variants may be obtained by a limited number of amino acid deletions, insertions and/or substitutions, particularly deletions, insertions and/or substitutions of at most 10, 9, 8, 7, 6, 5, 4, 3, 2 or 1 amino acid(s).

A Slfn2 variant of the present invention can e.g. be a functionally active variant, i.e. the variant maintains at least one of the biological functions of Slfn2 as described herein, e.g. its function in the context of pain (e.g. as manifestation of the pain phenotype "mechanic hyperalgesia") or as known in the literature. Preferably, maintenance of biological function is defined as having 50 % or more, 60 % or more, 70 % or more, 80 % or more or 90 % or more or 95 % or more of the activity of the natural occurring Slfn2. A non-functional variant of a Slfn2 protein (with less than the above-identified biological activity or even no detectable biological activity) may also be subject of one of the aspects of present invention, e.g. as negative control. The biological activity may be determined as known to the skilled person. For example, the manifestation of the pain phenotype "mechanic hyperalgesia" can be determined as detailed in the Examples and in Persson et al., 2009, Molecular Pain 5:7.

The Slfn2 variant may be modified in order to comprise a further component. Accordingly, the variant may be a molecule having a domain composed of a naturally occurring Slfn2 protein or a variant thereof as detailed herein and at least one further component. In one embodiment variant may be a fusion protein comprising (i) a Slfn2 protein or functionally active variant and (ii) a further protein component. For example, the protein may be coupled to a marker, such as a tag used for purification purposes (e.g. 6 His (or HexaHis) tag, Strep tag, HA tag, c-myc tag or glutathione S-transferase (GST) tag). If e.g. a highly purified Slfn2 protein or variant should be required, double or multiple markers (e.g. combinations of the above markers or tags) may be used. In this case the proteins are purified in two or more separation chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a hemagglutinin-epitope-tag), His-MBP (His-tag fused to a maltose-binding protein, FLAG-HA-tag (FLAG-tag fused to a hemagglutinin-epitope-tag), and the FLAG-Strep-tag. The marker could be used in order to detect the tagged protein, wherein specific antibodies could be used. Suitable antibodies include anti-HA (such as 12CA5 or 3F10), anti-6 His, anti-c-myc and anti-GST. Furthermore, the Slfn2 protein could be linked to a marker of a different category, such as a fluorescence marker or a radioactive marker, which allows for the detection of Slfn2. In a further embodiment, Slfn2 could be part of a fusion protein, wherein the second part could be used for detection, such as a protein component having enzymatic activity.

In another embodiment of the present invention, the Slfn2 variant could be a Slfn2 fragment, wherein the fragment is still functionally active. This may include Slfn2 proteins with short internal and/or C- and/or N-terminal deletions (e.g. deletions of at most 20, 19, 18, 17, 16, 15, 14, 13, 12, 11, 10, 9, 8, 7, 6 5, 4, 3, 2, or 1 amino acid). Additionally, the Slfn2 fragment may be further modified as detailed above for the Slfn2 protein.

Alternatively or additionally, the Slfn2 variant may comprise one or more amino acid substitution(s). Semi-conservative and especially conservative amino acid substitutions, wherein an amino acid is substituted with a chemically related amino acid are preferred. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. Typical semi-conservative and conservative substitutions are:

| Amino acid | Conservative substitution | Semi-conservative substitution |
|---|---|---|
| A | G;S;T | N;V;C |
| C | A; V; L | M; I; F; G |
| D | E;N;Q | A;S;T;K;R;H |
| E | D; Q; N | A; S; T; K; R; H |
| F | W; Y; L; M; H | I; V; A |
| G | A | S; N; T; D; E; N; Q |
| H | Y;F;K;R | L;M;A |
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A;T;G;N | D;E;R;K |
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure.

The Slfn2 protein or fragment or variant with substitution may be modified as detailed above for the Slfn2 protein or fragment or variant. In the following description of the invention all details given with respect to Slfn2 protein also relate to functionally active variants thereof, unless stated otherwise. The above modifications of the Slfn2 protein may be combined. The variant of the present invention may be e.g. fragment of Slfn2 having a marker fused to it, or a Slfn2 protein fragment comprising one or more amino acid substitutions.

A derivative of Slfn2 protein of a Slfn2 protein fragment is a Slfn2 protein or fragment carrying any kind of modification of the amino acid sequence that is not a deletion and/or carrying any other kind of modification, such as a chemical or biological modification. Derivatives comprise proteins carrying one or more modifications e.g. leading to the stabilization of the polypeptide, or enabling a specific targeting of the polypeptide to certain cells or facilitating its entry into or uptake by cells (such as cell-permeant phosphopeptides ortho coupling to cell-permeant peptide vectors, e.g. based on the antennapedia/penetratin, TAT, and signal-peptide based sequences; or coupling to parts of ligands for specific transporters or importers). According to one embodiment, the Slfn2 protein is a naturally occurring Slfn2 protein as detailed above, such as a naturally occurring mouse Slfn2 protein such as comprising or having SEQ ID NO: 2, or a naturally occurring human orthologous protein, such as comprising or having SEQ ID NO: 9.

The terms "nucleic acid" or "nucleic acid molecule" are used synonymously and are understood as single or double-stranded oligo- or polymers of deoxyribonucleotide or ribonucleotide bases or both. Typically, a nucleic acid is formed through phosphodiester bonds between the individual nucleotide monomers. In the context of the present invention, the term nucleic acid includes but is not limited to ribonucleic acid (RNA) and deoxyribonucleic acid (DNA) molecules. The depiction of a single strand of a nucleic acid also defines (at least partially) the sequence of the complementary strand. The nucleic acid may be single or double stranded, or may contain portions of both double and single stranded sequences. The nucleic acid may be obtained by biological, biochemical or chemical synthesis methods or any of the methods known in the art. As used herein, the term "nucleic acid" comprises the terms "polynucleotide" and "oligonucleotide".

In the context of the different aspects of present invention, the term nucleic acid comprises cDNA, genomic DNA, recombinant DNA, cRNA and mRNA. A nucleic acid may consist of an entire gene, or a portion thereof, the nucleic acid may also be a microRNA (miRNA) or small interfering RNA (siRNA). MiRNAs are short ribonucleic acid (RNA) molecules, on average only 22 nucleotides long, found in all eukaryotic cells. MircoRNAs (miRNAs) are post-transcriptional regulators that bind to complementary sequences on target messenger RNA transcripts (mRNAs), usually resulting in translational repression and gene silencing. Small interfering RNAs (siRNAs), sometimes known as short interfering RNA or silencing RNA, are short ribonucleic acid (RNA molecules), between 20-25 nucleotides in length. They are involved in the RNA interference (RNAi) pathway, where they interfere with the expression of specific genes. The nucleic acid can also be an artificial nucleic acid. Artificial nucleic acids include polyamide or peptide nucleic acid (PNA), morpholino and locked nucleic acid (LNA), as well as glycol nucleic acid (GNA) and threose nucleic acid (TNA). Each of these is distinguished from naturally-occurring DNA or RNA by changes to the backbone of the molecule.

The nucleic acids, can e.g. be synthesized chemically, e.g. in accordance with the phosphotriester method (see, for example, Uhlmann, E. & Peyman, A. (1460) Chemical Reviews, 90, 543-584). Aptamers are nucleic acids which bind with high affinity to a polypeptide, here Slfn2. Aptamers can be isolated by selection methods such as SELESIfn2 (see e.g. Jayasena (1469) Clin. Chem., 45, 1628-50; Klug and Famulok (1464) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1466) Nat. Biotechnol., 14, 1116-9; Klussmann et al. (1466) Nat. Biotechnol., 14, 1112-5). Forms which have been isolated in this way enjoy the advantage that they are not degraded by naturally occurring ribonucleases and, therefore, possess greater stability. Nucleic acids may be degraded by endonucleases or exonucleases, in particular by DNases and RNases which can be found in the cell. It is, therefore, advantageous to modify the nucleic acids in order to stabilize them against degradation, thereby ensuring that a high concentration of the nucleic acid is maintained in the cell over a long period of time (Beigelman et al. (1465) Nucleic Acids Res. 23:3989-94; WO 95/11910; WO 98/37240; WO 97/29116). Typically, such stabilization can be obtained by introducing one or more internucleotide phosphorus groups or by introducing one or more non-phosphorus internucleotides. Suitably modified internucleotides are compiled in Uhlmann and Peyman (1460), supra (see also Beigelman et al. (1465) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention.

The term "oligonucleotide" when used in the context of one of the different aspects of present invention, refers to a nucleic acid of up to about 50 nucleotides, e.g. 2 to about 50 nucleotides in length.

The term "polynucleotide" when used in the context of one of the different aspects of present invention, refers to a nucleic acid of more than about 50 nucleotides in length, e.g. 51 or more nucleotides in length.

Probes and Primers are short polynucleotides or oligonucleotides for the detection of nucleic acids in a sample or in vivo by hybridizing (probe) to the target nucleic acid or by hybridization to and amplification of the target nucleic acid. Nucleic acids suitable for use as probe or primer comprise e.g. a polynucleotide probe, one or more primers (e.g. a primer pair), preferably one or more primers for polymerase chain reaction (PCR), reverse transcription (RT) reaction, or DNA sequencing, a peptide- or polyamid- nucleic acid (PNA), a locked nucleic acid (LNA), a glycol nucleic acid (GNA), a threose nucleic acid (TNA), a microRNA (miRNA), and a small interfering RNA (siRNA). The one or more primers (e.g. a primer pair) can e.g. be for real time polymerase chain reaction (RT-PCR) or for quantitative real time polymerase chain reaction (qRT-PCR).

The term "probe" as used herein refers to a nucleic acid which is typically used for the detection of target RNA and/or DNA sequences that is complementary to the sequence of the probe. A probe hybridizes to single-stranded nucleic acid (DNA or RNA) whose nucleotide sequence allows for nucleotide pairing due to complementarity between the probe and the target sequence. The length of a probe depends on the intended use as well as the required specificity of the probe. Typically, a probe is 20-500 (i.e. 50, 55, 60, 65, 70, 75, 80, 85, 90, 95, 100, 110, 120, 130, 140, 150, 160, 170, 180, 190, 200, 300, 400, 500) nucleotides long, preferably 20-100 nucleotides, more preferably 20-50. Probes are used in various experimental set ups such as but not limited to Southern and Northern Blots, for real-time PCR and In Situ Hybridization (ISH) as well as for microarray experiments. A probe may be unlabeled, directly labelled, or indirectly labelled, such as with biotin to which a streptavidin complex may later bind. Said label may be a molecule detectable by spectroscopic, photochemical, biochemical, immunochemical, chemical, or other physical means. For example, suitable labels include 32P, fluorescent dyes, electron-dense reagents, enzymes (e.g., as commonly used in an ELISA), biotin, digoxigenin, or haptens and other entities which are or can be made detectable. A label may be incorporated into nucleic acids at any position, e.g. at the 3' end, at the 5' end or internally. The term "probe" also encompasses nucleic acids differing in the composition of their backbone such as but not limited to peptide nucleic acids (PNAs), locked nucleic acids (LNAs), glycol nucleic acids (GNAs) and threose nucleic acids (TNAs).

Said nucleic acid as a probe may be unlabeled, directly labeled, or indirectly labeled, such as with biotin to which a streptavidin complex may later bind. Said label may be a molecule detectable by spectroscopic, photochemical, biochemical, immunochemical, chemical, or other physical means. For example, suitable labels include 32P, fluorescent dyes, electron-dense reagents, enzymes (e.g., as commonly used in an ELISA), biotin, digoxigenin, or haptens and other entities which are or can be made detectable. A label may be incorporated into nucleic acids at any position, e.g. at the 3' end, at the 5' end or internally.

The term "primer" as used herein refers to a single-strand nucleic acid which typically serves as a starting point for DNA-replicating enzymes. A primer binds to or hybridises with a DNA template and typically comprises a sequence being complementary to the DNA sequence to which it is supposed to bind. A primer may also comprise additional sequences e.g. sequences serving as nuclease cleavage sites (e.g. Bam H1, Hind III, etc.). The length of a primer is chosen depending on the intended use. For instance, primers used for the amplification of DNA in Polymerase-Chain Reactions (PCR) typically have a length of at least 10 nucleotides, preferably between 10 to 50 (i.e. 15, 16, 17, 18, 19, 20, 21, 22, 23, 24, 25, 26, 27, 28, 29, 30, 31, 32, 33, 34, 35, 36, 37, 38, 39, 40, 45, 50) nucleotides, more preferably between 15 and 30 nucleotides. Shorter primers of at least 5 nucleotides are used for sequencing of DNA templates. Also encompassed in the term "primer" are "degenerate primers", which are a mixture of similar but not identical primers. A primer may be tagged or labeled with a marker molecule detectable by spectroscopic, photochemical, biochemical, immunochemical, chemical, or other physical means.

Probes and Primers for the detection of Slfn2 are known in the art (see e.g. Wern-Joo et al, 2007) and can be ordered custom-made by commercial vendors against any known sequence including that of Slfn2. Examples include (all taken from Wern-Joo et al., 2007):
5'-CTCACCTCAGAAAACAGGAGAATGC-3' (sense murine Slfn2; SEQ ID NO:11),
5'-CAGAAGTGAGTGACAGGCAGCTG-3' (anti-sense, murine Slfn2; SEQ ID NO:12),
5'-CGGCTAGCTTGGTGGCTCCTAGCGRR-3' (sense Slfn2 -1766; SEQ ID NO.13),
5'-CGGCTAGCGGAACTGAACTGGAGCT-3' (sense Slfn2 -1265; SEQ ID NO.14),
5'-CCCTCGAGCCATGTCAGCAGTTGGTAAAGC-3' (anti-sense Slfn 2 +198; SEQ ID NO:15). In addition, using standard software, the skilled artisan knows how to select suitable primers.

The above-mentioned peptide nucleic acids (PNAs), locked nucleic acids (LNAs), glycol nucleic acids (GNAs) and threose nucleic acids (TNAs) are artificial nucleic acids which can also be used as probes or primers. Each of these is distinguished from naturally-occurring DNA or RNA by changes to the backbone of the molecule. MiRNAs and siRNAs can also be used as probes.

The above-mentioned nucleic acids are e.g. for detecting the Slfn nucleic acid sequences of present invention, e.g. one or more of the Slfn nucleic acid sequences of embodiment 20 and are e.g. for use in one of the methods according to one of the aspects of present invention.

The above-mentioned nucleic acids may allow the determination of the expression level of Slfn2 on the Slfn2 gene or Slfn2 mRNA level, e.g. in a sample, such as in a sample from a subject or in an assay, such as a biochemical or cellular assay (e.g. in a mixture in the context of a biochemical assay or in a cell in the context of a cellular assay).

The term "variant" of a nucleic acid comprises fragments and derivatives of a nucleic acid. The variants of present invention also comprise functional derivatives of fragments of the nucleic acid. Non-naturally occurring variants may be obtained by a limited number of nucleotide deletions, insertions and/or substitutions, particularly deletions, insertions and/or substitutions of at most 10, 9, 8, 7, 6, 5, 4, 3, 2 or 1 nucleotide(s).

A fragment of a nucleic acid is a nucleic acid that carries one or more terminal (5' (upstream) and/or 3' (downstream)) or internal deletions of one, two or more nucleotides, when compared to the full-length nucleic acid. A functional fragment of the nucleic acid is any fragment of the Slfn2 nucleic acid having at least one and preferably two or more of the functional characteristics of the full-length nucleic acid (e.g. the ability to confer Slfn2-characteristic transcriptional regulation of downstream elements or the characteristic to code for a Slfn2 protein).

The term derivative of a nucleic acid comprises any type of modification (e.g. a chemical or biological modification) of the nucleic acid in comparison to the naturally-occurring form, and in the context of present invention especially in comparison to the Slfn2 nucleic acid according to SEQ IDs no. 1, 3, 4, 6 or 8 that is not a deletion. Derivatives comprise Slfn2 nucleic acids carrying one or more modifications leading to the stabilization of the nucleic acid (such as phosphoorothioate modification or modification of the nucleic acid backbone). A functional derivative of the nucleic acid is any derivative of this nucleic acid having at least one and preferably two or more of the functional characteristics of the unmodified nucleic acid.

The term Slfn2 nucleic acid encompasses nucleic acids coding for the above Slfn2 protein and regulatory nucleic acids of the Slfn2 gene as well as naturally occurring and non-naturally occurring variants thereof (as defined herein). Preferably, the term relates to coding or non-coding regions of the Slfn2 gene, wherein these sections are of a relevant size in order to be specific for that gene. Examples of those regions are introns, exons or regulatory elements such as a Slfn2 promoter. The term Slfn2 or Slfn2 nucleic acid also encompasses naturally occurring variants such as homologs and orthologs in same or different species, in particular in human (e.g. the nucleic acids according to SEQ IDs NO. 1 (mouse), 6 (rat) or 8 (human). The different aspects of present invention also relate to functional variants of Slfn2 nucleic acid, either naturally occurring or non-naturally occurring. Preferred aspects of a Slfn2 nucleic acid relate to the Slfn2 gene, promoter, DNA, cDNA or mRNA or oligonucleotides or polynucleotides able to specifically hybridize with one of these nucleic acids. Examples of Slfn2 nucleic acids are e.g. a Slfn2 nucleic acid coding for the naturally occurring Slfn2 protein as detailed above (e.g. a protein comprising or having an amino acid sequence according to SEQ ID NO: 2, 7 or 9) such as a nucleic acid comprising or having a nucleotide sequence according to SEQ ID NO: 1, 3, 4, 6, or 8). Another example of a Slfn2 nucleic acid is a Slfn2 nucleic acid able to drive expression of a downstream element such as a nucleic acid derived from the Slfn2 gene and comprising regulatory elements thereof but lacking the Slfn2 coding sequence, e.g. a nucleic acid comprising or consisting of a nucleotide sequence according to SEQ ID NO:4.

A Slfn2 nucleic acid variant of the present invention can be a functionally active variant, i.e. the variant maintains at least one of the biological functions of Slfn2 nucleic acid as described herein, e.g. its function in the context of pain (e.g. as manifestation of the pain phenotype "mechanic hyperalgesia", the ability to confer Slfn2-characteristic transcriptional regulation of downstream elements or the characteristic to code for a Slfn2 protein) or as known in the literature. Preferably, maintenance of biological function is defined as having 50 % or more, 60 % or more, 70 % or more, 80 % or more or 90 % or more or 95 % or more of the activity of the natural occurring Slfn2. A non-functional variant of a Slfn2 nucleic acid (with less than the above-identified biological activity or even no detectable biological activity) may also be subject of one of the aspects of present invention, e.g. as negative control. The biological activity may be determined as known to the skilled person, for example, the manifestation of the transcriptional activity as detailed in Wern-Joo et al., 2007; examples of functional and non-functional variants (fragments and derivatives) of nucleic acids, especially different promoter variants are disclosed in Wern-Joo et al, 2007 the disclosure of which is explicitly included herein, as reference. Preferred examples nucleic acids comprising a Slfn2 promoter comprise a Slfn2 nucleic acid from -1256 to +1 or from -1265 to +198 or from +1699 to +1 or from -1699 to +198 with respect to the sequence according to SEQ ID NO: 5.

The term "antibody or fragment thereof", as used herein, refers to immunoglobulin molecules and immunologically active portions of immunoglobulin molecules, i.e. molecules that contain an antigen binding site that specifically binds an antigen. Also comprised are immunoglobulin-like proteins that are selected through techniques including, for example, phage display to specifically bind to a target molecule or target protein. The immunoglobulin molecules can be of any type (e.g., IgG, IgE, IgM, IgD, IgA and IgY), class (e.g., IgG1, IgG2, IgG3, IgG4, IgA1 and IgA2) or subclass of immunoglobulin molecule. The "antibodies and fragments thereof" include, but are not limited to, polyclonal, monoclonal, monovalent, bispecific, heteroconjugate, multispecific, human, humanized (in particular CDR-grafted), deimmunized, or chimeric antibodies, single chain antibodies (e.g. scFv), Fab fragments, F(ab')₂ fragments, fragments produced by a Fab expression library, diabodies or tetrabodies (Holliger P. et al., 1993), nanobodies, anti-idiotypic (anti-Id) antibodies, and epitope-binding fragments of any of the above.

In some embodiments, the antibody fragments are mammalian, preferably human antigen-binding antibody fragments and include, but are not limited to, Fab, Fab' and F(ab')₂, Fd, single-chain Fvs (scFv), single-chain antibodies, disulfide-linked Fvs (dsFv) and fragments comprising either a VL or VH domain. Antigen-binding antibody fragments, including single-chain antibodies, may comprise the variable domain(s) alone or in combination with the entirety or a portion of the following: hinge region, CL, CH1, CH2, and CH3 domains. The antigen-binding fragments may also comprise any combination of variable domain(s) with a hinge region, CL, CH1, CH2, and CH3 domains.

Antibodies usable in the invention may be from any animal origin including birds and mammals. Preferably, the antibodies are human, simian (e.g. chimpanzee, bonobo, macaque), rodent (e.g. mouse and rat), donkey, sheep rabbit, goat, guinea pig, camel, horse, or chicken. It is particularly preferred that the antibodies are of human or murine origin. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins.

The techniques for preparing antibodies and antibody fragments are well known in the art. Means of preparing and characterizing antibodies or antibody fragments are also well known in the art (see, for example, Antibodies: A Laboratory Manual, Cold Spring Harbor Laboratory, 1988; incorporated herein by reference). Antibodies or antibody fragments specific for the Slfn2 protein may be used in a variety of assays in order to quantitate the Slfn2 protein. Well known methods include, for example, immunoprecipitation, antibody sandwich assays, ELISA and affinity chromatography methods (see above).

The antibody that recognizes the target antigen, here Slfn2 or a functionally active variant thereof is generally called the "primary antibody". Said antibody may be labeled with a detectable tag/label in order to allow direct detection of the target antigen. Said detectable tag/label may be an enzymatic, fluorescent or radioisotope tag/label. Usually, however, the primary antibody is not labeled for direct detection. Instead a "secondary antibody" that has been labeled with a detectable tag/label (e.g. enzymatic, fluorescent or radioisotope tag/label) is applied in a second step to probe for the primary antibody, which is bound to the target antigen. For example, the primary antibody or the secondary antibody may be labeled with an affinity tag such as biotin. As to detectable tags/labels which may be used for the detection of Slfn2 and/or as to the specific detection methods it is also referred to the information provided with respect to the methods of certain aspects of the present invention.

Antibodies against Slfn2 are known in the art (see e.g. Katsoulidis et al., 2009) and can be purchased from commercial vendors (amongst others antibodies-online.com). An example of an n-terminal anti Slfn2 antibody suitable for use in e.g. Western Blot or ELISA is a polyclonal rabbit anti-mouse antibody that has been generated using a synthetic peptide corresponding to the n-terminal residues of mouse Slfn2 and that can be purchased from antibodies-online under ABIN393095.

Transgenic animals, and cells: A transgenic animal is an animal that carries a gene or gene fragment (e.g. in its genome or episomal) that is alien to that animal, e.g. originated in another species, is of artificial origin or is usually not expressed in this animal (see e.g. the Slfn2 transgene of Berger et al carrying a Slfn2 transgene in an electra background). Slfn2 transgenic mice are known in the art, see e.g. Berger et al., 2010. The Slfn2 transgenes generated by Berger et al., have an electra genetic background and carry functional Slfn2 as transgene. Transgenic cells can e.g. be provided by isolating cells from such transgenic animals.

Knock-out animals and cells. A knock-out animal or cell is an animal or a cell, in which one or more genes are downregulated or turned off by means of a targeted mutation in the genome or a nucleic acid (e.g. a DNA vector, an oligonucleotide, or a siRNA) introduced transiently or stably into the animal or cell. A knockout animal (such as a rodent (mouse, rat) c. elegans, drosophila or another non-human animal) is a genetically engineered animal, in which one or more genes have been turned off through a targeted mutation.

Mice are currently the most closely related laboratory animal species to humans for which the knockout technique can easily be applied. They are widely used in knockout experiments, especially those investigating genetic questions that relate to human physiology. Gene knockout in rats is also widely used.

The downregulation of Slfn2 can be achieved by any type of modification of the animal or cell (stable or transient, preferably stable), that leads to a decrease of SLFN2 activity (i.e. its ability to SLFN2), SLFN2 transcript steady state level (i.e. by activation of SLFN2 transcription or transcript stabilisation) or SLFN2 protein steady state level (i.e. by activation of SLFN2 translation or its posttranslational processing; by modulation of SLFN2 posttranslational modification or by activation of its stabilisation or by inhibition of its degradation). This can for example be achieved by using dominant negative mutants of SLFN2, antisense oligonucleotides, RNAi constructs of SLFN2, by generating functional or genomic SLFN2 knock outs (which can e.g. be inducible) or other suitable techniques known within the state of the art. For an overview of the above techniques, see for example: Current protocols in Molecular biology (2000) J.G. Seidman, Chapter 23, Supplemtent 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al, 2003.

According to a preferred embodiment, a SLFN2 knock-out cell is used. Suitable cell lines for the generation of knock-outs are well known in the state of the art and comprise e.g Current protocols in Molecular Biology (2000) J.G. Seidman, Chapter 23, Supplement 52, John Wiley and Sons, Inc; or Gene Targeting a practical approach. (1995) Ed. A.L. Joyner, IRL Press.

The term knock-out as used herein also refers to knock-down in cells or animals using siRNA. Slfn2 knock-down cells (stable Slfn2-knock down NIH-3T3 cells) are known in the art (see e.g. Katsoulidis et al., 2009).

The term mass spectrometry probe in the context of present invention refers to a synthetic peptide or polypeptide analog to a native peptide or polypeptide of the Slfn2 protein which is cleavable with a protease (e.g. trypsin protease). Said probe enables absolute protein quantitation of Slfn2 with mass spectrometry, preferably with HPLC-MS or HPCL-MS/MS, particularly supported by multiple reaction monitoring (MRM). It preferably incorporates one stable isotope labeled amino acid, creating a slight increase (e.g. 6-10 daltons) in molecular weight. When mixed, the native peptide of the Slfn2 protein and the synthetic peptide co-elute chromatographically, co-migrate electrophoretically, and ionize with the same intensity. Nevertheless, by mass spectrometry, the native peptide of the Slfn2 protein and the synthetic peptide can easily be distinguished. In a typical mass spectrometry procedure, preferably HPLC-MS or HPLC-MS/MS procedure, a known amount of the synthetic peptide is added to a subject's sample. The sample is then digested (e.g. by a protease such as trypsin protease) and analyzed by mass spectrometry, preferably by HPLC-MS or HPLC-MS/MS. Extracted ion chromatograms are generated for the native peptide of the Slfn2 protein and the synthetic peptide internal standard. Using peak ratios, the quantity of native peptide of the Slfn2 protein is calculated. It also allows quantitation of the amount of the Slfn2 protein in the subject's sample. The techniques for selecting and preparing the synthetic peptide are well known in the art. For example, the skilled person knows how to select precursor-fragment-ion-transitions under the aspect of optimal selectivity and sensitivity.

The term protein scaffold in the context of present invention refers to non-antibody recognition proteins derived from one or more of structurally different protein families and able to bind with high specificity to a selected target, thereby mimeting the binding principle of immunoglobulins to varying degrees (see e.g. Hey, T., et al., 2005: Artificial, non-antibody binding proteins for pharmaceutical and industrial applications, Trends in Biotechnology, Vol. 23, No. 10, the disclosure of which is incorporated herein, by reference). Examples comprise lipocalin-derived repeat proteins such as darpins, ankyrin-repeat proteins such as anticalins, nanobodies, affibodies, maxibodies, transbodies, tetranectin, iMabs, Adnectin, domain antibodies, Kunitz-type domains, Evibodies, Affilins, Microbodies).

The terms "biochip" and "microarray" are interchangeably used herein.

The terms "attached" or "immobilized", as used herein, refer to the binding between the nucleic acid(s), protein, polypeptide, or peptide and the solid support and may mean that the binding between the nucleic acid, protein, polypeptide, or peptide and the solid support is sufficient to be stable under conditions of binding, washing, analysis and removal. The binding may be covalent or non-covalent. Covalent bonds may be formed directly between the nucleic acid, protein, polypeptide, or peptide and the solid support or may be formed by a cross linker or by inclusion of specific reactive groups on either the solid support or the nucleic acid, protein, polypeptide, or peptide, or both. Non-covalent binding may be electrostatic, hydrophilic and hydrophobic interactions or combinations thereof. Immobilization or attachment may also involve a combination of covalent and non-covalent interactions.

The term "test system" in the context of present invention refers to any system (e.g. biochemical, chemical or biological (i.e. cellular or animal) that allows for the performance of a (test) method according to one of the aspects of present invention. The test system of the invention may be used in order to elucidate mechanisms involved in pain. Particularly, the test system may be used to develop, identify and/or characterize agents involved in pain, which interact with a Slfn2 nucleic acid or protein, particularly activating or inactivating the same. The identified agent may be an interesting therapeutic drug, which could be used in the treatment of pain, particularly in neuropathic pain. Alternatively, Slfn2 could be used in the diagnosis of pain such as algesia.

A variety of test designs is known in the art to which the test system according to the present invention may be adapted. The test system may be used in order to determine the effect of a test compound on the test system. The skilled person will be able to design a test system, e.g. by adding further agents required in connection with the prevailing method, suitable for the particular test method intend.

The effect may be determined on the level of Slfn2 protein or gene, as well as on a signal transduction or expression level upstream or downstream. Examples include the Slfn2 gene level (upstream of the Slfn2 protein), the mRNA level (upstream of the Slfn2 protein and downstream of the Slfn2 gene), the protein level (downstream of the Slfn2 gene) and the phenotype level (downstream of the Slfn2 gene and protein).

The method may be a heterogeneous or homogeneous assay. As used herein, a heterogeneous assay is an assay, which includes one or more washing steps, whereas a homogeneous assay does not make use of washing steps. The reagents and compounds are only mixed and measured.

The test method may be either a continuous assay or a discontinuous assay. Continuous assays give the rate of reaction with no further work necessary. There are many different types of continuous assays. In spectrophotometer assays, the course of the reaction is followed by measuring a change in absorbance. Fluorescence is when a molecule emits light of one wavelength after absorbing light of a different wavelength. Fluorometric assays use a difference in the fluorescence of substrate from product to measure the enzyme reaction. These assays are in general much more sensitive than spectrophotometric assays, but can suffer from interference caused by impurities and the instability of many fluorescent compounds when exposed to light. Calorimetry is the measurement of the heat released or absorbed by chemical reactions. These assays are very general, since many reactions involve some change in heat and with use of a microcalorimeter, not much enzyme or substrate is required. These assays can be used to measure reactions that are impossible to assay in any other way. Chemiluminescence is the emission of light by a chemical reaction. Some enzyme reactions produce light and this can be measured to detect product formation. These types of assay can be extremely sensitive, since the light produced can be captured by photographic film over days or weeks, but can be hard to quantify, because not all the light released by a reaction will be detected. Static Light Scattering measures the product of weight-averaged molar mass and concentration of macromolecules in solution. Given a fixed total concentration of one or more species over the measurement time, the scattering signal is a direct measure of the weight-averaged molar mass of the solution, which will vary as complexes form or dissociate. Hence the measurement quantifies the stoichiometry of the complexes as well as kinetics. Light scattering assays of protein kinetics is a very general technique that does not require an enzyme.

Discontinuous assays comprise different steps of sample-taking from an enzyme reaction in intervals, wherein the amount of product (for determination of product generation) or substrate (for determination of substrate consumption) is measured in these samples. Radiometric assays measure the incorporation of radioactivity into substrates or its release from substrates. The radioactive isotopes most frequently used in these assays are ¹⁴C, ³²P, ³⁵S and ¹²⁵I. Since radioactive isotopes can allow the specific labeling of a single atom of a substrate, these assays are both extremely sensitive and specific. They are frequently used in biochemistry and are often the only way of measuring a specific reaction in crude extracts. Chromatographic assays measure product formation by separating the reaction mixture into its components by chromatography. This is usually done by high-performance liquid chromatography (HPLC), but can also use the simpler technique of thin layer chromatography. Although this approach can need a lot of material, its sensitivity can be increased by labeling the substrates/products with a radioactive or fluorescent tag.

In accordance with the present invention the effect of the test compound may be by interaction with a Slfn2 nucleic acid or protein. Accordingly, the interaction/binding of test compound to the Slfn2 nucleic acid or protein could be determined by detecting the complex of (i) the Slfn2 nucleic acid or protein and (ii) the test compound. Suitable methods of detecting complexes of two or more components are detailed below.

Alternatively, the effect, e.g. binding, of the test compound and the influence on Slfn2 nucleic acid or protein could be detected indirectly. For this, the effect downstream the Slfn2 nucleic acid or protein could be detected. For example, the effect on the transcription and translation related to Slfn2 could be determined. In one embodiment, the amount of Slfn2 mRNA or Slfn2 protein is detected.

Many known methods for detection that are designed to measure the presence or quantity of specific proteins or other nucleic acids depend on the use of tags, markers or labels. A component of the test system or the test compound may be labeled in a variety of ways to allow sufficient detection or purification. In one preferred embodiment a detectable marker is used in order to detect an effect on the test system. For this, (i) the nucleic acid or Slfn2 protein, (ii) the test compound and/or (iii) a further component of the test system may be labeled with one or more detectable markers.

Common labeling methods may be used for labeling of one or more functional groups of the component. For a protein, these could be for example the primary amino groups, present at the N-terminal of each polypeptide chain and the side chain of lysine residues; sulphhydryl groups, present on cysteine residues made available by treating disulphide bonds with reducing agent or by modifying lysine residues with a reagent such as succinimidyl-S-acetylthioacetate (SATA); or carbohydrate groups, usually present in the Fc region of antibodies, which may be oxidized to create active aldehydes for coupling. The component or compound may be labeled with a series of different agents, such as biotin (for avidine-biotin chemistry), enzymes, activated fluorescent dyes for labeling amines, sulphhydryls or other functional groups with e.g. FITC, fluorescein, rhodamine, Cy dyes or Alexa fluos. Radioactive label such as ³H, ³²P, ³⁵S, ¹²⁵I or ¹⁴C as well as common enzyme labels including penicillinase, horseradish peroxidase and alkaline phosphatase may be used as well. In an embodiment of the present invention the marker is a radiolabel, particularly ³H, ³²P, ³³P, ³⁵S, ¹²⁵I, or ¹⁴C. In another embodiment the marker is one or more fluorescence marker(s). Suitable fluorescence markers are described in the context of the methods of the present invention.

Particularly useful in these methods is the use of target-specific probes that are detectable via those chemical tags, markers or labels. Antibodies are the most common type of probe; their binding affinities for particular antigens enable those targets to be "found" and detected in a complex sample. However, antibodies are themselves proteins, and they are not specifically detectable in an assay system unless they are labeled for visualization or secondarily probed with another molecule that is labeled.

A marker (or tag or label) is any kind of substance which is able to indicate the presence of another substance or complex of substances. The marker can be a substance that is linked to or introduced in the substance to be detected. Detectable markers are used in molecular biology and biotechnology to detect e.g. a protein, a product of an enzymatic reaction, a second messenger, DNA, interactions of molecules etc. Examples of suitable marker or labels include a fluorophore, a chromophore, a radiolabel, a metal colloid, an enzyme, or a chemiluminescent or bioluminescent molecule. Examples of fluorophores include fluorescein, rhodamine, and sulfoindocyanine dye Cy5. Examples of radiolabels include ³H, ¹⁴C, ³²P, ³³P, ³⁵S, ^{99m}Tc or ¹²⁵I. Examples of enzymes include horseradish peroxidase, alkaline phosphatase, glucose oxidase, and urease. Further examples and preferred embodiments are detailed herein.

Different types of chemical labels or tags can be conjugated to secondary or primary antibodies and other molecules to facilitate their visualization (i.e., detection and measurement) by various methods. Radioisotopes were used extensively in the past, but they are expensive, have a short shelf-life, offer no improvement in signal:noise ratio and require special handling and disposal. Enzymes and fluorophores have largely replaced radioactive isotopes as detectable tags for assays. A number of advancements in reagents and instrumentation make these newer technologies more versatile and powerful. Enzymatic tags such as horseradish peroxidase (HRP) are most commonly used for blotting, immunoassays and immunohistochemistry methods. Fluorescent tags are used predominately for cellular imaging, nucleic acid amplification and sequencing and microarrays; however, fluorescence technology is developing rapidly for application in all types of assays.

The detection of protein often involves the use of specific antibodies. Accordingly, the detection of Slfn2 protein or a variant thereof may include a specific Slfn2 antibody. Alternatively, antibodies can be raised using well established techniques for immunizing animals with prepared forms of the antigen. A variety of reagents is available to assist in antibody production and purification, and various companies specialize in antibody production services. Depending on the application to be performed, different levels of purity and types of specificity are needed in a supplied primary antibody. To name just a few parameters, antibodies may be monoclonal or polyclonal, supplied as antiserum or affinity-purified solution, and validated for native protein or denatured protein detection. An antibody that recognizes the target antigen, here Slfn2 or fragment or variant thereof, is called the "primary antibody." If this antibody is labeled with a tag, direct detection of the antigen is possible. Usually, however, the primary antibody is not labeled for direct detection. Instead a "secondary antibody" that has been labeled with a detectable tag is applied in a second step to probe for the primary antibody, which is bound to the target antigen. Thus, the antigen is detected indirectly. Another form of indirect detection involves using a primary or secondary antibody that is labeled with an affinity tag such as biotin. Then a secondary (or tertiary) probe, such as streptavidin that is labeled with the detectable enzyme or fluorophore tag, can be used to probe for the biotin tag to yield a detectable signal. Several variants of these probing and detection strategies exist. However, each one depends on a specific probe (e.g., a primary antibody) whose presence is linked directly or indirectly to some sort of measurable tag (e.g., an enzyme whose activity can produce a colored product upon reaction with its substrate).

Usually, a primary antibody without a detectable label and some sort of secondary (indirect) detection method is required in assay methods. Nevertheless, nearly any antibody can be labeled with biotin, HRP enzyme or one of several fluorophores if needed. Most primary antibodies are produced in mouse, rabbit or one of several other species. Nearly all of these are antibodies of the IgG class. Therefore, it is relatively easy and economical for manufacturers to produce and supply ready-to-use, labeled secondary antibodies for most applications and detection systems. Even so, several hundred options are available, differing in the level of purity, IgG- and species-specificity, and detection label. The choice of secondary antibody depends upon the species of animal in which the primary antibody was raised (the host species). For example, if the primary antibody is a mouse monoclonal antibody then the secondary antibody must be an anti-mouse antibody obtained from a host other than the mouse.

With biotin-binding proteins as probes, the highly specific affinity interaction between biotin and avidin or streptavidin protein is the basis for many kinds of detection and affinity-purification methods. Biotin is very small (244 Daltons), so its covalent attachment to antibodies or other probes rarely interferes with their functions. Yet its presence as a label on a probe allows efficient and specific secondary detection with either avidin or streptavidin. Both kinds of biotin-binding proteins are available in purified forms labeled with enzymatic or fluorescent tags that enable detection in many kinds of assays systems.

Enzymatic labels are most commonly used as secondary antibody (or streptavidin) tags for detection in blotting and immunoassays. Enzymes provide detectable signal via their activity; reaction with a specific substrate chemical yields a colored, light-emitting, or fluorescent product. While reporter enzymes like beta-galactosidase and luciferase have been successfully used to make probes, alkaline phosphatase (AP) and horseradish peroxidase (HRP) are the two enzymes used most extensively as labels for protein detection. An array of chromogenic, fluorogenic and chemiluminescent substrates is available for use with either enzyme.

Alkaline phosphatase, usually isolated from calf intestine, is a large (140 kDa) protein that catalyzes the hydrolysis of phosphate groups from a substrate molecule resulting in a colored or fluorescent product or the release of light as a byproduct of the reaction. AP has optimal enzymatic activity at a basic pH (pH 8-10) and can be inhibited by cyanides, arsenate, inorganic phosphate and divalent cation chelators, such as EDTA. As a label for Western blotting, AP offers a distinct advantage over other enzymes. Because its reaction rate remains linear, detection sensitivity can be improved by simply allowing a reaction to proceed for a longer time period.

Horseradish peroxidase is a 40 kDa protein that catalyzes the oxidation of substrates by hydrogen peroxide, resulting in a colored or fluorescent product or the release of light as a byproduct of the reaction. HRP functions optimally at a near-neutral pH and can be inhibited by cyanides, sulfides and azides. Antibody-HRP conjugates are superior to antibody-AP conjugates with respect to the specific activities of both the enzyme and antibody. In addition, its high turnover rate, good stability, low cost and wide availability of substrates makes HRP the enzyme of choice for most applications. Because of the small size of the HRP enzyme, further increases in sensitivity may be achieved by using poly-HRP conjugated secondary antibodies and may eliminate the need for using ABC type amplification systems for some researchers.

Fluorescent Labels for Detection were historically used in a small number of cell biology applications such as flow cytometry (FC), fluorescence-activated cell sorting (FACS) and immunohistochemistry (IHC) using fluorescence microscopy. Until recently, the two most common fluorophores for labeling probes were fluorescein (fluorescein isothiocyanate, FITC) and rhodamine (tetramethyl rhodamine isothiocyanate, TRITC). Other labels include fluorescent proteins such as the various forms of green fluorescent protein (GFP) and the phycobiliproteins (allophycocyanin, phycocyanin, phycoerythrin and phycoerythrocyanin). While having the ability to produce an intense fluorescent signal for detection, fluorescent proteins can be difficult to optimize for conjugation purposes and may create steric hindrance or background signal issues in binding assays.

The use of fluorophore-conjugated probes in blotting and immunoassays requires fewer steps compared to the use of enzymatic labels because there is no substrate development step to perform. While the protocol is shorter, fluorescent detection requires special equipment and the sensitivity is not a high as that which can be obtained with enzymatic chemiluminescent systems. Although not as sensitive as enzymatic detection, fluorescent detection methods reduce chemical waste and have the added advantage of multiplex compatibility (using more than one fluorophore in the same experiment).

Alternatively or additionally, two markers may be used in order to detect proximity of two substances, e.g. the test compound or the known Slfn2 ligand and the Slfn2 protein. Papain and cathepsin L and H are known ligands for Slfn2. The markers may be, e.g. one radioactive or fluorescent marker and one scintillator (e.g. for a scintillation proximity assay) or two fluorescent markers may be used (e.g. for FRET). In one example the Slfn2 protein and the test substance could be labeled with a first and a second marker. In case the test substance is bound to the protein, and the labels are therefore in close proximity, energy could be transferred from the first to the second label, thus detecting the interacting of Slfn2 protein and test substance. This test could be designed as a competition binding test, wherein a known Slfn2 ligand carries one of the labels.

Examples of suitable marker combinations include
- radiolabels ³H, ³³P, ³⁵S or ¹⁴C, ¹²⁵I combined with scintillator such as Yttrium silicate or polyvinyl-toluene, e.g. compartmented in a microparticle or
- a donor fluorescent markers such as fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives combined with a acceptor fluorescent marker such as LC-Red 610, LC -Red 640, LC-Red 670, LC -Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium).

As an alternative to the detection by antibodies the method of the invention could be designed as a competition binding experiment, in which the displacement of the binding of a known Slfn2 ligand from Slfn2 by a test substance is studied. Successful displacement of the known ligand from the protein is an indicator for binding of the test substance to the protein. In this approach, it is advantageously to label the known Slfn2 ligand which allows for convenient testing of multiple test compounds (e.g. of a library), whereby not each of the test compounds needs to be labeled.

A ligand is a substance that is able to bind to and form a complex with a biomolecule, herein e.g. Slfn2 protein or nucleic acid. It is a molecule binding to a site on the biomolecule, by intermolecular forces such as ionic bonds, hydrogen bonds and Van der Waals forces. The docking (association) is usually reversible (dissociation). Actual irreversible covalent binding between a ligand and its target molecule is rare in biological systems. Ligand binding to a biomolecule may alter its activity, e.g. its ability to activate downstream signal transduction. Ligands include inhibitors and activators.

Inhibitors are molecules that bind to enzymes and decrease their activity. Since blocking an enzyme's activity can correct a metabolic imbalance, many drugs are enzyme inhibitors. Not all molecules that bind to enzymes are inhibitors; enzyme activators bind to enzymes and increase their enzymatic activity.

The binding of an inhibitor can stop a binding partner from interacting with the biomolecule and/or hinder the biomolecule from being active or activated. Inhibitor binding is either reversible or irreversible. Irreversible inhibitors usually react with the biomolecule and change it chemically. These inhibitors may e.g. modify key amino acid residues needed for the activity. In contrast, reversible inhibitors bind non-covalently and different types of inhibition are produced depending on whether these inhibitors bind the biomolecule.

Selective ligands have a tendency to bind to very limited types of targets (biomolecules) such as enzymes, while non-selective ligands bind to several types of targets. This plays an important role in pharmacology, where drugs that are non-selective tend to have more adverse effects, because they bind to several other biomolecules in addition to the one generating the desired effect.

For competition binding experiments a known ligand is labeled with at least one detectable marker and added to the incubation step of b). After step b) bound labeled ligand is separated from non-bound ligand. The separation may be done by a common separation step such as filtration, centrifugation, immobilization, phase separation and removal of liquids etc. The amount of signal provided by the label is indicative for the amount of ligand bound and therefore also for the amount of test compound bound to the biomolecule, as ligand and test compound compete for the binding to the biomolecule.

In an embodiment the assay for detection the effect of the test compound is an SPA (scintillation proximity assay), a FRET (fluorescence resonance energy transfer) assay, TR-FRET (time-resolved fluorescence resonance energy transfer) assay or a FP (fluorescence polarisation) assay.

SPA (scintillation proximity assay) is a type of technology that is used for biochemical screening which permits the rapid and sensitive measurement of a broad range of processes biologically in a homogeneous system. The type of beads that is involved in the SPA are microscopic in size and within the beads itself, there is a scintillant which emits light when it is stimulated. Stimulation occurs when radio-labeled molecules interact with the bead. This interaction will trigger the bead to emit light, which can be detected using scintillation counters.

In more detail, when the radio-labeled molecule is attached or is in close proximity to bead, light emission is stimulated. However, if the bead remains unbounded by the radio-labeled molecule, the bead will not be stimulated to emit light. This is due to the fact that the energy released from the unbounded radioactivity is too dissolute when it is too far from the SPA bead, hence the beads not being stimulated to produce a signal.

Tritium is highly recommended as it suits SPA very well. It is due to the 1.5 µm path length through water, which is very short. So, when the ß-particle is within that particular range of 1.5 µm with the scintillant bead, there is sufficient energy to stimulate the scintillant bead to emit light. If the distance between the greater than 1.5 µm, then the ß-particle is incapable of traveling the required distance to stimulate the bead as there is insufficient energy. There is also an assortment of bead coatings available that allows this method to be applied to a broad range of applications, such as enzyme assays and radio-immuno assays.

Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically <10 nm). As both molecules are fluorescent, the energy transfer is often referred to as "fluorescence resonance energy transfer", although the energy is not actually transferred by fluorescence. FRET is a useful tool to detect and quantify protein-agent interactions, protein-protein interactions, protein-DNA interactions, and protein-conformational changes. For monitoring binding of a protein to an agent, one protein to another or a protein to DNA, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Suitable neighbors for FRET are known in the art and the skilled practitioner will be able to choose a suitable combination of labels for both antibodies. As used herein with respect to donor and corresponding acceptor, "corresponding" refers to an acceptor fluorescent moiety having an emission spectrum that overlaps with the excitation spectrum of the donor. However, both signals should be separable from each other. Accordingly, the wavelength maximum of the emission spectrum of the acceptor should preferably be at least 30 nm, more preferably at least 50 nm, such as at least 80 nm, at least 100 nm or at least 150 nm greater than the wavelength maximum of the excitation spectrum of the donor.

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostil-bene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC - Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO). Alternatively, time-resolved fluorescence resonance energy transfer (TR-FRET) may be used for the test system of the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro- to millisecond measurements. Fluorescence lanthanide chelates have been used as energy donors in the late seventies. The commonly used lanthanides include samarium (Sm), europium (Eu), terbium (Tb) and dysprosium (Dy). Because of their specific photophysical and spectral properties, complexes of lanthanides are of major interest for fluorescence application in biology. Specifically, they have a large stroke's shift and extremely long emission half-lives (from microseconds to milliseconds) when compared to more traditional fluorophores.

Usually, organic chromophores are used as acceptors. These include allophycocyanin (APC). Suitable details on TR-FRET as well as acceptors are described in WO 98/15830.

Fluorescence polarisation (FP)-based assays are assays which use polarized light to excite fluorescent substrate in solution. These fluorescent substrates are free in solution and tumble, causing the emitted light to become depolarized. When the substrate binds to a larger molecule, i.e. the acyl group, its tumbling rates are greatly decreased, and the emitted light remains highly polarized.

Alternatively, mass spectrometry may be used. The term "mass spectrometry (MS)" refers to the use of an ionization source to generate gas phase ions from a sample on a surface and detecting the gas phase ions with a mass spectrometer. The term "laser desorption mass spectrometry" refers to the use of a laser as an ionization source to generate gas phase ions from a sample on a surface and detecting the gas phase ions with a mass spectrometer. A preferred method of mass spectrometry for biomolecules such as acylated acyl acceptor is matrix-assisted laser desorption/ionization mass spectrometry or MALDI. In MALDI, the analyte is typically mixed with a matrix material that, upon drying, co-crystallizes with the analyte. The matrix material absorbs energy from the energy source which otherwise would fragment the labile biomolecules or analytes. Another preferred method is surface-enhanced laser desorption/ionization mass spectrometry or SELDI. In SELDI, the surface on which the analyte is applied plays an active role in the analyte capture and/or desorption. In the context of the invention the sample comprises a biological sample that may have undergone chromatographic or other chemical processing and a suitable matrix substrate.

A further preferred method of mass spectrometry is electrospray ionization mass spectrometry (ESI-MS) or electron capture dissociation mass spectrometry (ECD-MS).

Tandem mass spectrometry (MS/MS) is also preferably used. A tandem mass spectrometer is one capable of multiple rounds of mass spectrometry, usually separated by some form of molecule fragmentation. For example, one mass analyzer can isolate one peptide from many entering a mass spectrometer. A second mass analyzer then stabilizes the peptide ions while they collide with a gas, causing them to fragment by collision-induced dissociation (CID). A third mass analyzer then sorts the fragments produced from the peptides. Tandem MS can also be done in a single mass analyzer over time, as in a quadrupole ion trap. There are various methods for fragmenting molecules for tandem MS, including collision-induced dissociation (CID), electron capture dissociation (ECD), electron transfer dissociation (ETD), infrared multiphoton dissociation (IRMPD), blackbody infrared radiative dissociation (BIRD), electron-detachment dissociation (EDD) and surface-induced dissociation (SID).

Mass spectrometry (MS), such as tandem mass spectrometry (MS/MS), can also be combined with chromatography. Thus, one preferred method is liquid chromatography-mass spectrometry (LC-MS, or alternatively HPLC-MS), preferably liquid chromatography-tandem mass spectrometry (LC-MS/MS, or alternatively HPLC-MS/MS). Liquid chromatography-mass spectrometry is an analytical chemistry technique that combines the physical separation capabilities of liquid chromatography (LC, or alternatively HPLC) with the mass analysis capabilities of mass spectrometry (MS). LC-MS is a powerful technique as it has very high sensitivity and selectivity. Generally its application is oriented towards the specific detection and potential identification of chemicals in the presence of other chemicals, e.g. in a complex mixture.

In mass spectrometry the "apparent molecular mass" refers to the molecular mass (in Daltons)-to-charge value, m/z, of the detected ions. How the apparent molecular mass is derived is dependent upon the type of mass spectrometer used. With a time-of-flight mass spectrometer, the apparent molecular mass is a function of the time from ionization to detection. The term "signal" refers to any response generated by a biomolecule under investigation. For example, the term signal refers to the response generated by a biomolecule hitting the detector of a mass spectrometer. The signal intensity correlates with the amount or concentration of the biomolecule. The signal is defined by two values: an apparent molecular mass value and an intensity value generated as described. The mass value is an elemental characteristic of the biomolecule, whereas the intensity value accords to a certain amount or concentration of the biomolecule with the corresponding apparent molecular mass value. Thus, the "signal" always refers to the properties of the biomolecule.

In mass spectrometry-based proteomics relative quantification may be used to compare protein abundance between samples. This can be achieved by labelling one sample with stable isotopes alone or incorporated into protein crosslinkers like D4-BS3, which leads to a mass shift in the mass spectrum. Differentially labelled samples are combined and analyzed together. The differences in the peak intensities of the isotope pairs accurately reflect difference in the abundance of the corresponding proteins. Well-established methods comprise isotope-coded affinity tags (ICAT), isobaric labelling, label-free quantification, metal-coded tags (MeCATs), N-terminal labelling, or stable isotope labelling with amino acids in cell culture (SILAC). It is preferred that the mass spectrometry, preferably the tandem mass spectrometry, employs tandem mass tags (TMT), isobaric tags for relative and absolute quantitation (iTRAQ), or isotope-coded affinity tags (ICATs).

The effect of the test compound on the Slfn 2 nucleic acid may be determined on a variety of expression or signal transduction levels.

The test compound could be designed to bind to a regulatory sequence of the Slfn2 gene (e.g. the SIfn2 promoter or a fragment thereof, e.g. as disclosed herein) or the SIfn2 gene itself. Thereby, the test compound could have an influence on the expression of the gene.

Accordingly, the binding of test compound to the regulatory sequence could be determined by detecting the complex of (i) the regulatory sequence (or fragment thereof) or gene and (ii) the test compound. Suitable methods of detecting complexes of two or more components are detailed herein.

The regulatory sequence is a segment of DNA where regulatory proteins such as transcription factors bind preferentially. These regulatory proteins bind to short stretches of DNA called regulatory regions, which are appropriately positioned in the genome, usually a short distance 'upstream' of the gene being regulated. In binding, the regulatory proteins can recruit another protein complex, called the RNA polymerase. In this way, they control gene expression. The regulatory sequence includes the promoter region which usually works in concert with other regulatory regions (enhancers, silencers, boundary elements/insulators) to direct the level of transcription of a given gene.

Alternatively, the effect, e.g. binding, of the test compound and the influence on the gene transcription could be detected indirectly. For this, the effect downstream the Slfn2 gene could be detected. For example, the effect on the transcription and translation related to SIfn2 could be determined. In one embodiment, the amount of SIfn2 mRNA or SIfn2 protein is detected.

Suitable methods of detecting mRNA are include e.g. Northern blot analysis, nuclease protection assays (NPA), in situ hybridization, and reverse transcription-polymerase chain reaction (RT-PCR).

For the Northern blotting procedure, RNA samples may be first separated by size via electrophoresis in an agarose gel under denaturing conditions. The RNA is then transferred to a membrane, crosslinked and hybridized with a labeled probe. Nonisotopic or high specific activity radio labeled probes can be used including random-primed, nick-translated, or PCR-generated DNA probes, in vitro transcribed RNA probes, and oligonucleotides. Additionally, sequences with only partial homology (e.g., cDNA from a different species or genomic DNA fragments that might contain an exon) may be used as probes.

The Nuclease Protection Assay (NPA) is an extremely sensitive method for the detection and quantitation of specific mRNAs. The basis of the NPA is solution hybridization of an antisense probe (radio labeled or non-isotopic) to an RNA sample. After hybridization, single-stranded, unhybridized probe and RNA are degraded by nucleases. The remaining protected fragments are separated e.g. on an acrylamide gel. Solution hybridization is typically more efficient than membrane-based hybridization, and it can accommodate up to 100 µg of sample RNA, compared with the 20-30 µg maximum of blot hybridizations. NPAs are also less sensitive to RNA sample degradation than Northern analysis since cleavage is only detected in the region of overlap with the probe (probes are usually about 100-400 bases in length).

In RT-PCR, an RNA template is copied into a complementary DNA (cDNA) using a retroviral reverse transcriptase. The cDNA is then amplified exponentially by PCR. Relative quantitative RT-PCR involves amplifying an internal control simultaneously with the gene of interest. The internal control is used to normalize the samples. Once normalized, direct comparisons of relative abundance of a specific mRNA can be made across the samples. Competitive RT-PCR is used for absolute quantitation. This technique involves designing, synthesizing, and accurately quantitating a competitor RNA that can be distinguished from the endogenous target by a small difference in size or sequence. Known amounts of the competitor RNA are added to experimental samples and RT-PCR is performed. Signals from the endogenous target are compared with signals from the competitor to determine the amount of target present in the sample.

The above methods may include nucleic acid labeling. A series of techniques are known to the skilled person allowing for labeling of DNA, RNA or oligonuleotides. These include for example Nick translational labeling, random primed DNA labeling, PCR labeling of DNA probes and oligonucleotide 3'/5' end labeling, transcriptional labeling of RNA probes, oligonucleotide 3'/5' end labeling and oligonucleotide tailing.

The nick translation method is based on the ability of DNase I to introduce randomly distributed nicks into DNA. DNA polymerase I synthesizes DNA complementary to the intact strand in a 5' → 3' direction using the 3'-OH termini of the nick as a primer. The 5' → 3' exonucleolytic activity of DNA Polymerase I simultaneously removes nucleotides in the direction of synthesis. The polymerase activity sequentially replaces the removed nucleotides with isotope-labeled or hapten-labeled deoxyribonucleoside triphosphates. At low temperature (15°C), the unlabeled DNA in the reaction is thus replaced by newly synthesized labeled DNA. Common labels include digoxigenin-, biotin-, or fluorochromes such as fluorescein or tetramethylrhodamin.

The method of "random primed" DNA labeling is based on the hybridization of a mixture of all possible hexanucleotides to the DNA to be labeled. All sequence combinations are represented in the hexanucleotide primer mixture, which leads to binding of primer to the template DNA in a statistic manner. Thus an equal degree of labeling along the entire length of the template DNA is guaranteed. The complementary strand is synthesized from the 3' OH termini of the random hexanucleotide primer using Klenow enzyme, labeling grade. Modified deoxyribonucleoside triphosphates (e.g. [³²P]-, [³⁵S]-, [³H]-, [¹²⁵I]-, digoxigenin- or biotin-labeled) present in the reaction are incorporated into the newly synthesized complementary DNA strand.

The polymerase chain reaction (PCR) allows the amplification of minute amounts of DNA. The only prerequisite is that some sequence information of the target sequence is known for synthesizing the appropriate primers. The combination of labeling with PCR is a powerful tool for the analysis of PCR products, and also for the preparation of labeled probes from small amounts of a respective target sequence. For example digoxigenin, a steroid hapten, may be used to label DNA, RNA, or oligonucleotides for hybridization, and subsequent color- or luminescent detection. The digoxigenin is usually coupled to dUTP via an alkali-labile ester bond. The labeled dUTP can be easily incorporated by enzymatic nucleic-acid synthesis using DNA polymerases.

Oligonucleotides may enzymatically be labeled at their 3'-end with terminal transferase either by incorporation of a label such as single digoxigenin-labeled dideoxyuridine-triphosphate (DIG-ddUTP) or by the addition of a longer nucleotide tail. Terminal Transferase catalyzes the template independent addition of deoxy- and dideoxynucleoside triphosphates to the 3'OH ends of double and single-stranded DNA fragments and oligonucleotides. Terminal transferase incorporates digoxigenin-, biotin-, and fluorochrome-labeled deoxy- and dideoxynucleotides as well as radioactive labeled deoxy-and dideoxynucleotides. Alternatively or additionally, oligonucleotides may be labeled at the 5'-terminus, e.g. by reacting with a phosphoramidite in a final step according to the classical solid phase phosphoramidite synthesis method. By this process a 5'-terminal amino function is created. Treatment with ammonia releases the oligonucleotide from the support and cleaves the protecting groups. In the subsequent step the digoxigenin moiety is introduced at the 5'-position.

Different labels are known which may be used in the above labeling methods. Some of them including their detection are exemplarily described in the following:
Biotin-labeled compounds can be detected for example by anti-biotin antibodies or by streptavidin conjugates. Anti-biotin antibodies (e.g. monoclonal anti-biotin antibody or Fab-fragment, conjugated with alkaline phosphatase (AP)) may be used in the detection of biotin-labeled nucleic acids by enzyme immunoassay with luminescence on nylon membranes. This method of detection may be employed for detection of biotin labeled nucleic acids on membranes (e.g. Southern blots, dot blots), in cells and tissues (e.g. *in situ* hybridization), immunoblotting, immunohistochemistry or ELISA. Streptavidin conjugates are used for the detection of biotin-labeled substances (*e.g*., biotinylated antibodies) which can be used for several immunological detection systems. For this, streptavidin e.g. from Streptomyces avidinii could be coupled to alkaline phosphatase or to ß-peroxidase. This method of detection may be employed with immunoblotting, immunohistochemistry or ELISA.

Probe-target hybrids may be detected with an enzyme-linked immunoassay. This immunochemical detection step is usually more sensitive than radioactive detection procedures. In this assay, the membrane may be blocked to prevent non-specific interaction of the antibody with the filter. Alkaline phosphatase-conjugated antibody, specific for digoxigenin, recognizes the digoxigenin molecule on the labeled hybrid. Addition of an alkaline phosphatase substrate allows the visualization of the hybrids.

For chemiluminescence detection, suitable substrates for alkaline phosphatase such as disodium 3-(4-methoxyspiro {1,2-dioxetane-3,2-(5-chloro)tricyclo [3.3.1.1^{3,7}]decan}-4-yl)phenyl phosphate or disodium 4-chloro-3-(methoxyspiro {1,2-dioxetane-3,2-(5-chloro)tricyclo [3.3.1.1^{3,7}]decan}-4-yl)phenyl phosphate belong to the group of the dioxetane phenyl phosphates. Upon dephosphorylation by alkaline phosphatase, an intermediate is formed whose decomposition results in light emission which can be recorded e.g. on X-ray film.

Colorimetric detection of DIG-labeled probes is usually performed with colorless substrates which form a redox system. Examples are like 5-bromo-4-chloro-3-indolyl-phosphate and 4-Nitro-blue-tetrazolium-chloride. 5-bromo-4-chloro-3-indolyl-phosphate is oxidized by the alkaline phosphatase to indigo by release of a phosphate group. In parallel, 4-Nitro-blue-tetrazolium-chloride is reduced to diformazan. The reaction products form a water insoluble dark blue to brownish precipitate, depending on the type of membrane.

Various reporter molecules can be coupled to detecting antibodies to visualize the specific probe-target hybridization including, but not limited to, enzyme-coupled antibodies, fluorochrome-labeled antibodies (detection by fluorescent microscope and specific filters which allow visualization of the wavelength emitted by the fluorescent dye) and antibodies coupled to colloidal gold (detection by electron microscope on cryostatic sections).

Multiple simultaneous hybridizations can be performed by using combinations of digoxigenin-, biotin- and fluorochrome-labeled probes to localize different chromosomal regions or different RNA sequences in one preparation. Such multiprobe experiments are made possible by the availability of different fluorescent dyes coupled to antibodies. These include fluorescein or FITC (fluorescein isothiocyanate; yellow), rhodamine or TRITC (tetramethylrhodamine isothiocyanate; red) and AMCA (amino-methylcoumarin acetic acid; blue).

The effect on the regulatory sequence could also by detected by attaching the regulators sequence to a reporter gene and introducing the resulting DNA construct into a cell or organism. For bacteria or eukaryotic cells in culture, this is usually in the form of a circular DNA molecule called a plasmid. It is important to use a reporter gene that is not natively expressed in the cell or organism under study, since the expression of the reporter is being used as a marker for successful uptake of the gene of interest. Commonly used reporter genes that induce visually identifiable characteristics usually involve fluorescent and luminescent proteins; examples include the gene that encodes jellyfish green fluorescent protein (GFP), which causes cells that express it to glow green under blue light, the enzyme luciferase, which catalyzes a reaction with luciferin to produce light, and the red fluorescent protein from the gene dsRed. Another common reporter in bacteria is the lacZ gene, which encodes the protein β-galactosidase. This enzyme causes bacteria expressing the gene to appear blue when grown on a medium that contains the substrate analog X-gal (an inducer molecule such as IPTG is also needed under the native promoter). An example of a selectable-marker reporter in bacteria is the chloramphenicol acetyltransferase (CAT) gene, which confers resistance to the antibiotic chloramphenicol. The influence of a test compound may be detected by the determining the amount of the above signal relative to a control.

Test systems comprise methods for detecting an effect on the Slfn2 protein.

Suitable methods for detecting a protein are described herein and include e.g. detection of a labeled protein (such as a fusion protein comprising a detectable marker, tag or enzyme component), protein immunostaining, protein immunoprecipitation, immunoelectrophoresis, immunoblotting, Western blotting, spectrophotometry, enzyme assays etc.. The method may require protein purification prior to the detection, which could involve protein isolation (e.g. by chromatography methods, protein extraction, protein solubilization, gel electrophoresis, and electrofocusing).

Protein immunostaining is an antibody-based method to detect a specific protein in a sample. The term immunostaining was originally used to refer to the immunohistochemical staining of tissue sections. Now however, immunostaining encompasses a broad range of techniques used in histology, cell biology, and molecular biology that utilize antibody-based staining methods. Immunohisto- or -cytochemistry of tissue sections or cells which are preserved by fixation.

While the first cases of IHC staining used fluorescent dyes, other non-fluorescent methods using enzymes such as peroxidase and alkaline phosphatase are now used more often. These enzymes are capable of catalyzing reactions that give a coloured product that is easily detectable by light microscopy. Alternatively, radioactive elements can be used as labels, and the immunoreactions can be visualized by autoradiography. Tissue preparation or fixation is essential for the preservation of cell morphology and tissue architecture. Inappropriate or prolonged fixation may significantly diminish the antibody binding capability. Many antigens can be successfully demonstrated in formalin-fixed paraffin-embedded tissue sections. Optimization of fixation methods and times, pre-treatment with blocking agents, incubating antibodies with high salt, and optimizing post-antibody wash buffers and wash times may be important for obtaining high quality immunostaining.

Western blotting allows the detection of specific proteins (native or denatured) from extracts made from cells or tissues, before or after any purification steps. Proteins are generally separated by size using gel electrophoresis before being transferred to a synthetic membrane (typically nitrocellulose or PVDF) via dry, semi-dry, or wet blotting methods. The membrane can then be probed using antibodies using methods similar to immunohistochemistry, but without a need for fixation. Detection is typically performed using peroxidase linked antibodies to catalyze a chemiluminescent reaction. Western blotting is a routine molecular biology method that can be used to semi quantitatively or quantitatively compare protein levels between extracts. The size separation prior to blotting allows the protein molecular weight to be gauged as compared with known molecular weight markers. Western blotting is an analytical technique used to detect specific proteins in a given sample of tissue homogenate or extract. It uses gel electrophoresis to separate proteins by the length of the polypeptide (denaturing conditions) or by the 3-D structure of the protein (native/ non-denaturing conditions).

The enzyme-linked immunosorbent assay or ELISA is a diagnostic method for quantitatively or semi-quantitatively determining protein concentrations from blood plasma, serum or cell/tissue extracts in a multi-well plate format (usually 96-wells per plate). Broadly, proteins in solution are adsorbed to ELISA plates. Antibodies specific for the protein of interest are used to probe the plate. Background is minimized by optimizing blocking and washing methods (as for IHC), and specificity is ensured via the presence of positive and negative controls. Detection methods are usually colorimetric or chemiluminescence based.

Electron microscopy or EM can be used to study the detailed micro architecture of tissues or cells. Immuno-EM allows the detection of specific proteins in ultrathin tissue sections. Antibodies labeled with heavy metal particles (e.g. gold) can be directly visualized using transmission electron microscopy. While powerful in detecting the subcellular localization of a protein, immuno-EM can be technically challenging, expensive, and require rigorous optimization of tissue fixation and processing methods.

The effect of the test compound on Slfn2 protein could be detected indirectly, e.g. by detecting downstream effects such as phenotypic assays (e.g. determination of algesia or analgesia phenotype).

The test system can e.g. be a biochemical or a cellular (cell-based) test system. A cell-based system can be advantageous, because it allows for easy amplification of the test system by propagating the cells and cellular mechanisms, e.g. signal transduction components downstream or upstream of Slfn2 protein or gene, as these may be used in order to detect a signal indicative for a pain stage of a cell.

The term "cell" herein refers to any prokaryotic or eukaryotic cell. In the context of reporter assays, the cell can be any cell capable of being transfected with a nucleic acid vector and of expressing a reporter gene. These comprise principally primary cells and cells from a cell culture, preferably a eukaryotic cell culture comprising cells derived either from multicellular organisms and tissue (such as HeLA, CHO, COS, SF9 or 3T3 cells) or single cell organisms such as yeast (e.g. s. pombe or s. cerevisiae), or a prokaryotic cell culture, preferably Pichia or E.coli. Cells and samples derived from tissue can be gained by well-known techniques, such as taking of blood, tissue punction or surgical techniques.

Cells that are cultured directly from an animal or a person are known as primary cells. With the exception of some cell lines derived from tumors, most primary cell cultures have limited lifespan. After a certain number of population doublings cells undergo the process of senescence and stop dividing, while generally retaining viability.

An established or immortalized cell line has acquired the ability to proliferate indefinitely either through random mutation or deliberate modification, such as artificial expression of the telomerase gene. There are numerous well established cell lines representative of particular cell types and it is within the knowledge of the skilled person to select a suitable cell line.

Accordingly, in a preferred embodiment of the invention the cell is a cell line. A cell line is a population of cells propagated in culture that are derived from, and therefore genetically identical to, a single common ancestor cell. Preferred cell lines are HEK 293 cells (primary human embryonic kidney), 3T3 cells (murine embryonic fibroblasts), CHO cells (Chinese hamster ovary), COS-7 cells (African green monkey cell line), HeLa cells (human epithelioid cervical carcinoma), JURKAT cells (human T-cell leukemia), BHK 21 cell (hamster normal kidney, fibroblast), and MCF-7 cells (human breast cancer).

The cell or cell line may be genetically modified to include Slfn2 or components needed for detection of an effect. A particularly preferred cell line encompasses a gene coding for Slfn2 under the control of a known promoter system. The promoter system may be controllable, e.g. inducible by a chemical, or constitutively active. Those promoter systems are well known to the skilled person.

Examples of cells suitable in the context of the present invention include without limitation L6 cells, 3T3 adipocytes, HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926, F98, GH3, GP&envAM12, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, PC12, NG108, N1E, NB41A3, SKNBE2, CHP212, SKNDZ, IMR32, SKNAS, SKNFI, D341, D283, Daoy, PFSK1, TE671, C6, F98, DITNC1, M059K, Hs683, A172, DBTRG05MG, Hs294T, C1300, NG108, primary neuronal cells, primary dendritic cells, primary human myoblasts, primary keratinocytes, SF9, SK-UT-1, ST, SW 480, SWU-2 OS, U-373, U-937, and Y-1. Other suitable cells (e.g. single-cell organisms such as yeast (e.g. s. cerevisiae or s. pombe) are known to the one of skill in the art. Different neural cell lines which may be especially useful in the context of present invention and which are also listed above and their culture conditions are described in "Neural Cell Lines", Murayama et al., Protocols for Neural Cell Culture, 3rd Edidion, Humana Press, Inc., 2001, p.219-228. Preferred cells comprise cells of neural origin, such as P19, PC12, NG108, N1E, NB41A3, SKNBE2, CHP212, SKNDZ, IMR32, SKNAS, SKNFI, D341, D283, Daoy, PFSK1, TE671, C6, F98, DITNC1, M059K, Hs683, A172, DBTRG05MG, Hs294T, C1300, NG108, primary neuronal cells, primary dendritic cells, and especially the cells of neuronal origin in this list: P19, PC12, NG108, N1E, NB41A3, SKNBE2, CHP212, SKNDZ, IMR32, SKNAS, SKNFI, D341, D283, Daoy, PFSK1, TE671 or primary neuronal cells. Among the most preferred cell lines are PC12 (rat), which is a widely used model of neuronal cell function and differentiation, C1300 (mouse neuroblastoma) or N1E that is a clone of C1300, which was derived from a tumor of the spinal cord. However, the skilled artisan knows how to choose the suitable cell or cell line for a given method or use.

Alternatively, cell lysates (crude, fractionated or purified) may be used. Exemplary methods for producing these are known to the skilled person and may include fragmentation, centrifugation and resuspending.

Moreover, whole organisms, e.g. simple organisms such as c. elegans or developmental stages of simple organisms such as egg or larvae of drosophila may be used for the different test methods of present invention, wherein simple organisms or developmental stages thereof may also be subject to HTS (e.g. c. elegans).

In a preferred embodiment of the present invention the method is a high-through-put screening method. High-throughput screening (HTS) is a method for scientific experimentation especially used in drug discovery and relevant to the fields of biology and chemistry. Using for example robotics, data processing and control software, liquid handling devices, and sensitive detectors, High-Throughput Screening or HTS allows a researcher to quickly conduct thousands or even millions of biochemical, genetic or pharmacological tests. Through this process one can rapidly identify active compounds, antibodies or genes which modulate a particular biomolecular pathway.

Usually, HTS uses automation to run a screen of an assay against a library of candidate compounds. Typical HTS screening libraries or "decks" can contain from 100,000 to more than 2,000,000 compounds.

Most often, the key testing vessel of HTS is the multi-well plate or microplate. Modern microplates for HTS generally have either 96, 384, 1536, or 3456 wells. These are all multiples of 96, reflecting the original 96 well microplate with 8x12 9mm spaced wells. Most of the wells contain experimentally useful matter, often an aqueous solution of dimethyl sulfoxide (DMSO) and some other chemical compound, the latter of which is different for each well across the plate. The other wells may be empty, intended for use as optional experimental controls.

To prepare for an assay, the researcher fills each well of the plate with some biological entity that he or she wishes to conduct the experiment upon. In the present case the test system comprising a Slfn2 nucleic acid or protein is to be filled in. After some incubation time has passed to allow the biological matter to absorb, bind to, or otherwise react (or fail to react) with the compounds in the wells, measurements are taken across all the plate's wells, either manually or by a machine. A specialized automated analysis machine can run a number of experiments on the wells (such as shining polarized light on them and measuring reflectivity, which can be an indication of protein binding). In this case, the machine may output the result of each experiment as a grid of numeric values, with each number mapping to the value obtained from a single well. A high-capacity analysis machine can measure dozens of plates in the space of a few minutes like this, generating thousands of experimental data points very quickly.

The term significant effect: If the test compound has a significant effect on the test system, the test compound is identified as compound involved in pain. For this, the effect of the test compound is compared to a control, particularly a negative control. A (statistically) significant effect in drug screening is e.g. an effect that is higher or lower than the mean value of the experiment and at the same time also higher or lower than the standard deviation (e.g. higher or lower than the mean value of an experiment (with double or triple determination of values in an experiment) plus/minus the 3-fold Standard Deviation (mean±3SD)). The skilled person knows how to discriminate significant effects from background noise or experimental deviations.

Controls are a part of the test methods, since they can eliminate or minimize unintended influences (such as background signals). Controlled experiments are used to investigate the effect of a variable on a particular system. In a controlled experiment one set of samples have been (or is believed to be) modified and the other set of samples are either expected to show no change (negative control) or expected to show a definite change (positive control). The control can be determined in one test run together with the test substance. It can be determined before of after determining the effect of the test compound or it may be a known value.

The test compound having an effect on the test system may result in changing, increasing or decreasing, the test system's signal. In the context of the present invention, the test compound has an effect in comparison to a control, if the test system contacted with the test compound produces a signal significantly lower or higher than that of a control (e.g. test system not contacted with the test compound). The person skilled in the art knows statistical procedures to assess whether two values are significantly different from each other such as Student's t-test or chi-square tests. Furthermore, the skilled person knows how to select a suitable control.

In a preferred embodiment, the signal of the test system is altered by the test compound by at least 10 %, preferably at least 25 %, more preferably at least 50 %, still more preferably at least 75 % and most preferably at least 90 % of the control, either positive or negative.

The term "compound" as used herein refers e.g. to a small molecule or a biomolecule.

The compound may be a protein, polypeptide, peptide, nucleic acid or small molecule such as a small organic molecule. Preferably, said compound is no compound which directly or indirectly influences, e.g. inhibits, the expression level of Slfn2, e.g. by binding to Slfn2 gene, Slfn2 mRNA, and/or Slfn2 protein. Thus, said compound is preferably a compound which is not involved in pain but is involved in another disease than pain. Accordingly, said compound is preferably a compound which is for treating, ameliorating or preventing another disease or symptom than pain.

In the context of the different aspects of present invention, the term "small molecule" refers to a low molecular weight organic compound which is by definition not a polymer. A small molecule may bind with high affinity to a biopolymer such as a protein or a nucleic acid and in addition may alter the activity or function of the biopolymer. The upper molecular weight limit for a small molecule is usually about 800 Daltons. This allows the diffusion of the small molecule across cell membranes so that it can reach intracellular sites of action.

It is preferred that the small molecule is derived from a library, e.g., a small molecule inhibitor library. Preferably, the small molecule is 800 daltons or less than 800 daltons in size, more preferably the small molecule is less than 500 daltons in size. Most preferably, the small molecule has a size of between 100 and 500 daltons. It is particularly preferred that the small molecule is a small organic molecule, more particularly a soluble, non-oligomeric, organic compound.

The compound involved in pain identified according to the different aspects of present invention could be used as a medicament. For the production of the medicament the identified target or its pharmaceutically acceptable salt has to be in a pharmaceutical dosage form in general consisting of a mixture of ingredients such as pharmaceutically acceptable carriers or auxiliary substances combined to provide desirable characteristics.

The formulation comprises at least one suitable pharmaceutically acceptable carrier or auxiliary substance. Examples of such substances are dematerialized water, isotonic saline, Ringer's solution, buffers, organic or inorganic acids and bases as well as their salts, sodium chloride, sodium hydrogen carbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvant such as gelatin, dextrin, cellulose and its derivatives, albumins, organic solvents, completing agents such as citrates and urea, stabilizers, such as protease or nuclease inhibitors, preferably apportioning, ε-aminocaproic acid or pepstatin A, preservatives such as benzyl alcohol, oxidation inhibitors such as sodium sulphite, waxes and stabilizers such as EDTA. Coloring agents, releasing agents, coating agents, sweetening, flavoring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the medicament is in general adjusted using a suitable organic or inorganic buffer, such as, for example, preferably using a phosphate buffer, tris buffer (tris(hydroxymethyl)aminomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicaments as well as suitable pharmaceutically acceptable carrier or auxiliary substance are well known to the one of skill in the art. Pharmaceutically acceptable carriers and auxiliary substances are a. o. chosen according to the prevailing dosage form and identified compound.

The pharmaceutical composition can be manufactured for oral, nasal, rectal, parenteral, vaginal, topic or vaginal administration. Parental administration includes subcutaneous, intracutaneous, intramuscular, intravenous, or intraperitoneal administration.

The medicament can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular patient will depend upon a variety of factors including the activity of the identified compound, the dosage form, the age, body weight and sex of the patient, the duration of the treatment and like factors well known in the medical arts.

The total daily dose of the compounds of this invention administered to a human or other mammal in single or in divided doses can be in amounts, for example, from about 0.01 to about 50 mg/kg body weight or more preferably from about 0.1 to about 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of the compounds of the present invention per day in single or multiple doses.

The term "adverse effect" (or side-effect) refers to a harmful and undesired effect resulting from a medication. An adverse effect may be termed a "side effect", when judged to be secondary to a main or therapeutic effect. Some adverse effects occur only when starting, increasing or discontinuing a treatment. Adverse effects may cause medical complications of a disease and negatively affect its prognosis. Examples of side effects are allergic reactions, vomiting, headache, or dizziness.

The term "kit" in the context of the present invention, is understood to be an article of manufacture comprising two or more of the components identified in this application, which are combined, coexisting spatially, to a functional unit, and which can contain further components. A kit according to present invention preferably comprises at least one component useful or necessary for performing one of the methods according to present invention.

The term "heterologous expression" or "overexpression" refers to the expression of a nucleic acid or protein that is driven by a nucleic acid (e.g. a vector) that has been introduced into a cell or organism either transiently or stably.

The terms "subject" or "individual" are used interchangeably herein. As used herein, an "individual" refers to a human or a non-human animal (e.g. a mammal, avian, reptile, fish, amphibian or invertebrate; preferably an individual that can either benefit from one of the different aspects of present invention (e.g. a method of treatment or a drug identified by present methods) or that can be used as laboratory animal for the identification or characterisation of a drug or a method of treatment. The individual can e.g. be a human, a wild-animal, domestic animal or laboratory animal; examples comprise: mammal, e.g. human, non-human primate (chimpanzee, bonobo, gorilla), dog, cat, rodent (e.g. mouse, guinea pig, rat, hamster or rabbit, horse, donkey, cow, sheep, goat, pig, camel; avian, such as duck, dove, turkey, goose or chick; reptile such as: turtle, tortoise, snake, lizard, amphibian such as frog (e.g. Xenopus laevis); fish such as koy or zebrafish; invertebrate such as a worm (e.g. c.elegans) or an insect (such as a fly, e.g. drosophila melanogaster). The term individual also comprises the different morphological developmental stages of avian, fish, reptile or insects, such as egg, pupa, larva or imago.

The individual may be an individual which is suspected to experience pain. The individual may be diagnosed and/or prognosed to experience pain. The individual as mentioned in the method above may also be an individual which is experiencing pain. The individual may be retested for experiencing pain and may be diagnosed and/or prognosed to still experiencing pain.

The term "sample", as used in the context of the different aspects of present invention, preferably refers to a biologically sample. The term "sample" or "sample of interest" are used interchangeably herein, referring to a small part intended to represent the whole of a tissue, an organ or an individual. Upon analysis a sample provides information about the tissue status or the health or diseased status of an organ or individual. Examples of samples include but are not limited to fluid samples such as cerebrospinal fluid, blood, serum, plasma, synovial fluid, urine, saliva, and lymphatic fluid, or solid samples such as biopsy samples, tissue, and tissue-extracts, e.g. taken from nervous tissue (e.g from the spinal cord), skin, muscle, cartilage, bone, synovium, perichondrium, capsule, and connective tissue. Further examples of samples are cell cultures or tissue cultures such as but not limited to cultures of neural cells.

Analysis of a sample may be accomplished on a visual or chemical basis. Visual analysis includes but is not limited to microscopic imaging or radiographic scanning of a tissue, organ or individual allowing for morphological evaluation of a sample. Chemical analysis includes but is not limited to the detection of the presence or absence of specific indicators or alterations in their amount or level. For example, a tissue sample may be removed from a subject by conventional biopsy techniques or a blood sample may be taken from a subject by conventional blood collection techniques. The sample, e.g. tissue or blood sample, may be obtained from a subject prior to initiation of the therapeutic treatment, during the therapeutic treatment, and/or after the therapeutic treatment, e.g. with a pain reducing compound.

It is preferred that the sample is a body fluid sample, a tissue sample, a cell colony sample, a single cell sample or a cell culture sample. More preferably, the tissue sample is a section or an explant sample, e.g. an explant sample of dorsal root ganglia or spinal cord. The term "body fluid sample" refers to a liquid sample derived from the body of a subject. Said body fluid sample may be a blood, urine, cerebrospinal fluid, cerumen (earwax), endolymph, perilymph, gastric juice, mucus, peritoneal fluid, pleural fluid, saliva, or sebum (skin oil) sample including components or fractions thereof. Said body fluid samples may be mixed or pooled. Thus, a body fluid sample may be a mixture of a blood sample and anurine sample or a mixture of a blood sample and cerebrospinal fluid sample. A "body fluid sample" may be provided by removing a body liquid from a subject, but may also be provided by using previously isolated body fluid sample material. Preferably, the blood sample of a subject is whole blood or a blood fraction such as serum or plasma. It is also particularly preferred to use blood cells also known as hemopoietic cells.

It is preferred that the tissue sample has a weight of between 0.1 and 500 mg, more preferably of between 0.5 and 250 mg, and most preferably of between 1 and 50 mg, i.e. 0.1, 0.2, 0.3, 0.4, 0.5, 0.6, 0.7, 0.8, 0.9, 1, 2, 2.5, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, or 500 mg.

It is also preferred that the cell sample (e.g. cell colony sample or cell culture sample) consists of between 100 and 1000 cells, more preferably of between 200 and 800 cells, and most preferably of between 400 and 600 cells.

It is further preferred that the body fluid sample has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. More preferably, the blood sample has a volume of between 0.1 and 20 ml, more preferably of between 0.5 and 10 ml, and most preferably of between 1 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

The term "reference", especially in the context of "reference individual", "reference sample" or "reference value" in the context of present invention refers to a comparison or standard that is characteristic or representative for a certain (health) status, disease etc. Thus, a reference value, is a standard value for a certain parameter (e.g. expression level of a certain indicator/biomarker molecule) that is typical for a certain status (e.g. a disease status or health status), a reference individual is an individual that has been selected for comparison and has a certain health state or disease, a reference sample can e.g. be a sample from a reference individual or an artificial sample with a characteristic level of a certain indicator or biomarker typical for a disease state or health state.

The term "reference sample" as used herein, refers to a sample which is analysed in a substantially identical manner as the sample of interest and whose information is compared to that of the sample of interest. A reference sample thereby provides a standard allowing for the evaluation of the information obtained from the sample of interest.

A reference sample may be derived from a healthy or normal tissue, organ or individual, thereby providing a standard of a healthy status of a tissue, organ or individual. Differences between the status of the normal reference sample and the status of the sample of interest may be indicative of the risk of disease development or the presence or further progression of such disease or disorder.

A reference sample may be derived from an abnormal or diseased tissue, organ or individual thereby providing a standard of a diseased status of a tissue, organ or individual. Differences between the status of the abnormal reference sample and the status of the sample of interest may be indicative of a lowered risk of disease development or the absence or bettering of such disease or disorder.

A reference sample may also be derived from the same tissue, organ, or individual as the sample of interest but has been taken at an earlier time point. Differences between the status of the earlier taken reference sample and the status of the sample of interest may be indicative of the progression of the disease, i.e. a bettering or worsening of the disease over time. A reference sample was taken at an earlier or later time point in case a period of time has lapsed between taking of the reference sample and taking of the sample of interest. Such period of time may represent years (e.g. 1, 2, 3, 4, 5, 10, 15, 20, 25, 30, 35, 40, 45, 50, 60, 70, 80, 90, 100 years), months (1, 2, 3, 4, 5, 6, 7, 8, 9, 10, 11, 12 months), weeks (e.g. 1, 2, 3, 4, 5, 6, 7, 8 weeks), days (e.g. 1, 2, 3, 4, 5, 10, 15, 20, 25, 30, 35, 40, 45, 50, 60, 70, 80, 90, 100, 200, 300, 400, 500 days), hours (1, 2, 3, 4, 5, 6, 7, 8, 9, 10, 11, 12 hours), minutes (e.g. 1, 2, 3, 4, 5, 10, 15, 20, 25, 30, 35, 40, 45, 50, 60 minutes), or seconds (e.g. 1, 2, 3, 4, 5, 10, 15, 20, 25, 30, 35, 40, 45, 50, 60 seconds).

The control sample representative for a status or stage of pain may be from a control subject known to experience pain. Preferably, the control sample is from a control subject known to experience algesia, particularly hyperalgesia. The control subject may be a mammal such as a human, rodent (e.g. rat, hamster, or mouse) or monkey, or may be another animal than a mammal such as an avian.

Preferably, both the sample and the control sample are from subjects of the same species (e.g. human), more preferably of the same gender (e.g. female or male) and/or of a similar age or phase of life (e.g. infant, young child, juvenile, adult, or elderly).

The terms "lowered" or "decreased", especially in the context of the level of Slfn2 refer to the level of Slfn2 in the sample being reduced in comparison to the reference or reference sample. The terms "elevated" or "increased", especially in the context of the level of Slfn2 refer to the level of Slfn2 in the sample being higher in comparison to the reference or reference sample. E.g. a miRNA that is detectable in higher amounts in cerebrospinal fluid or spinal cord tissue of one individual suffering from pain than in cerebrospinal fluid or spinal cord tissue of individuals not suffering from pain, has an elevated level. For Slfn2, an elevated level in a patient sample (such as cerebrospinal fluid or blood) indicates the presence of a pain-associated disease or pain-associated tissue state or pain or an increased susceptibility or increased probability to develop a pain-associated disease or pain-associated tissue state or pain, especially the presence or an increased susceptibility to neuropathic algesia or neuropathic pain.

The term "agonist" as used herein refers to a substance that causes an action such as receptor-signalling, gene expression, protein synthesis, and protein degradation. Typically, agonists act by binding to the active site or to allosteric sites of a receptor molecule thereby, triggering a specific reaction. Examples for agonists include but are not limited to nucleic acid molecules, such as mRNA or miRNA, or proteins, such as hormones, cytokines, growth factors, neurotransmitters, and transcription factors.

The term "antagonist" as used herein refers to a substance blocking the action of an agonist. Typically, antagonists act by binding to the active site or to allosteric sites of a receptor molecule, or interact with unique binding sites not normally involved in the regulation of the activity of the receptor. Typically, an antagonist competes with the agonist at structurally-defined binding sites. The antagonist activity may be reversible or irreversible depending on the longevity of the interaction of the antagonist-receptor complex. Examples for antagonists include but are not limited to nucleic acid molecules, such as siRNAs or miRNAs, or proteins such as hormones, cytokines, growth factors or neurotransmitter, antibodies, or transcription factors.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in an individual that have previously had the disorder(s); and (e) limiting or preventing recurrence of symptoms in individuals that were previously symptomatic for the disorder(s).

As used herein, "prevent", "preventing", "prevention", or "prophylaxis" of a disease or disorder means preventing that such disease or disorder occurs in patient.

The terms "pharmaceutical", "medicament" and "drug" are used interchangeably herein referring to a substance and/or a combination of substances being used for the identification, prevention or treatment of a tissue status or disease.

"Pharmaceutically acceptable" means approved by a regulatory agency of the Federal or a state government or listed in the U.S. Pharmacopeia or other generally recognized pharmacopeia for use in animals, and more particularly in humans.

The term "carrier", as used herein, refers to a pharmacologically inactive substance such as but not limited to a diluent, excipient, or vehicle with which the therapeutically active ingredient is administered. Such pharmaceutical carrier can be liquid or solid.

Liquid carrier include but are not limited to sterile liquids, such as saline solutions in water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. A saline solution is a preferred carrier when the pharmaceutical composition is administered intracavitary.

Solid form preparations include powders, tablets, pills, capsules, cachets, suppositories, and dispersible granules. A solid excipient can be one or more substances, which may also act as diluents, flavouring agents, binders, preservatives, tablet disintegrating agents, or an encapsulating material.

In powders, the excipient is preferably a finely divided solid, which is in a mixture with the finely divided inhibitor of the present invention. In tablets, the active ingredient of the present invention is mixed with the carrier having the necessary binding properties in suitable proportions and compacted in the shape and size desired. The powders and tablets preferably contain from 5% to 80%, more preferably from 20% to 70% of the active compound.

Suitable pharmaceutical "excipients" include starch, glucose, lactose, sucrose, gelatine, pectin, dextrin, malt, rice, flour, chalk, silica gel, magnesium carbonate, sodium stearate, magnesium stearate, glycerol monostearate, talc, sodium chloride, tragacanth, methylcellulose, sodium carboxymethylcellulose, dried skim milk, glycerol, propylene, glycol, water, ethanol a low melting wax, cocoa butter, and the like.

The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify. Examples of suitable pharmaceutical carriers are described in "Remington's Pharmaceutical Sciences" by E. W. Martin.

The term "composition" is intended to include the formulation of the active compound with encapsulating material as a carrier providing a capsule in which the active component with or without other carriers, is surrounded by a carrier, which is thus, in association with it.

The term "active ingredient" refers to the substance in a pharmaceutical composition or formulation that is biologically active, i.e. that provides pharmaceutical value. A pharmaceutical composition may comprise one or more active ingredients which may act in conjunction with or independently of each other.

The active ingredient can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as but not limited to those derived from sodium, potassium, ammonium, calcium, ferric hydroxides, isopropylamine, triethylamine, 2-ethylamino ethanol, histidine, procaine, and the like.

As used herein, "administering" includes *in vivo* administration, as well as administration directly to tissue *ex vivo*, such as vein grafts. In the context of the present invention oral, topical, transdermal and/or intracavitary administration is preferred.

A "therapeutically effective amount" is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

The terms "specifically binds", "specific binding" or the like, mean that an antibody or antigen-binding fragment thereof forms a complex with an antigen that is relatively stable under physiologic conditions. Specific binding can be characterized by an equilibrium dissociation constant of at least about 1x10⁻⁶ M or less (e.g., a smaller K_{D} denotes a tighter binding). Methods for determining whether two molecules specifically bind are well known in the art and include, for example, equilibrium dialysis, surface plasmon resonance, and the like. An isolated antibody that specifically binds murineSlfn2 may, however, exhibit cross-reactivity to other antigens such as Slfn2 or Slfn12L molecules from other species. Moreover, multi-specific antibodies (e.g., bispecifics) that bind to Slfn2 and one or more additional antigens are nonetheless considered antibodies that "specifically bind" Slfn2, as used herein.

As used herein, a "SLFN2 antagonist" denotes a compound that inhibits at least one biological activity of SLFN2, either the protein-activity of the transcriptional activity of the gene. Preferred SLFN2 antagonists are characterized in that they bind from 10% to 100% (preferably from 50% to 100%) of the SLFN2 protein present in a certain tissue (e.g. blood) when used in stoichiometric amounts.

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease or disorder means preventing that a disorder occurs in subject.

As used herein, the expression "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity.

The terms "therapeutically effective amount" or "therapeutic amount" are intended to mean that amount of a drug or pharmaceutical agent that will elicit the biological or medical response of a tissue, a system, animal or human that is being sought by a researcher, veterinarian, medical doctor or other clinician. The term "prophylactically effective amount" is intended to mean that amount of a pharmaceutical drug that will prevent or reduce the risk of occurrence of the biological or medical event that is sought to be prevented in a tissue, a system, animal or human by a researcher, veterinarian, medical doctor or other clinician. Particularly, the dosage a patient receives can be selected so as to achieve the amount of LDL (low density lipoprotein) cholesterol lowering desired; the dosage a patient receives may also be titrated over time in order to reach a target LDL level. The dosage regimen utilizing an antibody or an antigen-binding fragment thereof as described herein is selected in accordance with a variety of factors including type, species, age, weight, body mass index, sex and medical condition of the patient; the severity of the condition to be treated; the potency of the compound chosen to be administered; the route of administration; the purpose of the administration; and the renal and hepatic function of the patient.

As used herein, a "patient" means any mammal, reptile or bird that may benefit from a treatment with the compounds described herein. Preferably, a "patient" is selected from the group consisting of laboratory animals (e.g. mouse or rat), domestic animals (including e.g. guinea pig, rabbit, chicken, turkey, pig, sheep, goat, camel, cow, horse, donkey, cat, or dog), or primates including chimpanzees and human beings. It is particularly preferred that the "patient" is a human being.

"Pharmaceutically acceptable" means approved by a regulatory agency of the Federal or a state government or listed in the U.S. Pharmacopeia (United States Pharmacopeia-33/National Formulary-28 Reissue, published by the United States Pharmacopeial Convention, Inc., Rockville Md., publication date: April 2010) or other generally recognized pharmacopeia for use in animals, and more particularly in humans.

### ASPECTS OF THE INVENTION

The present invention will now be further described. In the following passages, different aspects of the invention are described in more detail, wherein this description and the terms used therein are to be understood in the context of the whole application, i.e. in light of the previous passages (e.g. the definitions) and the consecutive passages (e.g. the below listed examples and embodiments). Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

Accordingly, a first aspect of the invention relates to a method of detecting the effect of a compound on a pain-related tissue status or a pain-related disease, the method comprising the steps of:
a) providing a test system comprising Slfn2,
b) contacting the test system with a test compound, and
c) determining the effect of the test compound on the test system,
wherein the test compound is identified as being active in modulating a pain-related tissue status or disease, if a significant effect of the test compound on the test system relative to a control is detected.

In a second aspect, the invention relates to the use of Slfn2 for detecting the effect of a compound on a pain-related tissue status or a pain-related disease.

The first and second aspect relate to the identification and/or characterization of compounds for the treatment and/or prevention of a pain-related tissue status or pain-related disease, such as pain.

The test system of the first and second aspect can be any system as described herein, e.g. a biochemical assay (e.g. protein-activity or interaction) or cellular assay (e.g. a reporter assay or phenotypic assay), the test compound can be any compound as described herein, e.g. a small molecule or biological substance. If the test compound exhibits a significant effect on the test system relative to a control (e.g. a negative control), e.g. by increasing or decreasing protein activity, protein-protein interaction or reporter gene expression, the test compound is to be considered as being active in modulating (i.e. either increasing or decreasing) the pain related tissue status or - disease (e.g. pain).

According to a preferred embodiment of the first and second aspect, the effect of the compound is
a) a prevention, reduction or abolishment of a pain-related tissue status or disease (in this case the assay detects a compound that is useful for the prevention or treatment of a pain-related tissue status or -disease), or
b) an induction, increase or progression of a pain-related tissue status disease (in this case the method detects an adverse effect of a compound).

The method and use according to the first and second aspect are thus suitable for the idenfication of beneficial effects (e.g. the prevention or treatment of a pain-related tissue status or -disease) or adverse effects (e.g. the worsening of a pain-related tissue status or -disease) of compounds, as well as for the characterisation of compounds as having one or more of such effects.

Slfn2 of the two first aspects can be any Slfn2 as defined herein; according to a preferred embodiment of the first and second aspect, the Slfn2 is rodent, monkey or human Slfn2.

According to another preferred embodiment of the first and second aspect, Slfn2 is a Slfn2 gene or gene product, preferably a Slfn2 mRNA, or Slfn2 protein or a functionally active variant thereof.

According to another preferred embodiment of the first, second, third, fourth, fifth, sixth, seventh, eighth, ninth, tenth, eleventh, twelfth and thirteenth aspect of present invention, Slfn2 is a gene, wherein the gene
a) is a naturally occurring or non-naturally occurring Slfn2 gene variant, or
b) codes for a Slfn2 protein or a functionally active variant thereof that has
   i. an amino acid sequence according to SEQ ID NO: 2, 7 or 9,
   ii. an amino acid sequence that is a fragment of the amino acid sequence according to (i), or
   iii. an amino acid sequence that has at least 80%, at least 90%, at least 95% or at least 99% sequence identity to the amino acid sequence according to (i) or (ii).

According to another preferred embodiment of the first, second, third, fourth, fifth, sixth, seventh, eighth, ninth, tenth, eleventh, twelfth and thirteenth aspect of present invention, Slfn2 is a gene product, wherein the gene product
a) is a naturally occurring Slfn2 protein variant, preferably a homolog or ortholog in same or different species, or a non-naturally occurring Slfn2 protein variant,
b) is a Slfn2 protein or a functionally active variant thereof having
   (i) an amino acid sequence according to SEQ ID NO: 2, 7 or 9,
   (ii) an amino acid sequence that is a fragment of the amino acid sequence according to (i), or
   (iii) an amino acid sequence that has at least 80%, at least 90%, at least 95% or at least 99% sequence identity to the amino acid sequence according to (i) or (ii),
c) is a Slfn2 nucleic acid encoding a protein as defined in b), or
d) is a Slfn2 nucleic acid having
   (i) a nucleic acid sequence according to SEQ ID NO: 1, 6 or 8,
   (ii) a nucleic acid sequence that is a fragment of the nucleic acid sequence according to (i), or
   (iii) a nucleic acid sequence that has at least 80%, at least 90%, at least 95% or at least 99% sequence identity to the nucleic acid sequence according to (i) or (ii).

According to another preferred embodiment of the first and second aspect, the method is a luciferase assay (for detection of the effect on Slfn2 expression), an interaction assay (for detection of protein-protein or protein-DNA interaction) or a biochemical or cellular activity assay (for detection of the effect on the Slfn2 protein activity). The effect can e.g. be a decrease or increase in Slfn2 activity or expression. The expression level of Slfn2 can e.g. be determined on the gene, mRNA or protein level.

According to another preferred embodiment of the first and second aspect the Slfn2 gene or a functionally active variant thereof is brought into direct or indirect contact with a test compound and the influence of the test compound on the Slfn2 gene or functionally active variant thereof is measured or detected.

According to another preferred embodiment of the first or second aspect aspect the functionally active variant of the Slfn2 gene is a fragment of a Slfn2 gene, preferably a fragment able to drive expression of a downstream nucleic acid such as a reporter gene if functionally coupled thereto (e.g. the Slfn2 promoter or a functional fragment thereof), examples of such a fragment of the Slfn2 gene comprise: a fragment from position - 1766 to position +1, a fragment from position -1766 to position +50, a fragment from position -1766 to position +100, a fragment from position -1766 to position + 150, a fragment from position -1766 to position +198, a fragment from position -1265 to position +1, a fragment from position -1265 to position +50, a fragment from position - 1265 to position +100, a fragment from position -1265 to position + 150, or a fragment from position -1265 to position +198, all with respect to the sequence according to SEQ ID NO:5.

Preferred embodiments of pain-related or pain-associated disease or pain-associated tissue status according to first, second, third, fourth, fifth, sixth, seventh, eighth, ninth, tenth, eleventh, twelfth and thirteenth aspect of present invention: The pain-related disease can e.g. be pain or a pain-associated disease, wherein the pain-associated disease is preferably migraine, depression or epilepsy. The pain-associated tissue status or pain-associated disease may also be characterized by an altered level of pain-related regulatory molecules or signaling molecules, indicators of pain and/or degenerative molecules present in the tissue. In one embodiment of the different aspects, the pain is selected from one of the following: (i) acute pain, subacute pain, or chronic pain, (ii) nociceptive pain, neuropathic pain, inflammatory pain, or a mixture thereof, (iii) disease-associated pain, or (iv) algesia, and wherein the acute pain is preferably somatogenetic (organic) pain, the chronic pain is preferably somatogenetic (organic) pain or psychogenic (psychosomatic) pain, the nociceptive pain is preferably superficial somatic pain or deep pain, such as deep somatic pain or visceral pain, the neuropathic pain is preferably central neuropathic pain or peripheral neuropathic pain, the mixed pain is preferably a mixture of nociceptive pain and neuropathic pain, the disease-associated pain is preferably pain associated with depression, migraine, epilepsy, cancer, or diabetes, and the algesia is preferably hypo- or hyperalgesia. In another embodiment of the different aspects, the pain-related tissue status is a tissue damage, trauma, inflammation, infarction, necrosis, edema, neoplasia of any tissue, the tissue preferably being selected from the group consisting of bone, cartilage, muscle, skin, neural tissue, such as a nerve or spinal chord, connective tissue such as perichondrium, periosteum, tendon, capsule, skin and and/or wherein the tissue status is characterized by an altered level of pain-related regulatory molecules or signaling molecules, indicators of pain and/or degenerative molecules present in the tissue.

In a third aspect, the invention relates to the use of Slfn2 for determining the dosage of a compound for the prevention or treatment of a pain-related tissue status or disease.

The determination of the dosage can be performed according to any suitable method. According to one aspect it is determined according to the method of aspect 9 and one or more of its different embodiments.

According to a preferred embodiment of the third aspect, the effect of the compound is
a) a prevention, reduction or abolishment of a pain-related tissue status or disease (in this case the assay detects a compound that is useful for the prevention or treatment of a pain-related tissue status or -disease), or
b) an induction, increase or progression of a pain-related tissue status disease (in this case the method detects an adverse effect of a compound).

According to another preferred embodiment of the third aspect, the Slfn2 is rodent, monkey or human Slfn2.

According to another preferred embodiment of the third aspect, Slfn2 is a Slfn2 gene or gene product, preferably a Slfn2 mRNA, or Slfn2 protein or a functionally active variant thereof.

According to another preferred embodiment of the third aspect, Slfn2 is a gene, wherein the gene is as defined above for under the first aspect of present invention.

According to another preferred embodiment of the third aspect, Slfn2 is a gene product, wherein the gene product is as defined above under the first aspect of present invention.

The effect can e.g. be a decrease or increase in Slfn2 activity or expression. The expression level of Slfn2 can e.g. be determined on the gene, mRNA or protein level, wherein the level of Slfn2 is preferably detected in a sample, and wherein sample preferably comprises tissue and/or fluid.

According to another preferred embodiment of the third aspect the expression level of Slfn2 is determined in a sample obtained in a time interval prior to step (i).

According to another preferred embodiment of the third aspect, the tissue sample is selected from the group consisting of neural or extraneural tissue or cells, preferably neural tissue such as spinal chord or nerve, and the body fluid sample is selected from the group consisting of cerebrospinal fluid, synovial fluid, serum and plasma.

According to another preferred embodiment of the third aspect, the pain-related or pain-associated disease or pain-associated tissue status is as defined above under the first aspect of present invention.

In a fourth aspect, the invention relates to the use of a cell overexpressing Slfn2 or of a non-human transgenic animal heterologously expressing Slfn2 for determining the effect of a compound on a pain-related tissue status or disease.

In a fifth aspect, the invention relates to the use of a cell overexpressing Slfn2 or of a non-human transgenic animal heterologously expressing Slfn2 as a model system for enhanced pain sensitivity. As the Slfn2 level has been shown in the experiments to strongly correlate with pain, especially neuropathic pain in test animals (see experiments), a cell or cell line or non-human transgenic animal overexpressing or heterologously expressing Slfn2 is suitable as model system for enhanced pain sensitivity.

In the context of the fourth and fifth aspect, the cell can be any kind of cell that is suitable for transient or stable expression of heterologous gene products, e.g. one of the cells or cell-lines described above or below; e.g. a neural or neuronal cell I or cell line.

The transgenic animal of the fourth and fifth aspect can be any non-human animal, e.g. one of the animals described herein, such as a rodent, e.g. a mouse or a rat.

The effect of the compound in the fourth and fifth aspect is preferably determined by means of one of the methods of present invention, e.g. according to a method of the first, seventh, eighth or eleventh aspect.

According to another preferred embodiment of the fourth or fifth aspect, the Slfn2 overexpressed or heterologously expressed is rodent, monkey or human Slfn2.

According to another preferred embodiment of the fourth or fifth aspect, Slfn2 is a Slfn2 gene or gene product, preferably a Slfn2 mRNA, or Slfn2 protein or a functionally active variant thereof.

According to another preferred embodiment of the fourth and fifth aspect Slfn2 is a gene and the gene is as defined above under the first aspect of present invention.

According to another preferred embodiment of the fourth and fifth aspect Slfn2 is a gene product and the gene product is as defined above under the first aspect of present invention.

According to another preferred embodiment of the fourth and fifth aspect, the effect is a decrease or increase in Slfn2 activity or expression, wherein the expression level of Slfn2 can e.g. be determined on the gene, mRNA or protein level.

According to another preferred embodiment of the fourth and fifth aspect, the level of Slfn2 is detected in a sample, wherein sample preferably comprises tissue and/or fluid.

According to another preferred embodiment of the fourth and fifth aspect, the tissue sample is selected from the group consisting of neural or extraneural tissue or cells, preferably neural tissue such as spinal cord or nerve, and the body fluid sample is selected from the group consisting of cerebrospinal fluid, synovial fluid, serum and plasma.

According to another preferred embodiment of the fourth and fifth aspect, the pain-related or pain-associated disease or pain-associated tissue status is as defined above under the first aspect of present invention.

In a sixth aspect, the invention relates to the use of a Slfn2 knock-out cell or a non-human Slfn2 knock-out animal as a model system for lowered pain sensitivity.

By means of the sixth aspect it can e.g. be tested, if the compounds to be tested for their ability of modulating a pain-related tissue status or pain-related disease such as pain, are able to enhance or restore the lowered or totally abolished SLFN2 activity in the employed cell or animal.

The knock-out cell can be any cell as herein described, e.g. a human, mouse or rat cell. The knock-out animal can be any non-human animal, e.g. an animal as herein described such as a rodent, especially a mouse or rat.

According to a preferred embodiment of the sixth aspect, the Slfn2 is rodent, monkey or human Slfn2.

According to another preferred embodiment of the sixth aspect, Slfn2 is a Slfn2 gene or gene product, preferably a Slfn2 mRNA, or Slfn2 protein or a functionally active variant thereof.

According to another preferred embodiment of the sixth aspect, Slfn2 is a gene wherein the gene is as defined above under the first aspect of present invention.

According to another preferred embodiment of the sixth aspect, Slfn2 is a gene product, wherein the gene product is as defined above under the first aspect of present invention.

According to another preferred embodiment of the sixth aspect, the functionally active variant of the Slfn2 gene is a fragment of a Slfn2 gene, preferably a fragment able to drive expression of a downstream nucleic acid such as a reporter gene if functionally coupled thereto.

According to another preferred embodiment of the sixth aspect, the functionally active variant of the Slfn2 gene is a fragment of a Slfn2 gene, preferably a fragment able to drive expression of a downstream nucleic acid such as a reporter gene if functionally coupled thereto (e.g. the Slfn2 promoter or a functional fragment thereof), examples of such a fragment of the Slfn2 gene comprise: a fragment from position -1766 to position +1, a fragment from position -1766 to position +50, a fragment from position -1766 to position +100, a fragment from position -1766 to position + 150, a fragment from position -1766 to position +198, a fragment from position -1265 to position +1, a fragment from position -1265 to position +50, a fragment from position -1265 to position +100, a fragment from position -1265 to position + 150, or a fragment from position - 1265 to position +198, all with respect to the sequence according to SEQ ID NO:5.

According to another preferred embodiment of the sixth aspect, the pain-related or pain-associated disease or pain-associated tissue status is as defined above under the first aspect of present invention.

In a seventh aspect, the invention relates to the use of a method for determining a pain-modulating effect of a compound comprising
(i) administering a test compound to a subject
(ii) detecting whether said compound increases or decreases the Slfn2 expression level in said subject in comparison to a control subject not having been administered said compound,
wherein a decrease in Slfn2 expression level after treatment with the compound in comparison to the level in the control animal is indicative of a pain reducing effect of the compound and an increase is indicative of pain as (adverse) effect of the compound.
In an eighth aspect, the invention relates to the a method of for determining a pain-modulating effect of a compound comprising
(i) determining the expression level of Slfn2 in a subject
(ii) applying a compound to the subject, and
(iii) determining the expression level of Slfn2 after application of the compound, wherein
the compound is displaying pain as adverse effect, if the expression level of Slfn2 is increased in one or more of the samples obtained after application of the compound in comparison to the level obtained before application of the compound and a decrease in Slfn2 expression level after treatment with the compound in comparison with the level before treatment is indicative of a pain reducing effect of the compound.

The subject or subjects to be used in the seventh or eighths aspect can e.g. be one or more voluntary human subjects (either healthy or patients carrying a pain-related tissue status or pain-related disease) or one or more non-human subjects (either healthy or carrying a pain-related tissue status or disease, wherein this tissue status or disease in the non-human subject can preferably be invoked by purpose, e.g. by means of a genetic modification or a surgical procedure, see e.g. the murine models described above and below, e.g. those as used in the experiments).

The expression level of Slfn2 in the seventh or eights aspect can be determined by any method as commonly used in the art, e.g. one of the methods as described above or below or used in the examples.

According to a preferred embodiment of the seventh and eighth aspect, the effect of the compound is
a) a prevention, reduction or abolishment of a pain-related tissue status or disease (in this case the assay detects a compound that is useful for the prevention or treatment of a pain-related tissue status or -disease), or
b) an induction, increase or progression of a pain-related tissue status disease (in this case the method detects an adverse effect of a compound).

The method and use according to the seventh and eighth aspect are thus suitable for the idenfication of beneficial effects (e.g. the prevention or treatment of a pain-related tissue status or -disease) or adverse effects (e.g. the worsening of a pain-related tissue status or -disease) of compounds, as well as for the characterisation of compounds as having one or more of such effects.

According to another preferred embodiment of the seventh and eighth aspect, the Slfn2 is rodent, monkey or human Slfn2.

According to another preferred embodiment of the seventh and eighth aspect, Slfn2 is a Slfn2 gene or gene product, preferably a Slfn2 mRNA, or Slfn2 protein or a functionally active variant thereof.

According to another preferred embodiment of the seventh and eighth aspect, Slfn2 is a gene, wherein the gene is as defined above under the first aspect of present invention.

According to another preferred embodiment of the seventh and eighth aspect, Slfn2 is a gene product, wherein the gene product is as defined above under the first aspect of present invention.

According to another preferred embodiment of the seventh and eighth aspect, the method is a luciferase assay (for detection of the effect on Slfn2 expression), an interaction assay (for detection of protein-protein or protein-DNA interaction) or a biochemical or cellular activity assay (for detection of the effect on the Slfn2 protein activity). The effect can e.g. be a decrease or increase in Slfn2 activity or expression. The expression level of Slfn2 can e.g. be determined on the gene, mRNA or protein level.

According to another preferred embodiment of the seventh and eighth aspect the Slfn2 gene or a functionally active variant thereof is brought into direct or indirect contact with a test compound and the influence of the test compound on the Slfn2 gene or functionally active variant thereof is measured or detected.

According to another preferred embodiment of the first or second aspect aspect the functionally active variant of the Slfn2 gene is a fragment of a Slfn2 gene, preferably a fragment able to drive expression of a downstream nucleic acid such as a reporter gene if functionally coupled thereto (e.g. the Slfn2 promoter or a functional fragment thereof), examples of such a fragment of the Slfn2 gene comprise: a fragment from position - 1766 to position +1, a fragment from position -1766 to position +50, a fragment from position -1766 to position +100, a fragment from position -1766 to position + 150, a fragment from position -1766 to position +198, a fragment from position -1265 to position +1, a fragment from position -1265 to position +50, a fragment from position - 1265 to position +100, a fragment from position -1265 to position + 150, or a fragment from position -1265 to position +198, all with respect to the sequence according to SEQ ID NO:5.

According to another preferred embodiment of the seventh and eighth aspect, the pain-related or pain-associated disease or pain-associated tissue status is as defined above under the first aspect of present invention.

In a ninth aspect, the invention relates to a method for determining the dosage of a compound for the prevention or treatment of a pain-related tissue status or disease, comprising the steps of
(i) determining the level of Slfn2 in an individual, and optionally determining the level of Slfn2 in a reference, and
(ii) determining the dosage of a pharmaceutical depending on the level of Slfn2 in the first individual optionally in comparison with that of the reference.

The reference can be any suitable reference, e.g. as herein described. Examples comprise a reference value, a reference organism or a reference sample.

The determination of the level of Slfn2 can be performed according to any suitable method known in the art, e.g. as herein described. In one embodiment the individual has been given a compound and the level of Slfn2 is determined after a time sufficient for the compound to be internalized and exerting its effect(s) on Slfn-2 expression. In this embodiment the reference (e.g. sample (artificial or taken from another individual or from the same individual at an earlier time point), value or organism) has or is representative for a Slfn2 level in presence of a certain pain-related tissue status or a pain-related disease (e.g. pain) that can be treated with a known dosage of compound (e.g. a known amount of compound per kg body weight in the same species as the individual). If the level of Slfl2 is equal to that of the reference, the dosage to be applied is determined to be equal to that as known for the reference. If the level in the individual is higher or lower than that of the reference, the dosage needs to be higher or lower.

The method can also be performed with more references, wherein each reference is characteristic for a certain dosage to be used in the species of the individual.

According to a preferred embodiment of the ninth aspect, the Slfn2 is rodent, monkey or human Slfn2.

According to another preferred embodiment of the ninth aspect, Slfn2 is a Slfn2 gene or gene product, preferably a Slfn2 mRNA, or Slfn2 protein or a functionally active variant thereof.

According to another preferred embodiment of the ninth aspect, Slfn2 is a gene, wherein the gene is as defined above under the first aspect of present invention.

According to another preferred embodiment of the ninth aspect, Slfn2 is a gene product, wherein the gene product is as defined above under the first aspect of present invention.

According to another preferred embodiment of the ninth aspect, the expression level of Slfn2 is determined in a sample obtained in a time interval prior to step (i).

According to another preferred embodiment of the ninth aspect, the level of Slfn2 is detected in a sample, and wherein sample preferably comprises tissue and/or fluid, wherein the tissue sample is preferably selected from the group consisting of neural or extraneural tissue or cells, preferably neural tissue such as nerve or spinal cord, and the body fluid sample is selected from the group consisting of cerebrospinal fluid, synovial fluid, serum and plasma.

According to another preferred embodiment of the ninth aspect, the pain-related or pain-associated disease or pain-associated tissue status is as defined above under the first aspect of present invention.

In a tenth aspect, the invention relates to a method for adapting the dosage of a compound for the prevention or treatment of a pain-related tissue status or disease, comprising the steps of
(a) determining the level of Slfn2 in an individual
(b) determining the level of Slfn2 in one or more reference samples,
(c) examining the tested sample as to whether the level of Slfn2 present in said sample is different from the level in the one or more references, and
(d) adapting the dosage of a pharmaceutical depending on whether the level of Slfn2 in the tested sample is different from the level in the one or more reference samples.

The reference can be any suitable reference, e.g. as herein described. Examples comprise a reference value, a reference organism or a reference sample.

The determination of the Slfn2 level can be performed by means of any suitable method known in the art, e.g. a method as described herein.

In one embodiment, the level of Slfn2 is measured before compound application. In this case, the reference sample(s) is (are) characteristic of a Slfn2 level in patients of the same species having a pain-related disorder or pain-related tissue status (e.g. pain) that can be treated or prevented with a certain dosage (a standard dosage). If the Slfn level in the individual is higher than that in the reference, the dosage for the individual needs to be increased. If it is lower than that of the reference, the dosage can be lowered.

In another embodiment, the individual has been given a compound and the level of Slfn2 is determined after a time sufficient for the compound to be internalized and exerting its effect(s) on Slfn-2 expression. In this embodiment, the reference sample(s) is (are) representative for a Slfn2 value that is characteristic for the absence of a certain pain-related tissue status or pain-related disease (e.g. the absence of pain) or is characteristic for patients of the same species having successfully been treated with the compound. In this case, the dosage needs to be increased if the level of Slfn2 in the individual's sample is higher than in that of the reference sample(s) and can be decreased if the level is lower than that of the reference sample(s).

According to a preferred embodiment of the tenth aspect, the Slfn2 is rodent, monkey or human Slfn2.

According to another preferred embodiment of the tenth aspect Slfn2 is a Slfn2 gene or gene product, preferably a Slfn2 mRNA, or Slfn2 protein or a functionally active variant thereof.

According to another preferred embodiment of the tenth aspect, Slfn2 is a gene and the gene is as defined above under the first aspect of present invention.

According to another preferred embodiment of the tenth aspect, Slfn2 is a gene product and wherein the gene product is as defined above under the first aspect of present invention.

According to another preferred embodiment of the tenth aspect, the expression level of Slfn2 is determined on the gene, mRNA or protein level, preferably in a sample, and wherein sample preferably comprises tissue and/or fluid.

According to another preferred embodiment of the tenth aspect, the expression level of Slfn2 is determined in a sample obtained in a time interval prior to step (i).

According to another preferred embodiment of the tenth aspect, the tissue sample is selected from the group consisting of neural or extraneural tissue or cells, preferably neural tissue such as nerve or spinal cord, and the body fluid sample is selected from the group consisting of cerebrospinal fluid, synovial fluid, serum and plasma.

According to another preferred embodiment of the tenth aspect, the pain-related or pain-associated disease or pain-associated tissue status is as defined above under the first aspect of present invention.

In an eleventh aspect, the invention relates to a method of determining the beneficial and/or adverse effects of a substance on a pain-related tissue status or disease, comprising the steps of
(i) determining the level of Slfn2 in a test sample,
(ii) determining the level of Slfn2 in one or more reference samples, and
(iii) examining the tested sample as to whether the level of Slfn2 present in said sample is different from the level in the one or more reference samples,
wherein the test sample was exposed differently to said substance than the one or more reference samples.

A reference sample may be "treated differently" or "exposed differently" than a sample of interest in case both samples are treated in a substantially identical way except from a single factor. Such single factors include but are not limited to the time of exposure, the concentration of exposure, or the temperature of exposure to a certain substance. Accordingly, a sample of interest may be exposed to a different dosage of a certain substance than the reference sample or may be exposed for a different time interval than the reference sample or may be exposed at a different temperature than the reference sample. Different dosages to which a sample of interest may be exposed to include but are not limited to the 2-fold, 3- fold, 4-fold, 5-fold, 10-fold, 20-fold, 30-fold, 40-fold, 50-fold, 100-fold and/or 1000-fold increased or decreased dosage of the dosage the reference sample is exposed to. Different exposure times to which a sample of interest may be exposed to include but are not limited to the 2-fold, 5-fold, 10-fold, 20-fold, 30-fold, 40-fold, 50-fold, 100-fold and/or 1000-fold longer or shorter time period than the exposure of the reference. Different temperatures of exposure to which a sample of interest may be exposed to include but are not limited to the 2-fold, 5-fold, 10-fold, 20-fold, 30-fold, 40-fold, 50-fold, 100-fold and/or 1000-fold increased or decreased temperature than the exposure of the reference. In a non-limiting example a sample of interest may be exposed to a 10-fold increased concentration of a substance than the reference sample. The analysis of both samples is then conducted in a substantially identical manner allowing determining the effects, i.e. a beneficial or an adverse effect, of the increased concentration of such substance on the sample of interest. The skilled person will appreciate that this example applies mutatis mutandis to different ranges of concentrations, different exposure times, and/or different temperatures at exposure.

Determination of effect can be performed according to any method as known in the art, e.g. a method as herein described. The sample, e.g. the test or reference sample can be any suitable sample as known in the art, e.g. as herein described.

According to another preferred embodiment of the eleventh aspect, the effect of the compound is
a) a prevention, reduction or abolishment of a pain-related tissue status or disease (in this case the compound displays a beneficial effect), or
b) an induction, increase or progression of a pain-related tissue status or disease (in this case the compound displays an adverse effect).

According to another preferred embodiment of the eleventh aspect, the Slfn2 is rodent, monkey or human Slfn2, wherein Slfn2 preferably is a Slfn2 gene or gene product, preferably a Slfn2 mRNA, or Slfn2 protein or a functionally active variant thereof.

According to another preferred embodiment of the eleventh aspect, Slfn2 is a gene and the gene is as defined above for the first aspect of present invention.

According to another preferred embodiment of the eleventh aspect Slfn2 is a gene product and the gene product is as defined above for the first aspect of present invention.

According to another embodiment of to the eleventh aspect, the expression level of Slfn2 can be determined on the gene, mRNA or protein level, preferably in a sample, wherein sample preferably comprises tissue and/or fluid.

According to another preferred embodiment of the eleventh aspect, the expression level of Slfn2 is determined in a sample obtained in a time interval prior to step (i).

According to another preferred embodiment of the eleventh aspect, the tissue sample is selected from the group consisting of neural or extraneural tissue or cells, preferably neural tissue such as nerve or spinal cord, and the body fluid sample is selected from the group consisting of cerebrospinal fluid, synovial fluid, serum and plasma.

According to another preferred embodiment of the eleventh aspect, the pain-related or pain-associated disease or pain-associated tissue status is as defined in the first aspect.

In a twelfth aspect, the invention relates to the use of Slfn2 as a target molecule for the discovery of a compound preventing or reducing or abolishing a pain related tissue status or disease.

The discovery or characterization of a compound according to the twelfth aspect can e.g. be performed by means of one of the methods according to the first, seventh, eighth or eleventh aspect of present invention.

According to a preferred embodiment of the twelfth aspect of present invention, Slfn2 is rodent, monkey or human Slfn2.

According to another preferred embodiment of the twelfth aspect of present invention, Slfn2 is a Slfn2 gene or gene product, preferably a Slfn2 mRNA, or Slfn2 protein or a functionally active variant thereof.

According to another preferred embodiment of the twelfth aspect of present invention, Slfn2 is a gene and wherein the gene is as defined above under the first aspect of present invention.

According to another preferred embodiment of the twelfth aspect of present invention, wherein Slfn2 is a gene product and the gene product is as defined above under the first aspect of present invention.

According to another preferred embodiment of the twelfth aspect, the pain-related or pain-associated disease or pain-associated tissue status is as defined in the first aspect.

In a thirteenth aspect, the invention relates to a kit for detecting the effect of a compound on a pain-related tissue status or a pain-related disease comprising
a. a means for detecting Slfn2 and optionally
b. a data carrier comprising instructions for one (or more) of the above methods and optionally
c. a container.

The kit can comprise any feature as described herein; in the least it contains a means for detecting Slfn2. The kit can also comprise a means and a data carrier, a means and a container or a means and a data carrier and a container. The kit can also comprise one or further additional components, e.g. such as herein described.

The means for detecting Slfn2 can be any means that is able to specifically (i.e. stronger, i.e. 5%, 10%, 15%, 20%, 25%, 30%, 35%, 40%, 50%, 60%, 70%, 80%, 90%, 100% or more stronger than a non-Slfn2 molecule or entity), especially in a sample; preferred means are those as herein described.

In one embodiment of the thirteenth aspect, the detection of the effect of the compound is preferably performed by means of one of the methods according to the first, seventh, eighth or eleventh aspect of present invention, wherein the kit can also be used in the context of a method of the ninth or tenth aspect of present invention.

According to another embodiment of the thirteenth aspect of present invention, the effect of the compound is a prevention, reduction or abolishment of a pain-related tissue status or disease (i.e. a beneficial effect of the compound) or an induction, increase or progression of a pain-related tissue status disease (i.e. in this case, the compound displays an adverse effect)

According to another preferred embodiment of the thirteenth aspect, Slfn2 is rodent, monkey or human Slfn2.

According to another preferred embodiment of the thirteenth aspect, Slfn2 is a Slfn2 gene or gene product, preferably a Slfn2 mRNA, or Slfn2 protein or a functionally active variant thereof.

According to another preferred embodiment of the thirteenth aspect, Slfn2 is a gene and wherein the gene is as defined above under the first aspect of present invention.

According to another preferred embodiment of the thirteenth aspect, Slfn2 is a gene product and wherein the gene product is as defined above under the first aspect of present invention.

According to another preferred embodiment of the thirteenth aspect, the effect is e.g. be a decrease or increase in Slfn2 activity or expression. The expression level of Slfn2 can e.g. be determined on the gene, mRNA or protein level. According to another embodiment of the thirteenth aspect, the detection of the effect is performed by means of a luciferase assay (for detection of the effect on Slfn2 expression), an interaction assay (for detection of protein-protein or protein-DNA interaction) or a biochemical or cellular activity assay (for detection of the effect on the Slfn2 protein activity).

According to another preferred embodiment of the thirteenth aspect, the Slfn2 gene or a functionally active variant thereof is brought into direct or indirect contact with a test compound and the influence of the test compound on the Slfn2 gene or functionally active variant thereof is measured or detected.

According to another preferred embodiment of the thirteenth aspect the functionally active variant of the Slfn2 gene is a fragment of a Slfn2 gene, preferably a fragment able to drive expression of a downstream nucleic acid such as a reporter gene if functionally coupled thereto (e.g. the Slfn2 promoter or a functional fragment thereof), examples of such a fragment of the Slfn2 gene comprise: a fragment from position - 1766 to position +1, a fragment from position -1766 to position +50, a fragment from position -1766 to position +100, a fragment from position -1766 to position + 150, a fragment from position -1766 to position +198, a fragment from position -1265 to position +1, a fragment from position -1265 to position +50, a fragment from position - 1265 to position +100, a fragment from position -1265 to position + 150, or a fragment from position -1265 to position +198, all with respect to the sequence according to SEQ ID NO:5.

According to another embodiment of the thirteenth aspect, the level of Slfn2 is detected in a sample, wherein sample preferably comprises tissue and/or fluid.

According to another embodiment of the thirteenth aspect, the tissue sample is selected from the group consisting of neural or extraneural tissue or cells, preferably neural tissue such as nerve or spinal cord, and the body fluid sample is selected from the group consisting of cerebrospinal fluid, synovial fluid, serum and plasma.

According to another embodiment of the thirteenth aspect, the pain-related or pain-associated disease or pain-associated tissue status is as defined above under the first aspect.

According to another embodiment of the thirteenth aspect the means for detecting Slfn2 is a means for determining the expression level of Slfn2, preferably on the gene, protein or RNA level. The means for detecting Slfn2 is e.g. selected from the group consisting of nucleic acid, preferably DNA, RNA or PNA, peptide and protein, preferably monoclonal or polyclonal antibody or protein scaffold.

According to another embodiment of the thirteenth aspect, the kit comprises
(a) a container, and/or
(b) a data carrier, wherein the data carrier comprises information such as
   (i) instructions concerning methods for identifying the risk for developing and/or identifying the presence and/or monitoring progression of a pain-related tissue status or disease.
   (ii) instructions for use of the means for detecting Slfn2, preferably in a sample, more preferably in a sample from an individual and/or of the kit,
   (iii) quality information such as information about the lot/batch number of the means for detecting Slfn2 and/or of the kit, the manufacturing or assembly site or the expiry or sell-by date, information concerning the correct storage or handling of the kit,
   (iv) information concerning the composition of the buffer(s), diluent(s), reagent(s) for detecting Slfn2 and/or of the means for detecting Slfn2,
   (v) information concerning the interpretation of information obtained when performing the above-mentioned methods identifying and/or monitoring progression of a pain-related tissue status or disease,
   (vi) a warning concerning possible misinterpretations or wrong results when applying unsuitable methods and/or unsuitable means, and/or
   (vii) a warning concerning possible misinterpretations or wrong results when using unsuitable reagent(s) and/or buffer(s).

According to another embodiment of the thirteenth aspect, the kit comprises one or more reference compounds selected from the group consisting of: a compound inhibiting Slfn2 expression, a compound inhibiting Slfn2 activity, a compound not inhibiting Slfn2 expression, a compound not inhibiting Slfn2 activity.

According to another embodiment of the thirteenth aspect, the kit comprises instructions for performing one of the methods according to embodiments 1 or 7, 8, 9, 10 or 11.

According to another embodiment of the thirteenth aspect, the means comprises
a) one or more nucleic acids or derivatives for detecting a Slfn2 gene or gene product or functionally active variant thereof, preferably selected from the group consisting of a nucleic acid probe, a polyamide or peptide nucleic acid (PNA), microRNA (miRNA), small interfering RNA (siRNA), primers for polymerase chain reaction (PCR), primers for reverse transcription (RT) reaction, and primers for DNA sequencing and/or
b) a peptide, polypeptide or protein for detecting a Slfn2 gene or gene product or functionally active variant thereof, preferably selected from the group consisting of a protein ligand, preferably an antibody, a fragment or derivate thereof, a protein scaffold, such as a darpin or an anticalin, or wherein the polypeptide or peptide is a probe, preferably a mass spectrometry probe and/or
c) a biochip, a set of beads.

In a fourteenth aspect, the invention relates to a compound (or composition comprising a compound) able to lower Slfn2 activity and/or expression for the prevention or treatment of a pain-related tissue status or disease.

According to a preferred embodiment of the fourteenth aspect, the Slfn2 is rodent, monkey or human Slfn2, wherein Slfn2 can e.g. be a Slfn2 gene or gene product, preferably a Slfn2 mRNA, or Slfn2 protein or a functionally active variant thereof.

According to another embodiment of the fourteenth aspect, the compound further comprises a pharmaceutically acceptable carrier and/or excipient and optionally one or more additional active substances.

According to another embodiment of the fourteenth aspect, the compound is administered orally, topically, intravasal, intraspinal or intracavitary.

According to another embodiment of the fourteenth aspect, the pain-related or pain-associated disease or pain-related tissue status is as defined above under the first aspect.

According to one embodiment of the fourteenth aspect, the compound is BMS-345541. BMS-345541 is an inhibitor of the IKK2 kinase and inhibits the NF-kappa B pathway. BMS-345541 has been shown to inhibit Slfn-2 transcriptional activation (Wern-Joo et al., 2004) and is thus a Slfn-2 inhibitor. BMS-345541 and its structure could thus be used for the development of a pharmaceutical for the treatment of pain-related diseases or tissue states, preferably pain. BMS-345541 can be purchased from commercial suppliers, like Sigma Aldrich etc. The chemical name of BMS-345541 is N-(1,8-Dimethylimidazo[1,2-a]quinoxalin-4-yl)-1,2-ethanediamine hydrochloride, it has the CAS Number 547757-23-3, the empirical formula C₁₄H₁₇N₅HCl and a molecular weight of 291.78. The structure of BMS-345541 is shown as follows:

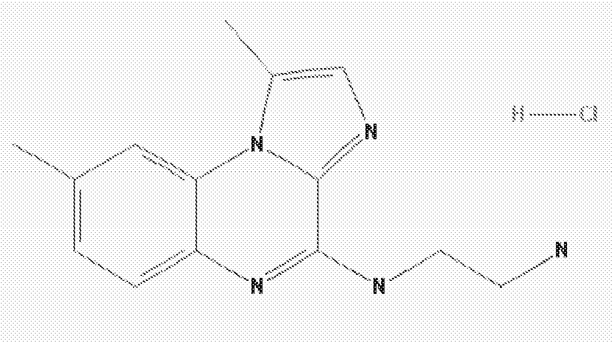

For the development of a compound for the treatment or prevention of pain in the context of the different aspects of present invention, the skilled person would thus make use of the compound BMS-345541. If necessary, the skilled artisan could take the structure of BMS-345541 and modify it so that it would not have any (severe) adverse effects but would still be able to inhibit NF-kappa B activation of Slfn-2 transcription. In a similar way other known inhibitors of transcriptional activators AP1 or NF-kappa B could be used in the context of the fourteenth aspect of present invention.

In a fifteenth aspect, the invention relates to a method of preventing or treating a pain-related tissue status or disease, wherein a therapeutically effective amount of the compound according to the fourteenth aspect is administered to an individual at risk of developing a pain-related tissue status or disease or suffering from a pain-related tissue status or disease.

According to a preferred embodiment of the fifteenth aspect, the individual is a human or non-human animal as herein defined, preferably a mammal, such as a rodent, monkey or human.

According to another embodiment of the fourteenth or fifteenth aspect, the compound is admixed with a pharmaceutically acceptable carrier and/or excipient and optionally one or more additional active substances. The compound can thus be admixed (formulated) with a pharmaceutically acceptable carrier, a pharmaceutically acceptable excipient, one or more (2, 3, 4, 5, 6, 7, 8, 9, 10 or more) additional active substances, a pharmaceutically acceptable carrier and a pharmaceutically acceptable excipient, a pharmaceutically acceptable carrier and one or more (2, 3, 4, 5, 6, 7, 8, 9, 10 or more) additional active substances, a pharmaceutically acceptable excipient and one or more (2, 3, 4, 5, 6, 7, 8, 9, 10 or more) additional active substances or a pharmaceutically acceptable carrier and a pharmaceutically acceptable excipient and and one or more (2, 3, 4, 5, 6, 7, 8, 9, 10 or more) additional active substances.

According to another embodiment of the fourteenth or fifteenth aspect, the compound is administered orally, topically, intravasal, intraspinal or intracavitary and is thus formulated or prepared for oral, topical, intravasal, intraspinal or intracavitary administration.

According to another embodiment of the fifteenth aspect, the pain-related or pain-associated disease or pain-related tissue status is as defined above under the first aspect.

### LIST OF PREFERRED EMBODIMENTS (NOT LIMITING):

In the following, preferred embodiments of present invention are listed but are not to be understood as limiting present invention:
1. A method of detecting the effect of a compound on a pain-related tissue status or a pain-related disease, the method comprising the steps of:
   a) providing a test system comprising Slfn2,
   b) contacting the test system with a test compound, and
   c) determining the effect of the test compound on the test system,
   wherein the test compound is identified as being active in modulating a pain-related tissue status or disease, if a significant effect (pos or neg) of the test compound on the test system relative to a control is detected.
2. Use of Slfn2 for detecting the effect of a compound on a pain-related tissue status or a pain-related disease.
3. Use of Slfn2 for determining the dosage of a compound for the prevention or treatment of a pain-related tissue status or disease.
4. Use of a cell overexpressing Slfn2 or of a non-human transgenic animal heterologously expressing Slfn2 for determining the effect of a compound on a pain-related tissue status or disease.
5. Use of a cell overexpressing Slfn2 or of a non-human transgenic animal heterologously expressing Slfn2 as a model system for enhanced pain sensitivity.
6. Use of a Slfn2 knock-out cell or a non-human Slfn2 knock-out animal as a model system for lowered pain sensitivity.
7. A method for determining a pain-modulating effect of a compound comprising
   (i) administering a test compound to a subject (human or non-human animal (not ill)
   (ii) detecting whether said compound increases or decreases the Slfn2 expression level in said subject in comparison to a control animal not having been administered said compound, sample or imaging
   wherein a decrease in Slfn2 expression level after treatment with the compound in comparison to the level in the control animal is indicative of a pain reducing effect of the compound and an increase is indicative of pain as (adverse) effect of the compound.
8. A method of for determining a pain-modulating effect of a compound comprising
   (i) determining the expression level of Slfn2 in a subject sample or imaging, animal or human not ill,
   (ii) applying a compound to the subject, and
   (iii) determining the expression level of Slfn2 (in one or more sample(s) obtained) after application of the compound (after one or more time interval(s) sufficient for the compound to be internalized and to possibly decrease pain or exert pain as adverse effect), wherein
   the compound is displaying pain as adverse effect, if the expression level of Slfn2 is increased in one or more of the samples obtained after application of the compound in comparison to the level obtained before application of the compound and a decrease in Slfn2 expression level after treatment with the compound in comparison with the level before treatment is indicative of a pain reducing effect of the compound.
9. A method for determining the dosage of a compound for the prevention or treatment of a pain-related tissue status or disease, comprising the steps of
   (i) determining the level of Slfn2 in an individual, and optionally determining the level of Slfn2 in a reference, and
   (ii) determining the dosage of a pharmaceutical depending on the level of Slfn2 in the first individual (e.g. tested sample) optionally in comparison with that of the reference.
10.A method for adapting the dosage of a compound for the prevention or treatment of a pain-related tissue status or disease, comprising the steps of
   (i) determining the level of Slfn2 in an individual (preferably not invasive or sample),
   (ii) determining the level of Slfn2 in one or more references (preferably not invasive such as imaging or samples),
   (iii) examining the tested sample as to whether the level of Slfn2 present in said sample is different from the level in the one or more references (samples), and
   (iv) adapting the dosage of a pharmaceutical depending on whether the level of Slfn2 in the tested sample is different from the level in the one or more reference samples.
11.A method of determining the beneficial and/or adverse effects of a substance on a pain-related tissue status or disease, comprising the steps of
   (i) determining the level of Slfn2 in a test sample,
   (ii) determining the level of Slfn2 in one or more reference samples, and
   (iii) examining the tested sample as to whether the level of Slfn2 present in said sample is different from the level in the one or more reference samples,
   wherein the test sample was exposed differently to said substance than the one or more reference samples.
12. Use of Slfn2 as a target molecule for the discovery of a compound preventing or reducing a pain related tissue status or disease.
13.A kit for detecting the effect of a compound on a pain-related tissue status or a pain-related disease comprising
   a) a means for detecting Slfn2 and optionally
   b) a data carrier comprising instructions for a method according to one of the embodiments 4-19 and optionally
   c) a container.
14.A compound able to lower Slfn2 activity and/or expression for the prevention or treatment of a pain-related tissue status or disease.
15.A method of preventing or treating a pain-related tissue status or disease, wherein a therapeutically effective amount of the compound according to embodiment 14 is administered to an individual at risk of developing a pain-related tissue status or disease or suffering from a pain-related tissue status or disease.
16.The method according to one of the embodiments 1, 7, 8 or 11, the use according to one of the embodiments 2 or 3 or the kit according to embodiment 13, wherein the effect of the compound is
   a) a prevention, reduction or abolishment of a pain-related tissue status or disease, or (Stichwort Pain treatment and prevention)
   b) an induction, increase or progression of a pain-related tissue status disease (Stichwort: Pain as adverse effect of a compound).
17.The method according to one of the embodiments 1, 7-12 or 16, the use according to one of the embodiments 2-6 or 16, the kit according to embodiment 13 or 16 or the compound according to embodiment 14 or 16, wherein the Slfn2 is rodent, monkey or human Slfn2.
18.The method according to one of the embodiments 1, 7-12, 16 or 17, the use according to one of the embodiments 2-6, 16 or 17, the kit according to embodiment 13, 16 or 17 or the compound according to embodiment 14, 16 or 17 wherein Slfn2 is a Slfn2 gene or gene product, preferably a Slfn2 mRNA, or Slfn2 protein or a functionally active variant thereof.
19.The method, use or compound according to embodiment 18, wherein Slfn2 is a gene and wherein the gene
   a) is a naturally occurring or non-naturally occurring Slfn2 gene variant, or
   b) codes for a Slfn2 protein or a functionally active variant thereof that has
      i. an amino acid sequence according to SEQ ID NO: 2, 7 or 9,
      ii. an amino acid sequence that is a fragment of the amino acid sequence according to (i), or
      iii. an amino acid sequence that has at least 80%, at least 90%, at least 95% or at least 99% sequence identity to the amino acid sequence according to (i) or (ii).
20.The method, use or compound according to embodiment 18, wherein Slfn2 is a gene product and wherein the gene product
   a) is a naturally occurring Slfn2 protein variant, preferably a homolog or ortholog in same or different species, or a non-naturally occurring Slfn2 protein variant,
   b) is a Slfn2 protein or a functionally active variant thereof having
      (i) an amino acid sequence according to SEQ ID NO: 2, 7 or 9,
      (ii) an amino acid sequence that is a fragment of the amino acid sequence according to (i), or
      (iii) an amino acid sequence that has at least 80%, at least 90%, at least 95% or at least 99% sequence identity to the amino acid sequence according to (i) or (ii),
   c) is a Slfn2 nucleic acid encoding a protein as defined in b), or
   d) is a Slfn2 nucleic acid having
      (i) a nucleic acid sequence according to SEQ ID NO: 1, 6 or 8,
      (ii) a nucleic acid sequence that is a fragment of the nucleic acid sequence according to (i), or
      (iii) a nucleic acid sequence that has at least 80%, at least 90%, at least 95% or at least 99% sequence identity to the nucleic acid sequence according to (i) or (ii).
21.The method according to embodiment 1 or 16-20, the use according to embodiment 2, 4 or 16-20 or the kit according to embodiment 13 or 16-20, wherein the effect is a decrease or increase in Slfn2 activity or expression.
22.The method, use or kit of embodiment 22, wherein the expression level of Slfn2 is determined on the gene, mRNA or protein level.
23.The use of embodiment 12, 14 or 16-20, wherein Slfn2 is a Slfn2 gene or gene product, preferably a Slfn2 mRNA, or Slfn2 protein or a functionally active variant thereof.
24.The method according to embodiment embodiment 8-10 or 16-20, wherein the expression level of Slfn2 is determined in a sample obtained in a time interval prior to step (i).
25.The method, use or test kit according to embodiment 18, wherein the gene or a functionally active variant thereof is brought into direct or indirect contact with a test compound and the influence of the test compound on the Slfn2 gene or functionally active variant thereof is measured or detected.
26. The method, use or test kit according to embodiment 25, wherein the functionally active variant of the Slfn2 gene is a fragment of a Slfn2 gene, preferably a fragment able to drive expression of a downstream nucleic acid such as a reporter gene if functionally coupled thereto.
27.The method use or test kit according to embodiment 26, wherein the fragment comprises positions -1766 or -1265 to +1 or +50 or +100 or +150 or +198 with respect to the sequence according to SEQ ID NO:5.
28.The method according to one of the embodiments 7-10, 16-20, 21, 22 or 24-27, the use according to embodiment 20 or 21-27 or the test kit according to embodiment 13, 16-20, 21, 22 or 24-27 wherein the level of Slfn2 is detected in a sample, and wherein sample preferably comprises tissue and/or fluid.
29.The method, use or test kit of embodiment 28, wherein the tissue sample is selected from the group consisting of neural or extraneural tissue or cells, preferably neural tissue, and the body fluid sample is selected from the group consisting of cerebrospinal fluid, synovial fluid, serum and plasma.
30.The compound of embodiment 14 further comprising a pharmaceutically acceptable carrier and/or excipient and optionally one or more additional active substances.
31.The method of embodiment 15 or the compound of claims 14 or 30, wherein the compound is administered orally, topically, intravasal, intraspinal or intracavitary or is for oral, topical, intravasal, intraspinal or intracavitary administration.
32. The method according to one of the embodiments 1, 9-11, 15 or 16-22, 24-29 or 31, the use according to one of the embodiments 2-4,12, 16-29, the compound according to embodiment 15, 30 or 31 or the kit according to embodiment 13, 1-22 or 24-29, wherein the pain-related disease is pain or a pain-associated disease.
33. The method, use, test kit or compound according to embodiment 32, wherein the pain-associated disease is migraine, depression or epilepsy.
34. The method, use, test kit or compound according to embodiment 32, wherein the pain-associated tissue status or pain-associated disease is characterized by an altered level of pain-related regulatory molecules or signaling molecules, indicators of pain and/or degenerative molecules present in the tissue.
35. The method, use, test kit or compound according to embodiment 32, wherein the pain-related disease disease is a pain.
36. The method, use, test kit or compound according to embodiment 35, wherein the pain is
   (i) acute pain, subacute pain, or chronic pain,
   (ii) nociceptive pain, neuropathic pain, inflammatory pain, or a mixture thereof,
   (iii) disease-associated pain, or
   (iv) algesia.
37. The method, use, test kit or compound according to embodiment 36, wherein
   (i) the acute pain is somatogenetic (organic) pain,
   (ii) the chronic pain is somatogenetic (organic) pain or psychogenic (psychosomatic) pain,
   (iii) the nociceptive pain is superficial somatic pain or deep pain, preferably deep somatic pain or visceral pain,
   (iv) the neuropathic pain is central neuropathic pain or peripheral neuropathic pain,
   (v) the mixed pain is a mixture of nociceptive pain and neuropathic pain,
   (vi) the disease-associated pain is pain associated with depression, migraine, epilepsy, cancer, or diabetes, or
   (vii) the algesia is hyperalgesia.
38. The method, use, test kit or compound according to embodiment 32, wherein the pain-related tissue status is a tissue damage, trauma, inflammation, infarction, necrosis, edema, neoplasia of any tissue, the tissue preferably being selected from the group consisting of bone, cartilage, muscle, skin, neural tissue, such as a nerve, connective tissue such as perichondrium, periosteum, tendon, capsule, skin and and/or wherein the tissue status is characterized by an altered level of pain-related regulatory molecules or signaling molecules, indicators of pain and/or degenerative molecules present in the tissue.
39. The kit according to embodiment 13, 1-22 or 24-29 or 31-38, wherein the means for detecting Slfn2 is a means for determining the expression level of Slfn2, preferably on the gene, protein or RNA level.
40. The kit according to one of the embodiments 13, 1-22 or 24-29 or 31-39, wherein the means for detecting Slfn2 is selected from the group consisting of nucleic acid, preferably DNA, RNA or PNA, peptide and protein, preferably monoclonal or polyclonal antibody or protein scaffold.
41.The kit according to any of embodiments 13, 1-22 or 24-29 or 31-40, wherein the kit comprises
   (a) a container, and/or
   (b) a data carrier, wherein the data carrier comprises information such as
      (i) instructions concerning methods for identifying the risk for developing and/or identifying the presence and/or monitoring progression of a pain-related tissue status or disease
      (ii) instructions for use of the means for detecting Slfn2, preferably in a sample, more preferably in a sample from an individual and/or of the kit,
      (iii) quality information such as information about the lot/batch number of the means for detecting Slfn2 and/or of the kit, the manufacturing or assembly site or the expiry or sell-by date, information concerning the correct storage or handling of the kit,
      (iv) information concerning the composition of the buffer(s), diluent(s), reagent(s) for detecting Slfn2 and/or of the means for detecting Slfn2,
      (v) information concerning the interpretation of information obtained when performing the above-mentioned methods identifying and/or monitoring progression of a pain-related tissue status or disease,
      (vi) a warning concerning possible misinterpretations or wrong results when applying unsuitable methods and/or unsuitable means, and/or
      (viii) a warning concerning possible misinterpretations or wrong results when using unsuitable reagent(s) and/or buffer(s).
42.The kit according to one of the embodiments 13, 1-22 or 24-29 or 31-41 comprising one or more reference compounds selected from the group consisting of:
   a) a compound inhibiting Slfn2 expression
   b) a compound inhibiting Slfn2 activity
   c) a compound not inhibiting Slfn2 expression
   d) a compound not inhibiting Slfn2 activity.
43.The kit according to one of the embodiments 13, 1-22 or 24-29 or 31-42 comprising instructions for performing one of the methods according to embodiments 1 or 7-11.
44. The kit according to one of the embodiments 13, 1-22 or 24-29 or 31-43, wherein the means comprises
   a) one or more nucleic acids or derivatives for detecting a Slfn2 gene or gene product or functionally active variant thereof, preferably selected from the group consisting of a nucleic acid probe, a polyamide or peptide nucleic acid (PNA), microRNA (miRNA), small interfering RNA (siRNA), primers for polymerase chain reaction (PCR), primers for reverse transcription (RT) reaction, and primers for DNA sequencing and/or
   b) a peptide, polypeptide or protein for detecting a Slfn2 gene or gene product or functionally active variant thereof, preferably selected from the group consisting of a protein ligand, preferably an antibody, a fragment or derivate thereof, a protein scaffold, such as a darpin or an anticalin, or wherein the polypeptide or peptide is a probe, preferably a mass spectrometry probe and/or
   c) a biochip, a set of beads.

### LITERATURE:

Schwarz, D.A., Katayama, C.D. and Hedrick, S.M., Immunity Vol.9 (1998), p.657-668, Schlafen, a New Family of Growth Regulatory Genes that Affect Thymocyte Development.
Wern-Joo Sohn, et al., Molecular Immunology 44 (2007), 3273-3282, Novel transcriptional regulation of the schlafen-2 gene in macrophages in response to TLR-triggered stimulation.
Katsoulidis, E., et al., Journal of Biological Chemistry Vol.284, Nr. 37 (2009), p.25051 - 25064, Role of Schlafen 2 (SLFN2) in the Generation of Interferon alpha-induced Growth Inhibitory Responses.
Horton, M.R. and Powell, J.D., Nature Immunology Vol. 11, Nr.4 (2010), p.281-282, Quieting T cells with Slfn2.
Berger, M. et al., Nature Immunology Vol.11, Nr.4 (2010), p.335-343, A Slfn2 mutation causes lymphoid and myeloid immunodeficiency due to loss of immune cell quiescence.
Bourquin AF et al (2006) Assessment and analysis of mechanical allodynia-like behavior induced by spared nerve injury (SNI) in the mouse. Pain 122:p.1-14.
Storey JD. (2002) A direct approach to false discovery rates. Journal of the Royal Statistical Society, Series B, 64: 479-498.

If not indicated otherwise, standard laboratory methods in the context of present invention were or can be performed according to the following standard literature:
Sambrook et al. (1989) Molecular Cloning: A Laboratory Manual. Second edition. Cold Spring Harbor Laboratory Press, Cold Spring Harbor, NY. 545 pp.
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly updated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994.
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York".
Transgenic Animal Technology A Laboratory Handboook. C.A. Pinkert, editor; Academic Press Inc., San Diego, California, 1994 (ISBN: 0125571658).
Gene targeting: A Practical Approach, 2nd Ed., Joyner AL, ed. 2000. IRL Press at Oxford University Press, New York.
Manipulating the Mouse Embryo: A Laboratory Manual. Nagy, A, Gertsenstein, M., Vintersten, K., Behringer, R., 2003, Cold Spring Harbor Press, New York.
Remington's Pharmaceutical Sciences, 17th Edition, 1985 (for physiologically tolerable salts (anorganic or organic), see esp. p. 1418).

### LEGEND TO THE FIGURES

Figure 1: ClustalW 2.1 (available at http://www.ebi.ac.uk/Tools/msa/clustalw2/) sequence alignment of murine, rat and homo sapiens SIfn2/12L amino acid sequences: sequence 1 is murine (mus musculus) Slfn2 according to AF_099973.1/AAC838261 (SEQ ID NO:2), sequence 2 is rat (rattus norwegicus) Slfn2 according to NP_001100501/NM_001107031.1 (SEQ ID NO:7), sequence 3 is homo sapiens SLFN12L according to NM_001195790.1/NP_001182719.1 (SEQ ID NO:9). The identity score with ClustalW is 83% between mSlfn2 and ratSlfn2, 50% between mSlfn2 and hsSLFN12L and 50% between rat Slfn2 and hsSLFN12L. The conserved variant AAA domain is shown underlined. The isoleucine at position 135 of murine Slfn2 (corresponding to position 135 in rat Slfn2 and position 123 in hsSLFN12L), the mutation of which gives rise to the "electra" phenotype in mice (see description for details) is marked bolt and underlined. As can be gained from the sequence comparison, this position is conserved among the three different species hinting to an equal importance of said amino acid or surrounding protein region within the respective rat and homo sapiens proteins.

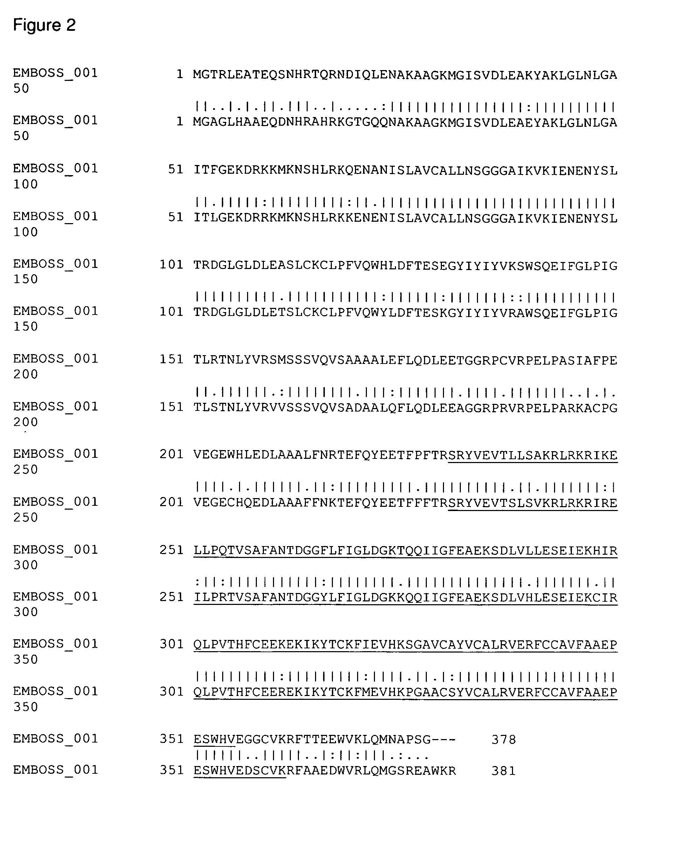

Figure 2: Sequence alignment of murine Slfn2 (SEQ ID NO:2, sequence 1 of comparison) and rat Slfn2 (SEQ ID NO:7, sequence 2 of comparison) using the EMBOSS needle 6.3.1 program (working on Needleman-Wunsch algorithm) of the EMBL EBI database (http://www.ebi.ac.uk/Tools/psa/emboss_needle/). The divergent AAA domains (from position 231 to 355 of murine Slfn2 and from position 231 to 361 of rat Slfn2) as outlined in the NCBI database (see respective accession number) have been marked underlined.
Figure 3: Sequence alignment of murine Slfn2 (SEQ ID NO:2, sequence 1 of comparison) and homo sapiens SLFN12L (SEQ ID NO:9, sequence 2 of comparison) using the EMBOSS needle 6.3.1 program (working on Needleman-Wunsch algorithm) of the EMBL EBI database (http://www.ebi.ac.uk/Tools/psa/emboss_needle/). The divergent AAA domains (from position 231 to 355 of murine Slfn2 and from position 218 to position 349 for hsSLFN12L) as outlined in the NCBI database (see respective accession number) have been marked underlined.
Figure 4: Promoter structure of murine Slfn2 according to Wern-Joo et al, 2009.

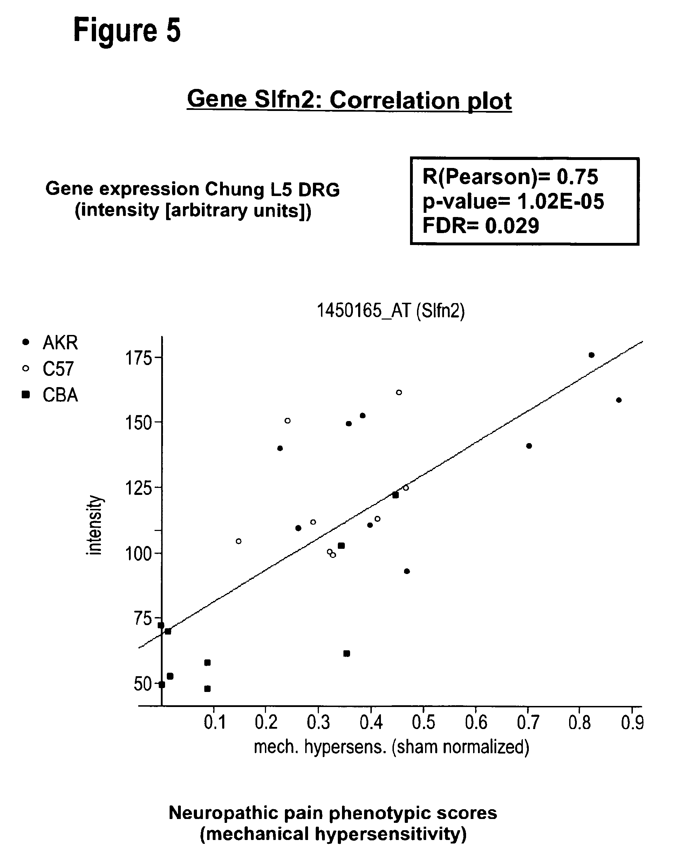

Figure 5: Figure 5 shows for every individual mouse its neuropathic pain phenotype (mechanical hypersensitivity, X-axis) and the corresponding gene expression (signal intensity, Y-axis) in the L5 DRG. Mouse data are colour-coded depending on the used strain. A Pearson correlation analysis has been performed and revealed a significant positive correlation of the two parameters pain phenotype and Slfn2 gene expression. This means for individual mice that the higher the L5 DRG expression of Slfn2 in Chung-operated neuropathic mice was, the more pronounced the mechanical hyperalgesia as exhibited in the behavioral test.
   This significant correlation indicates a causal relationship of Slfn2 gene expression for the induction of the neuropathic pain phenotype.

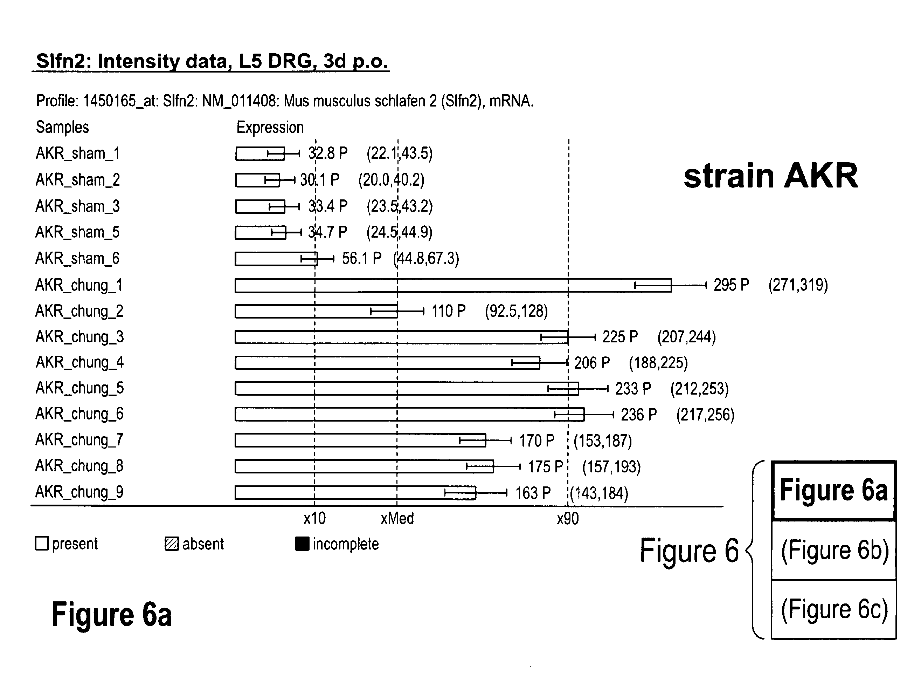

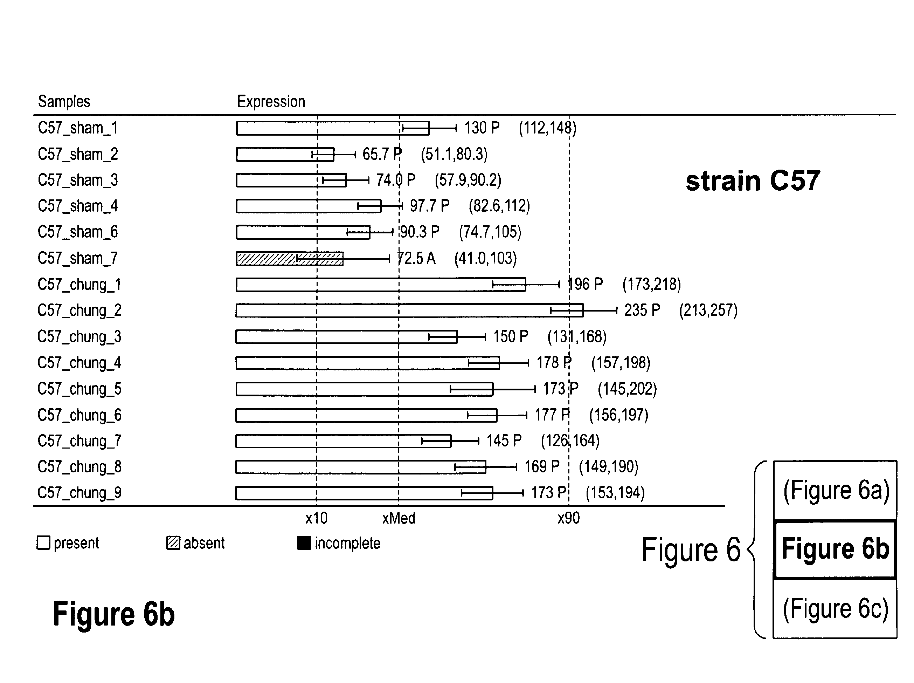

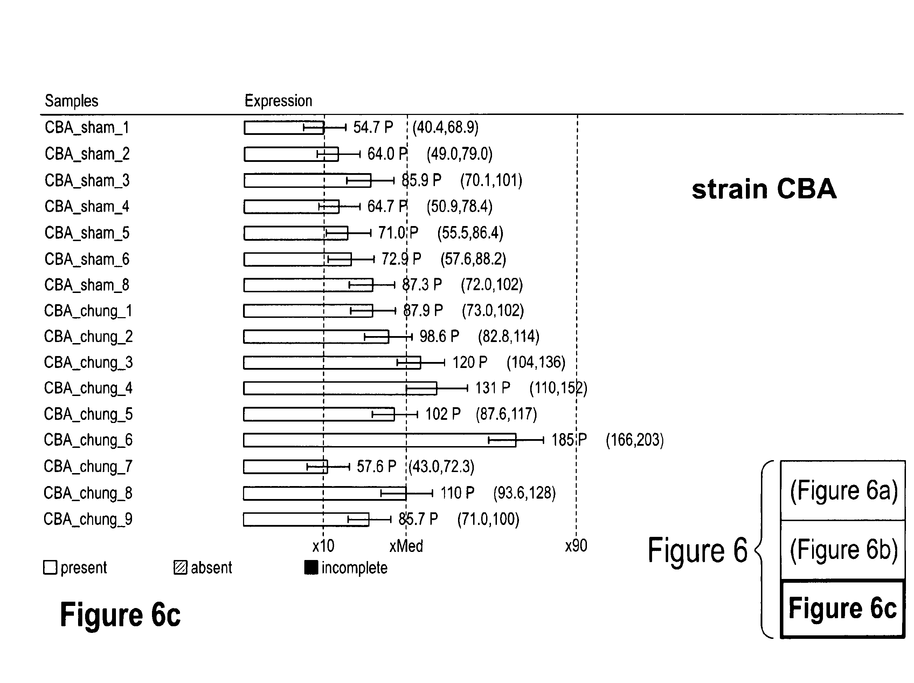

Figure 6: shows exemplary intensity data for Slfn2 of L5 DRG (3d p.o.)

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used but some experimental errors and deviations should be accounted for. Unless indicated otherwise, molecular weight is average molecular weight, temperature is in degrees Centigrade, and pressure is at or near atmospheric. Identification of Slfn2 as protein involved in algesia.

### Example 1

In order to identify new targets for pain therapy, a correlational analysis for identifying genes whose regulation contributes to chronic neuropathic pain was carried out (see also Persson et al., 2009, Molecular Pain 5:7). In summary, RNA samples of dorsal root ganglia (DRGs) of inbred mouse strains AKR/J (AKR), C57BL/6J (C57/B6) and CBA/J (CBA) were examined. Inbred mouse strains obtained from The Jackson Laboratory (Bar Harbor, ME, USA). The spinal nerve at position L5 of Chung-operated (Chung model of neuropathic pain (Kim and Chung, 1992, Pain 50: 355-363) and of corresponding sham-operated control animals were subjected to axotomy.

### Surgical nerve lesion and sham surgical procedures:

In anesthetized mice, the left sciatic nerve was exposed at its trifurcation and the two major branches (tibial and common peroneal nerves) were ligated and transected distal to the ligation. Sham surgery was identical, the nerves were exposed but not ligated and transected (see Bourquin et al. 2006).

### Manifestation of pain phenotype:

The manifestation of the pain phenotype "mechanic hyperalgesia" was determined by means of the paw withdrawal threshold at all mice before removal of DRGs (Persson et al., supra, particularly section "Behavioral testing"). The three mouse strains differ in their phenotypes. In CBA mice, C57/B6 mice and AKR mice, the phenotype is manifested at a low, middle and high level, respectively.

### Determination of paw withdrawal threshold:

Paw withdrawal thresholds (PWTs) were assessed using a dynamic plantar aesthesiometer (see Szabo et al. 2005). After acclimation in a compartment with metal mesh floor, the stimulator was positioned under the animal's hindpaw, a straight metal filament driven by an electrodynamic actuator touched the plantar surface and exerted an increasing upward force until the animal removed the paw (paw withdrawal threshold, PWT). PWTs were assessed for hindpaws of the ipsilateral, operated side and of the contralateral side.

### RNA isolation from dorsal root ganglia:

In order to carry out gene expression experiments, a method for isolating total RNA of murine DRGs was developed (Persson et al., supra, particularly section "RNA extraction for TaqMan and microarray analysis"), wherein the method provided for RNA in a sufficient amount (> 300 ng) and quality. Total RNA from DRGs was isolated with the PicoPure RNA Isolation Kit (Arcturus) following the manufacturer's instructions. RNA quality was assessed using the 2100 Bioanalyzer and RNA 6000 Nano LabChip kit (Agilent). After having extracted RNA from L5 DRGs of the three mouse strains, either Chung-operated or sham-operated control animals, the RNA probes were hybridized on Affymetrix microarrays (MOE430 2.0).

### Affymetrix GeneChip^{™} Microarray-Analysis:

Samples were profiled with Affymetrix microarrays (MOE430 2.0). At least five animals of each group were tested. First-strand cDNA synthesis was performed using 500ng total RNA with a 100pM T7-(dT)24 oligomer (GGCCAGTGAATTGTAATACGACTCACTATAGGGAGGCGG-dT₂₄) according to Baugh, L.R, Hill, A.A., Brown, E.L. and Hunter, C.P. (2001) Nucleic Acids Res. 29, e29 and SuperScript II Reverse Transcriptase following the manufacturer's instructions. Double-stranded cDNA was synthesized and then extracted using phenol-chloroform followed by an ethanol precipitation step. An *in vitro* transcription reaction was performed with the doublestranded cDNA sample using the BioArray High Yield RNA Transcription Labeling kit (Enzo) according to the manufacturer's instructions. Transcription reactions were incubated at 37°C for 16h. cRNA was purified using the RNeasy Mini kit (Qiagen) protocol for RNA cleanup and quantified by a spectrophotometer. The biotin-labeled cRNA was fragmented using a RNA fragmentation buffer (200mM Tris-acetate, 500mM KOAc, 150mM MgOAc, pH 8.1). Hybridization and staining of mouse MOE430_2 GeneChips^{™} (Affymetrix) was performed according to the manufacturer's instructions. The microarrays were scanned using a GeneChip 3000 Scanner, and the scanned data were imported and analyzed using Resolver v5.1 expression data analysis software (Rosetta Biosoftware).

The Affymetrix gene expression data were statistically analyzed and filtered prior to a correlation analysis. The following filter criteria were used:
- Abs. fold-change in at least 60 % of all Chung-operated animals ≥ 1.5 or
- in at least 20 % of all Chung-operated animals ≥ 2.0 (each with respect to the mean value of all sham-operated control animals) and
- gene expression intensity in at least 5 animals > 50 (background level).

Phenotype data of the individual mice of the three strains and their gene expression data (expressed as log ratio (Chung-operated vs. sham-operated)) or expression intensity of Chung-operated animals were used for correlation analysis.

For each gene which fulfilled the above filter criteria, a Pearson correlation coefficient of gene expression data and phenotype data (mechanic hypersensitivity) was calculated (Persson et al., supra, particularly section "Correlational analysis"). In order to determine the significance of correlation coefficients of the single genes, a "false discovery rate" (FDR) was introduced (Storey, J.D. (2002) J. R. Statist. Soc. 64, part 3, 479-498). Pearson correlation coefficients of genes having an FDR > 0.05 were regarded as significant. Using the log ratio data (Chung-operated vs. sham-operated) and expression intensity 74 sequences and 114 genes, respectively, were identified. Slfn2 was one of the genes identified to exhibit the best correlation data. The data for these sequences/genes, especially Slfn2, showed a significant correlation of gene expression and phenotype data (FDR < 0.05). Slfn2 was not previously known to be involved in pain and hyperalgesia.

### Correlational analysis:

For correlational analysis, the "pain phenotype" was defined for each nerve-transsected animal (Chung animal) as *C1* - *S1*, where *C1* = *In(ipsilateral PWT* / *contralateral PWT) and S1* = *mean_{all sham animals within same strain} In(ipsilateral PWT* / *contralateral PWT).*

Two measures of differential transcriptional regulation were defined for each Chung animal and each measured gene based on its intensity expression data. The "raw intensity measure" was taken as the intensity measure computed by the Resolver expression data analysis software (v5.1) for the respective gene and animal. The "log ratio measure" was computed for a specific gene and Chung animal as *In(C2* / *S2),* where *C2* = *Chung expression intensity* and *S2* = *mean_{all sham animals within same strain} Sham expression intensity.*

Before correlations were computed, the set of genes was filtered to exclude genes that were expressed below noise level and without significant Chung vs. sham regulation. Eligible genes must be regulated in at least 60% of Chung animals with an absolute fold-change =>1.5 or in at least 20% of Chung animals with an absolute fold-change =>2.0. Also, corresponding gene expression had to be detectable ("present") in at least five animals as defined by a respective intensity p-value< 0.001.

Pearson correlation coefficients for each gene between the pain phenotypic scores and one of the defined measures of transcriptional regulation were computed using the R software package (http://www.r-project.org/). Based on these, p-values of statistical significance and corresponding false-discovery rates (FDRs) were generated following the method of Storey et al. (2002). Genes with FDR< 0.05 under "log ratio measure" or "intensity measure" were considered significantly correlated.

### Slfn2:

For the Slfn2 gene which was among the genes with the best correlation of expression and pain phenotype, the correlation analysis of log ratio data of L5 DRG signal intensities and mechanic hypersensitivity yielded a Pearson correlation coefficient of 0.75 (p value of 1.02* 10⁻⁵) and an FDR of 0.029.

## Claims

1. A method of detecting the effect of a compound on a pain-related tissue status or a pain-related disease, the method comprising the steps of:
(a) providing a test system biochem/cell/animal(not suffering from disease) comprising Slfn2,
(b) contacting the test system with a test compound, and
(c) determining the effect of the test compound on the test system,
wherein the test compound is identified as being active in modulating a pain-related tissue status or disease, if a significant effect (pos or neg) of the test compound on the test system relative to a control is detected.

2. Use of Slfn2 for detecting the effect of a compound on a pain-related tissue status or a pain-related disease.

3. Use of Slfn2 for determining the dosage of a compound for the prevention or treatment of a pain-related tissue status or disease.

4. Use of a cell overexpressing Slfn2 or of a non-human transgenic animal heterologously expressing Slfn2 for determining the effect of a compound on a pain-related tissue status or disease.

5. Use of a cell overexpressing Slfn2 or of a non-human transgenic animal heterologously expressing Slfn2 as a model system for enhanced pain sensitivity.

6. Use of a Slfn2 knock-out cell or a non-human Slfn2 knock-out animal as a model system for lowered pain sensitivity.

7. A method for determining a pain-modulating effect of a compound comprising
(i) administering a test compound to a subject (human or non-human animal (not ill)
(ii) detecting whether said compound increases or decreases the Slfn2 expression level in said subject in comparison to a control animal not having been administered said compound, sample or imaging
wherein a decrease in Slfn2 expression level after treatment with the compound in comparison to the level in the control animal is indicative of a pain reducing effect of the compound and an increase is indicative of pain as (adverse) effect of the compound.

8. A method of for determining a pain-modulating effect of a compound comprising
(i) determining the expression level of Slfn2 in a subject sample or imaging, animal or human not ill,
(ii) applying a compound to the subject, and
(iii) determining the expression level of Slfn2 (in one or more sample(s) obtained) after application of the compound (after one or more time interval(s) sufficient for the compound to be internalized and to possibly decrease pain or exert pain as adverse effect), wherein
the compound is displaying pain as adverse effect, if the expression level of Slfn2 is increased in one or more of the samples obtained after application of the compound in comparison to the level obtained before application of the compound and a decrease in Slfn2 expression level after treatment with the compound in comparison with the level before treatment is indicative of a pain reducing effect of the compound. (obtained from the first subject after application of the compound in comparison to the level in one or more of the samples obtained from second subject obtained after principally the same time intervals as for the first subject).

9. A method for determining the dosage of a compound for the prevention or treatment of a pain-related tissue status or disease, comprising the steps of
(i) determining the level of Slfn2 (in a sample of the sagen- ind/sample) in an individual, and optionally determining the level of Slfn2 in a reference (sample for comparison with the level of Slfn2 in the sample), and
(ii) determining the dosage of a pharmaceutical depending on the level of Slfn2 in the first individual (e.g. tested sample) optionally in comparison with that of the reference.

10. A method for adapting the dosage of a compound for the prevention or treatment of a pain-related tissue status or disease, comprising the steps of
(i) determining the level of Slfn2 in an individual (preferably not invasive or sample),
(ii) determining the level of Slfn2 in one or more references (preferably not invasive such as imaging or samples),
(iii) examining the tested sample as to whether the level of Slfn2 present in said sample is different from the level in the one or more references (samples), and
(d) adapting the dosage of a pharmaceutical depending on whether the level of Slfn2 in the tested sample is different from the level in the one or more reference samples.

11. A method of determining the beneficial and/or adverse effects of a substance on a pain-related tissue status or disease, comprising the steps of
(i) determining the level of Slfn2 in a test sample,
(ii) determining the level of Slfn2 in one or more reference samples, and
(iii) examining the tested sample as to whether the level of Slfn2 present in said sample is different from the level in the one or more reference samples,
wherein the test sample was exposed differently to said substance than the one or more reference samples.

12. Use of Slfn2 as a target molecule for the discovery of a compound preventing or reducing a pain related tissue status or disease.

13. A kit for detecting the effect of a compound on a pain-related tissue status or a
pain-related disease comprising
a. a means for detecting Slfn2 and optionally
b. a data carrier comprising instructions for a method according to one of the claims 4-19 and optionally
c. a container.

14. A compound able to lower Slfn2 activity and/or expression for the prevention or treatment of a pain-related tissue status or disease.

15. A method of preventing or treating a pain-related tissue status or disease, wherein a therapeutically effective amount of the compound according to claim 14 is administered to an individual at risk of developing a pain-related tissue status or disease or suffering from a pain-related tissue status or disease.


SEQUENCE LISTING

<110>  SANOFI

<120>  Methods and uses relating to the identification and profiling of
       compounds for use in the treatment or prevention of pain

<130>  DE2011/128

<160>  14

<170>  PatentIn version 3.5

<210>  1
<211>  1684
<212>  DNA
<213>  Mus musculus

<400>  1
gctttaatgc agcaaggaac aaagaactca catgggctta gaggtgaaca cacaaccagg      60

gctgcagtta gaactcttcc aagtgccaaa gcccaccggc gaagacatct gctggccagg     120

acttgttgca gacagagggt ctttctcagg tgacactagc ctgctttacc aagactgctg     180

acatgggtac tagacttgag gcaactgagc aaagcaacca cagaactcag aggaatgaca     240

ttcagctgga aaatgcaaag gctgctggga aaatgggcat cagtgttgat ctggaagcca     300

aatatgctaa gctgggtcta aatctcggag caatcacttt tggagagaag gataggaaga     360

aaatgaagaa ttctcacctc agaaaacagg agaatgcaaa catctctcta gctgtatgtg     420

ctctcctgaa ttcgggaggt ggagcaatca aggttaaaat tgaaaatgaa aattatagtc     480

tcactaggga tggcctggga ctagatttgg aagcctctct ttgtaaatgt ctgccctttg     540

tccagtggca cctggacttc acggagagcg aaggctacat ttatatctac gtgaaatcgt     600

ggagccaaga aatctttggg ctgcctattg gcaccctaag aaccaatttg tatgtaagga     660

gcatgtcatc ttctgtacaa gtgagcgccg ctgctgccct ggaatttctc caggacctgg     720

aggaaactgg agggagaccc tgtgtcagac cagagttgcc tgcaagcata gccttccctg     780

aagtggaagg agaatggcac ctggaggatt tggctgctgc attgtttaac aggacagaat     840

ttcagtacga ggaaactttc ccctttacca gatccagata tgttgaagtt acattgcttt     900

cagcgaaacg cctgcgaaaa cgcatcaaag agctcctccc tcaaactgtt tctgcttttg     960

caaacacgga tgggggattt ttgttcattg gtttggatgg caaaacccag caaattattg    1020

gttttgaagc agagaagagc gatctcgtgc ttctagagag tgaaatagaa aagcacatcc    1080

ggcagctgcc tgtcactcac ttctgtgagg agaaggagaa gatcaagtac acgtgcaaat    1140

tcatcgaagt gcacaaatcc ggagctgtgt gtgcatatgt gtgtgcgctc agagtggaga    1200

gattctgctg tgcagtattc gctgcagagc ccgagtcctg gcatgtggaa ggcggctgtg    1260

tgaagaggtt taccacagag gaatgggtga aactccagat gaatgcccca tcaggttgaa    1320

gggggattaa taatctgtat gagggcggca gagatggctc agtggctaag agcactgact    1380

gctcttccag agatctgagc tgtaaccccc cccccccagc ctgaaacctg gttgctcagg    1440

tttcactgtt agcaagaaga gagcatggga gaggggggag gagcctgccg ccctatcgtt    1500

catcctaccc tttgtccacc cgagactaga ggggtgtgtc cgttccacgc agacaaaggg 1560

gcccagaggg tctgtggcag tggtcacctg aaactggact ccaggatgat ttggcgggaa 1620

tgggcccctt ccccctcctt cataaaattg agctttgctc agaaaaaaaa aaaaaaaaaa 1680

aaaa 1684

<210> 2
<211> 378
<212> PRT
<213> Mus musculus

<400> 2

Met Gly Thr Arg Leu Glu Ala Thr Glu Gln Ser Asn His Arg Thr Gln
1               5                   10                  15

Arg Asn Asp Ile Gln Leu Glu Asn Ala Lys Ala Ala Gly Lys Met Gly
            20                  25                  30

Ile Ser Val Asp Leu Glu Ala Lys Tyr Ala Lys Leu Gly Leu Asn Leu
        35                  40                  45

Gly Ala Ile Thr Phe Gly Glu Lys Asp Arg Lys Lys Met Lys Asn Ser
    50                  55                  60

His Leu Arg Lys Gln Glu Asn Ala Asn Ile Ser Leu Ala Val Cys Ala
65                  70                  75                  80

Leu Leu Asn Ser Gly Gly Gly Ala Ile Lys Val Lys Ile Glu Asn Glu
                85                  90                  95

Asn Tyr Ser Leu Thr Arg Asp Gly Leu Gly Leu Asp Leu Glu Ala Ser
            100                 105                 110

Leu Cys Lys Cys Leu Pro Phe Val Gln Trp His Leu Asp Phe Thr Glu
        115                 120                 125

Ser Glu Gly Tyr Ile Tyr Ile Tyr Val Lys Ser Trp Ser Gln Glu Ile
    130                 135                 140

Phe Gly Leu Pro Ile Gly Thr Leu Arg Thr Asn Leu Tyr Val Arg Ser
145                 150                 155                 160

Met Ser Ser Ser Val Gln Val Ser Ala Ala Ala Ala Leu Glu Phe Leu
                165                 170                 175

Gln Asp Leu Glu Glu Thr Gly Gly Arg Pro Cys Val Arg Pro Glu Leu
            180                 185                 190

Pro Ala Ser Ile Ala Phe Pro Glu Val Glu Gly Glu Trp His Leu Glu
        195                 200                 205

Asp Leu Ala Ala Ala Leu Phe Asn Arg Thr Glu Phe Gln Tyr Glu Glu

210                 215                 220

Thr Phe Pro Phe Thr Arg Ser Arg Tyr Val Glu Val Thr Leu Leu Ser
225                 230                 235                 240

Ala Lys Arg Leu Arg Lys Arg Ile Lys Glu Leu Leu Pro Gln Thr Val
                245                 250                 255

Ser Ala Phe Ala Asn Thr Asp Gly Gly Phe Leu Phe Ile Gly Leu Asp
            260                 265                 270

Gly Lys Thr Gln Gln Ile Ile Gly Phe Glu Ala Glu Lys Ser Asp Leu
        275                 280                 285

Val Leu Leu Glu Ser Glu Ile Glu Lys His Ile Arg Gln Leu Pro Val
    290                 295                 300

Thr His Phe Cys Glu Glu Lys Glu Lys Ile Lys Tyr Thr Cys Lys Phe
305                 310                 315                 320

Ile Glu Val His Lys Ser Gly Ala Val Cys Ala Tyr Val Cys Ala Leu
                325                 330                 335

Arg Val Glu Arg Phe Cys Cys Ala Val Phe Ala Ala Glu Pro Glu Ser
            340                 345                 350

Trp His Val Glu Gly Gly Cys Val Lys Arg Phe Thr Thr Glu Glu Trp
        355                 360                 365

Val Lys Leu Gln Met Asn Ala Pro Ser Gly
    370                 375

<210>  3
<211>  1696
<212>  DNA
<213>  Mus musculus

<400>  3
agtctgtgca atgctttaat gcagcaagga acaaagaact cacatgggct tagaggtgaa      60

cacacaacca gggctgcagt tagaactctt ccaagtgcca aagcccaccg gcgaagacat     120

ctgctggcca ggacttgttg cagacagagg gtctttctca ggtgacacta gcctgcttta     180

ccaagactgc tgacatgggt actagacttg aggcaactga gcaaagcaac cacagaactc     240

agaggaatga cattcagctg gaaaatgcaa aggctgctgg gaaaatgggc atcagtgttg     300

atctggaagc caaatatgct aagctgggtc taaatctcgg agcaatcact tttggagaga     360

aggataggaa gaaaatgaag aattctcacc tcagaaaaca ggagaatgca aacatctctc     420

tagctgtatg tgctctcctg aattcgggag gtggagcaat caaggttaaa attgaaaatg     480

aaaattatag tctcactagg gatggcctgg gactagattt ggaagcctct ctttgtaaat     540

gtctgccctt tgtccagtgg cacctggact tcacggagag cgaaggctac atttatatct     600

acgtgaaatc gtggagccaa gaaatctttg ggctgcctat tggcacccta agaaccaatt 660

tgtatgtaag gagcatgtca tcttctgtac aagtgagcgc cgctgctgcc ctggaatttc 720

tccaggacct ggaggaaact ggagggagac cctgtgtcag accagagttg cctgcaagca 780

tagccttccc tgaagtggaa ggagaatggc acctggagga tttggctgct gcattgttta 840

acaggacaga atttcagtac gaggaaactt tcccctttac cagatccaga tatgttgaag 900

ttacattgct ttcagcgaaa cgcctgcgaa aacgcatcaa agagctcctc cctcaaactg 960

tttctgcttt tgcaaacacg gatgggggat ttttgttcat tggtttggat ggcaaaaccc 1020

agcaaattat tggttttgaa gcagagaaga gcgatctcgt gcttctagag agtgaaatag 1080

aaaagcacat ccggcagctg cctgtcactc acttctgtga ggagaaggag aagatcaagt 1140

acacgtgcaa attcatcgaa gtgcacaaat ccggagctgt gtgtgcatat gtgtgtgcgc 1200

tcagagtgga gagattctgc tgtgcagtat tcgctgcaga gcccgagtcc tggcatgtgg 1260

aaggcggctg tgtgaagagg tttaccacag aggaatgggt gaaactccag atgaatgccc 1320

catcaggttg aagggggatt aataatctgt atgagggcgg cagagatggc tcagtggcta 1380

agagcactga ctgctcttcc agagatctga gctgtaaccc ccccccccca gcctgaaacc 1440

tggttgctca ggtttcactg ttagcaagaa gagagcatgg gagagggggg aggagcctgc 1500

cgccctatcg ttcatcctac cctttgtcca cccgagacta gaggggtgtg tccgttccac 1560

gcagacaaag gggcccagag ggtctgtggc agtggtcacc tgaaactgga ctccaggatg 1620

atttggcggg aatgggcccc ttccccctcc ttcataaaat tgagctttgc tcagaaaaaa 1680

aaaaaaaaaa aaaaaa 1696

<210> 4
<211> 1767
<212> DNA
<213> Mus musculus

<400> 4
ttggtggctc ctagcgttgt acaatcacat tttgacacaa ggggttgggc aatggtgtct 60

ccattgctct tttgctctga cttaggcagg gcggggtggg ggtgggggtt ggtaggtggg 120

gcagttgtca gtcataggac aggagaaagt attggtgata aattggtgat taggccttat 180

ctgggccacc tggcttgaag gtctttgact ccaagccctg gtggtcaggc cttaccctgg 240

atatttgacc tgtaactcaa gatgagaagg caacaaagga gctagttcct gggaacatag 300

actcagctgg accctgccag ccgatggaga acttgtcccc gaggtggctg gactcggaga 360

gctgtctcct tataaatgct ctcctggctg cctccttcct taagcctggg cactgcactt 420

tgtccttgat ggagaagtcc ccagagtttg ctgcagttct aatcccagac ctttggagtt 480

gaattccttc tcactgactt tggaactgaa ctggagctcg gaggaggagc tcggaggagg 540

agctcggagg aggaggagga ggaggaggag gagggaggag ggaggagagt gaagagggga 600

gagaaagaag agaagacttc aggcacacag agcatgggca ctggactttc tccacccagc 660

agctgcctct gtccagatgc tgctcctccc tggctccccc actttcccta cccctgttct 720

tttcttcctc atgtccctca aagccctggt tgttggcttg ccctggtctc tggctagtct 780

tgtgacattc cttaggaacg ggagggtaca gttctgagca agaggttgtg cttgggatac 840

tggaggaggg tgactcattg gactccccct agcaacttct tctttcacct cctgacaagt 900

acagaaggaa aattttgtct ttcttcattt ttggtcttct tcattactga tgcaaatatt 960

ctcctttacg tttttgcatg tttccatcaa cccacgagaa ctccagaaac ctcctgtact 1020

ttatttctta aattagagat taagaaagag attctaaagg tctctgagtc agtttcctca 1080

gagctggtct ggtggtgaca ggcaatgaaa cagcccacag ggaagaaaga gccctgttct 1140

ggacccttag gctggaggga cagtggagtt ctggacctct ggaagggcag tcagtgctct 1200

tatccactga accatctctc cagccctatt tcttttcttt tcttatcttt tcttttttaa 1260

tatttatttt attggatatt ttctttattt acatttcaaa tgttatcccc tttcctggtc 1320

ctggccccgg tccttccttc ctcccccaac tccctctccc atccaccccc aactcccttt 1380

cccttccctc aagccccctc tcccacccca ccccccctcc ccaatcagtt tcttaaggct 1440

ggttagggag gtgttactgt aacaaagtag caaaagaggt gtactgcttt agggtagccc 1500

tgggaatccc atagagagaa ggaatctgct ggcagagagg atcctgctgt gcccatctta 1560

aacccgggac tggagggact ctaaaggcgt taacaagggc acctgctctt ttgagcaaat 1620

ggactttgct gcttgggaat gcctgaggca gatagaaatc tgactagtca actgtgagct 1680

tgtgttacag tctgcccaat agaaaagtgg aggcagggct ttcacttcct ggagggtgac 1740

agttctggga tttccagtct tccacca 1767

<210> 5
<211> 3461
<212> DNA
<213> Mus musculus

<400> 5
ttggtggctc ctagcgttgt acaatcacat tttgacacaa ggggttgggc aatggtgtct 60

ccattgctct tttgctctga cttaggcagg gcggggtggg ggtgggggtt ggtaggtggg 120

gcagttgtca gtcataggac aggagaaagt attggtgata aattggtgat taggccttat 180

ctgggccacc tggcttgaag gtctttgact ccaagccctg gtggtcaggc cttaccctgg 240

atatttgacc tgtaactcaa gatgagaagg caacaaagga gctagttcct gggaacatag 300

actcagctgg accctgccag ccgatggaga acttgtcccc gaggtggctg gactcggaga 360

gctgtctcct tataaatgct ctcctggctg cctccttcct taagcctggg cactgcactt 420

tgtccttgat ggagaagtcc ccagagtttg ctgcagttct aatcccagac ctttggagtt 480

gaattccttc tcactgactt tggaactgaa ctggagctcg gaggaggagc tcggaggagg 540

agctcggagg aggaggagga ggaggaggag gagggaggag ggaggagagt gaagagggga 600

gagaaagaag agaagacttc aggcacacag agcatgggca ctggactttc tccacccagc 660

agctgcctct gtccagatgc tgctcctccc tggctccccc actttcccta cccctgttct 720

tttcttcctc atgtccctca aagccctggt tgttggcttg ccctggtctc tggctagtct 780

tgtgacattc cttaggaacg ggagggtaca gttctgagca agaggttgtg cttgggatac 840
tggaggaggg tgactcattg gactccccct agcaacttct tctttcacct cctgacaagt 900
acagaaggaa aatttgtctt ttcttcattt ttggtcttct tcattactga tgcaaatatt 960
ctcctttacg tttttgcatg tttccatcaa cccacgagaa ctccagaaac ctcctgtact 1020
ttatttctta aattagagat taagaaagag attctaaagg tctctgagtc agtttcctca 1080
gagctggtct ggtggtgaca ggcaatgaaa cagcccacag ggaagaaaga gccctgttct 1140
ggacccttag gctggaggga cagtggagtt ctggacctct ggaagggcag tcagtgctct 1200
tatccactga accatctctc cagccctatt tcttttcttt tcttatcttt tcttttttaa 1260
tatttatttt attggatatt ttctttattt acatttcaaa tgttatcccc tttcctggtc 1320
ctggccccgg tccttccttc ctcccccaac tccctctccc atccaccccc aactcccttt 1380
cccttccctc aagccccctc tcccacccca ccccccctcc ccaatcagtt tcttaaggct 1440
ggttagggag gtgttactgt aacaaagtag caaaagaggt gtactgcttt agggtagccc 1500
tgggaatccc atagagagaa ggaatctgct ggcagagagg atcctgctgt gcccatctta 1560
aacccgggac tggagggact ctaaaggcgt taacaagggc acctgctctt ttgagcaaat 1620
ggactttgct gcttgggaat gcctgaggca gatagaaatc tgactagtca actgtgagct 1680
tgtgttacag tctgcccaat agaaaagtgg aggcagggct ttcacttcct ggagggtgac 1740
agttctggga tttccagtct tccaccagtc tgtgcaatgc tttaatgcag caaggaacaa 1800
agaactcaca tgggcttaga ggtgaacaca caaccagggc tgcagttaga actcttccaa 1860
gtgccaaagc ccaccggcga agacatctgc tggccaggac ttgttgcaga cagagggtct 1920
ttctcagtga cactagcctg ctttaccaag actgctgaca tgggtactag acttgaggca 1980
actgagcaaa gcaaccacag aactcagagg aatgacattc agctggaaaa tgcaaaggct 2040
gctgggaaaa tgggcatcag tgttgatctg gaagccaaat atgctaagct gggtctaaat 2100
ctcggagcaa tcacttttgg agagaaggat aggaagaaaa tgaagaattc tcacctcaga 2160
aaacaggaga atgcaaacat ctctctagct gtatgtgctc tcctgaattc gggaggtgga 2220
gcaatcaagg ttaaaattga aaatgaaaat tatagtctca ctagggatgg cctgggacta 2280
gatttggaag cctctctttg taaatgtctg ccctttgtcc agtggcacct ggacttcacg 2340
gagagcgaag gctacattta tatctacgtg aaatcgtgga gccaagaaat ctttgggctg 2400
cctattggca ccctaagaac caatttgtat gtaaggagca tgtcatcttc tgtacaagtg 2460
agcgccgctg ctgccctgga atttctccag gacctggagg aaactggagg gagaccctgt 2520
gtcagaccag agttgcctgc aagcatagcc ttccctgaag tggaaggaga atggcacctg 2580
gaggatttgg ctgctgcatt gtttaacagg acagaatttc agtacgagga aactttcccc 2640
tttaccagat ccagatatgt tgaagttaca ttgctttcag cgaaacgcct gcgaaaacgc 2700
atcaaagagc tcctccctca aactgtttct gcttttgcaa acacggatgg gggatttttg 2760
ttcattggtt tggatggcaa aacccagcaa attattggtt ttgaagcaga gaagagcgat 2820
ctcgtgcttc tagagagtga aatagaaaag cacatccggc agctgcctgt cactcacttc 2880

tgtgaggaga aggagaagat caagtacacg tgcaaattca tcgaagtgca caaatccgga 2940

gctgtgtgtg catatgtgtg tgcgctcaga gtggagagat tctgctgtgc agtattcgct 3000

gcagagcccg agtcctggca tgtggaaggc ggctgtgtga agaggtttac cacagaggaa 3060

tgggtgaaac tccagatgaa tgccccatca ggttgaaggg ggattaataa tctgtatgag 3120

ggcggcagag atggctcagt ggctaagagc actgactgct cttccagaga tctgagctgt 3180

aacccccccc ccccagcctg aaacctggtt gctcaggttt cactgttagc aagaagagag 3240

catgggagag gggggaggag cctgccgccc tatcgttcat cctacccttt gtccacccga 3300

gactagaggg gtgtgtccgt tccacgcaga caaaggggcc cagagggtct gtggcagtgg 3360

tcacctgaaa ctggactcca ggatgatttg gcgggaatgg gccccttccc cctccttcat 3420

aaaattgagc tttgctcaga aaaaaaaaaa aaaaaaaaaa a 3461

<210> 6
<211> 2078
<212> DNA
<213> Rattus norvegicus

<400> 6
cttcaatgca gcaaagaaca gagaactcaa gtgagcttag aggtgagctc acagccaggg 60

ctgcagatag aactctccca agtgccaaca accagcctcg aagacgtccc cggccaggac 120

ttgttgcaga caacactagt tgtctttctc aagtgacact agcctgcctc accaaaggag 180

ctgactgctg gcatgggcgc tggacttcat gcagctgagc aagacaacca cagagctcac 240

aggaagggca ctgggcagca aaatgcaaaa gctgctggga aaatgggcat cagcgttgac 300

ctggaagctg agtatgctaa gctgggtcta aatctaggag caatcactct tggagagaag 360

gataggagga aaatgaagaa ctctcacctc aggaaaaagg agaatgagaa catctctctg 420

gctgtgtgtg ctctcctgaa ttcaggaggt ggagcaatca aggttaaaat tgaaaatgaa 480

aattatagtc tcactaggga tggcctggga ctagatttgg aaacctctct ttgtaaatgt 540

ctcccgtttg tccagtggta cctggacttc actgagagca aaggctacat ttatatctac 600

gtgagagcat ggagtcagga aatctttggg ctgccgattg gcaccctgag caccaatttg 660

tacgtaaggg tcgtttcatc ctccgtacaa gtgagcgctg atgctgcgct acaatttctc 720

caggacctgg aggaagctgg agggagaccc cgtgtcagac cagagttgcc tgcaaggaaa 780

gcctgccctg gagtggaagg agaatgtcac caggaggatt tggctgctgc attttttaac 840

aagaccgaat ttcagtatga ggaaactttc ttctttacca ggtccaggta tgttgaagtt 900

acatcacttt cagtgaaacg cctgcgaaaa cgcatcagag agatcctccc tcgaactgtt 960

tctgcattcg caaacacgga tgggggatat ttgttcattg gcttggatgg gaaaaaacag 1020

caaataattg gctttgaagc agagaagagc gaccttgtgc atctagagag tgaaatagaa 1080

aagtgcatcc gacagctgcc tgtcactcac ttctgtgagg agagggagaa gatcaagtac 1140

acgtgcaaat tcatggaagt gcacaaaccc ggagccgcgt gttcatacgt gtgtgcgctc 1200

cgagtggaga gattctgctg tgcagtgttc gctgcagagc ccgaatcctg gcacgtggaa 1260

gacagctgtg tgaagaggtt tgccgcagag gactgggtta ggctccagat gggttccagg 1320

gaagcttgga aaaggtgact aataatcagt tagtgaaaaa taaagatttt taagttcccc 1380

acttgaccct gctgtgagga caagtgcacc tcccaaaaga atagcatcac atgttttggt 1440

gaggtagcct ggctatagcc accacaaacc atctggcctc gataaggcct gacacaacag 1500

ttctgaggaa aacaagaact agggtcagct tgccccagaa gcagggtggc ctagagttga 1560

actgaggcca tgtagttttc cctataaatc ccttccccta attgggctcg gggttgactc 1620

ctctgtctcc tgtgtgagat acgtgtcatc cccagagctc tggcttctca aatatactta 1680

ttatatcaag accggtttct cgtgagttct tgggggttgc gtcatcttga gatttgagtg 1740

ggggtcttcc ccaccttggt ggtctttcat tacaaccatc tgtaatgata tctgatgccc 1800

tcttctaact attttccagc atccacatgg tgattcacaa ccacctagaa ccccagttta 1860

tttatttttt ttggctgttt tttttttttt tttggtttta catggagtgg tttaatgaga 1920

gaagaagggt agaagaggag aggaacaaag gaggctggcc atgggcacgt gggggaaggg 1980

ggggatgggg agagaaggga cagggacaaa gaggacacca gaagaggaga gcaagagaga 2040

ggagtaagag caaactgatg agcttccagg gactaagc 2078

<210> 7
<211> 381
<212> PRT
<213> Rattus norvegicus

<400> 7

Met Gly Ala Gly Leu His Ala Ala Glu Gln Asp Asn His Arg Ala His
1               5                   10                  15

Arg Lys Gly Thr Gly Gln Gln Asn Ala Lys Ala Ala Gly Lys Met Gly
            20                  25                  30

Ile Ser Val Asp Leu Glu Ala Glu Tyr Ala Lys Leu Gly Leu Asn Leu
        35                  40                  45

Gly Ala Ile Thr Leu Gly Glu Lys Asp Arg Arg Lys Met Lys Asn Ser
    50                  55                  60

His Leu Arg Lys Lys Glu Asn Glu Asn Ile Ser Leu Ala Val Cys Ala
65                  70                  75                  80

Leu Leu Asn Ser Gly Gly Gly Ala Ile Lys Val Lys Ile Glu Asn Glu
                85                  90                  95

Asn Tyr Ser Leu Thr Arg Asp Gly Leu Gly Leu Asp Leu Glu Thr Ser
            100                 105                 110

Leu Cys Lys Cys Leu Pro Phe Val Gln Trp Tyr Leu Asp Phe Thr Glu
        115                 120                 125

Ser Lys Gly Tyr Ile Tyr Ile Tyr Val Arg Ala Trp Ser Gln Glu Ile
    130                 135                 140

Phe Gly Leu Pro Ile Gly Thr Leu Ser Thr Asn Leu Tyr Val Arg Val
145                 150                 155                 160

Val Ser Ser Ser Val Gln Val Ser Ala Asp Ala Ala Leu Gln Phe Leu
                165                 170                 175

Gln Asp Leu Glu Glu Ala Gly Gly Arg Pro Arg Val Arg Pro Glu Leu
            180                 185                 190

Pro Ala Arg Lys Ala Cys Pro Gly Val Glu Gly Glu Cys His Gln Glu
        195                 200                 205

Asp Leu Ala Ala Ala Phe Phe Asn Lys Thr Glu Phe Gln Tyr Glu Glu
    210                 215                 220

Thr Phe Phe Phe Thr Arg Ser Arg Tyr Val Glu Val Thr Ser Leu Ser
225                 230                 235                 240

Val Lys Arg Leu Arg Lys Arg Ile Arg Glu Ile Leu Pro Arg Thr Val
                245                 250                 255

Ser Ala Phe Ala Asn Thr Asp Gly Gly Tyr Leu Phe Ile Gly Leu Asp
            260                 265                 270

Gly Lys Lys Gln Gln Ile Ile Gly Phe Glu Ala Glu Lys Ser Asp Leu
        275                 280                 285

Val His Leu Glu Ser Glu Ile Glu Lys Cys Ile Arg Gln Leu Pro Val
    290                 295                 300

Thr His Phe Cys Glu Glu Arg Glu Lys Ile Lys Tyr Thr Cys Lys Phe
305                 310                 315                 320

Met Glu Val His Lys Pro Gly Ala Ala Cys Ser Tyr Val Cys Ala Leu
                325                 330                 335

Arg Val Glu Arg Phe Cys Cys Ala Val Phe Ala Ala Glu Pro Glu Ser
            340                 345                 350

Trp His Val Glu Asp Ser Cys Val Lys Arg Phe Ala Ala Glu Asp Trp
        355                 360                 365

Val Arg Leu Gln Met Gly Ser Arg Glu Ala Trp Lys Arg
    370                 375                 380

<210>  8
<211>  1767
<212>  DNA
<213>  Homo sapiens

<400>  8
atggacctcg ccagaaaaga atttctgcgt ggaaatggct tagctgctgg gaaaatgaac      60

atcagtattg atttagacac aaactatgct gagctggttc taaatgtggg aagagtcact     120

cttggagaga acaatagaaa aaaaatgaag gattgtcaac tgagaaaaca gcagaatgaa     180

aatgtctcac gagctgtgtg tgctctgctg aattctggag ggggagtgat caaggctgaa     240

gttgagaata aaggctatag ttataaaaaa gatggaatag ggctagattt ggaaaattct     300

tttagtaaca tgctgccatt tgttcctaat ttcctggact tcatgcagaa tggtaactac     360

tttcacattt ttgtgaaatc atggagcttg gaaacctctg gtccgcagat tgccacgttg     420

agctccagtt tgtacaagag agatgtaacg tctgcaaaag tcatgaatgc ttctgctgca     480

ctggagttcc tcaaagacat ggaaaaaact ggagggagag catatttaag accagaattc     540

cctgcaaaaa gggcctgtgt tgatgtacaa gaagaaagta acatggaagc cttggctgct     600

gattttttta acagaacaga acttggttat aaagaaaaat tgacctttac tgaatccaca     660

cacgttgaaa taaaaaactt ctcgactgaa aagttgttac aacgaattac agagattctc     720

cctcaatatg tttctgcatt tgcaaatact gatggaggat atttattcgt tggtctaaat     780

gaagataaag aagtaattgg ctttaaagca gagaagagtt atcttactaa gttagaagaa     840

gtaacaaaaa attccattgg gaaactgcct gtgcatcact tctgtgtgga gaaggggacg     900

ataaattact tatgcaaatt ccttggagta tatgataaag gaaggctttg tggatatgtg     960

tatgcactca gagtggaacg cttctgctgt gcagtgtttg ctaaaaagcc tgattcctgg    1020

cacgtgaaag ataacagagt taagcagttg accgagaagg aatggatcca gttcatggtg    1080

gattcagaac cagtatgtga ggaactgccc tctccagcaa gtacatcatc acctgtctcc    1140

cagagttatc ctcttcgtga atatattaac ttcaaaattc agccactgag atatcacctt    1200

ccagggctat cagaaaagat aacttgtgct ccaaaaacct tctgcagaaa tctgttctca    1260

caacatgaag gacttaagca attaatatgt gaagaaatgg gctctgtcaa taagggctca    1320

ctgatcttct ctaggagctg gtctttggat ctgggcttgc aagagaacca caaagtcctc    1380

tgtgatgctc ttctgatttc ccaggacaag cctccagtcc tatacacctt ccacatggta    1440

caggatgagg agtttaaaga ctattctaca caaactgccc aaactttaaa acagaagctg    1500

gcaaaaattg gtggttacac taaaaaagtg tgtgtcatga caaagatctt ctacttgagc    1560

cctgaaggca agacaagctg ccagtatgat ttaaactcgc aagtaattta ccctgaatcc    1620

tactattgga caacagctca aacaatgaaa gacttggaaa aggccctttc aaatatctta    1680

cctaaggaga atcaaatctt tttgtttgtt tgtttgtttc gtttttgttt gtttgtttgt    1740

tggtttgttt gtttttcttt gagatga                                        1767

<210>  9
<211>  588
<212>  PRT
<213>  Homo sapiens

<400>  9

Met Asp Leu Ala Arg Lys Glu Phe Leu Arg Gly Asn Gly Leu Ala Ala
1               5                   10                  15

Gly Lys Met Asn Ile Ser Ile Asp Leu Asp Thr Asn Tyr Ala Glu Leu
            20                  25                  30

Val Leu Asn Val Gly Arg Val Thr Leu Gly Glu Asn Asn Arg Lys Lys
        35                  40                  45

Met Lys Asp Cys Gln Leu Arg Lys Gln Gln Asn Glu Asn Val Ser Arg
    50                  55                  60

Ala Val Cys Ala Leu Leu Asn Ser Gly Gly Gly Val Ile Lys Ala Glu
65                  70                  75                  80

Val Glu Asn Lys Gly Tyr Ser Tyr Lys Lys Asp Gly Ile Gly Leu Asp
                85                  90                  95

Leu Glu Asn Ser Phe Ser Asn Met Leu Pro Phe Val Pro Asn Phe Leu
            100                 105                 110

Asp Phe Met Gln Asn Gly Asn Tyr Phe His Ile Phe Val Lys Ser Trp
        115                 120                 125

Ser Leu Glu Thr Ser Gly Pro Gln Ile Ala Thr Leu Ser Ser Ser Leu
    130                 135                 140

Tyr Lys Arg Asp Val Thr Ser Ala Lys Val Met Asn Ala Ser Ala Ala
145                 150                 155                 160

Leu Glu Phe Leu Lys Asp Met Glu Lys Thr Gly Gly Arg Ala Tyr Leu
                165                 170                 175

Arg Pro Glu Phe Pro Ala Lys Arg Ala Cys Val Asp Val Gln Glu Glu
            180                 185                 190

Ser Asn Met Glu Ala Leu Ala Ala Asp Phe Phe Asn Arg Thr Glu Leu
        195                 200                 205

Gly Tyr Lys Glu Lys Leu Thr Phe Thr Glu Ser Thr His Val Glu Ile
    210                 215                 220

Lys Asn Phe Ser Thr Glu Lys Leu Leu Gln Arg Ile Thr Glu Ile Leu
225                 230                 235                 240

Pro Gln Tyr Val Ser Ala Phe Ala Asn Thr Asp Gly Gly Tyr Leu Phe
                245                 250                 255

Val Gly Leu Asn Glu Asp Lys Glu Val Ile Gly Phe Lys Ala Glu Lys
            260                 265                 270

Ser Tyr Leu Thr Lys Leu Glu Glu Val Thr Lys Asn Ser Ile Gly Lys

275                 280                 285

Leu Pro Val His His Phe Cys Val Glu Lys Gly Thr Ile Asn Tyr Leu
    290                 295                 300

Cys Lys Phe Leu Gly Val Tyr Asp Lys Gly Arg Leu Cys Gly Tyr Val
305                 310                 315                 320

Tyr Ala Leu Arg Val Glu Arg Phe Cys Cys Ala Val Phe Ala Lys Lys
                325                 330                 335

Pro Asp Ser Trp His Val Lys Asp Asn Arg Val Lys Gln Leu Thr Glu
            340                 345                 350

Lys Glu Trp Ile Gln Phe Met Val Asp Ser Glu Pro Val Cys Glu Glu
        355                 360                 365

Leu Pro Ser Pro Ala Ser Thr Ser Ser Pro Val Ser Gln Ser Tyr Pro
    370                 375                 380

Leu Arg Glu Tyr Ile Asn Phe Lys Ile Gln Pro Leu Arg Tyr His Leu
385                 390                 395                 400

Pro Gly Leu Ser Glu Lys Ile Thr Cys Ala Pro Lys Thr Phe Cys Arg
                405                 410                 415

Asn Leu Phe Ser Gln His Glu Gly Leu Lys Gln Leu Ile Cys Glu Glu
            420                 425                 430

Met Gly Ser Val Asn Lys Gly Ser Leu Ile Phe Ser Arg Ser Trp Ser
        435                 440                 445

Leu Asp Leu Gly Leu Gln Glu Asn His Lys Val Leu Cys Asp Ala Leu
    450                 455                 460

Leu Ile Ser Gln Asp Lys Pro Pro Val Leu Tyr Thr Phe His Met Val
465                 470                 475                 480

Gln Asp Glu Glu Phe Lys Asp Tyr Ser Thr Gln Thr Ala Gln Thr Leu
                485                 490                 495

Lys Gln Lys Leu Ala Lys Ile Gly Gly Tyr Thr Lys Lys Val Cys Val
            500                 505                 510

Met Thr Lys Ile Phe Tyr Leu Ser Pro Glu Gly Lys Thr Ser Cys Gln
        515                 520                 525

Tyr Asp Leu Asn Ser Gln Val Ile Tyr Pro Glu Ser Tyr Tyr Trp Thr
    530                 535                 540

Thr Ala Gln Thr Met Lys Asp Leu Glu Lys Ala Leu Ser Asn Ile Leu
545                 550                 555                 560

Pro Lys Glu Asn Gln Ile Phe Leu Phe Val Cys Leu Phe Arg Phe Cys
                565                 570                 575

Leu Phe Val Cys Trp Phe Val Cys Phe Phe Leu Arg
            580                 585

<210>  10
<211>  25
<212>  DNA
<213>  Artificial Sequence

<220>
<223>  sense murine Slfn2

<400>  10
ctcacctcag aaaacaggag aatgc                                          25

<210>  11
<211>  23
<212>  DNA
<213>  Artificial Sequence

<220>
<223>  anti-sense, murine Slfn2

<400>  11
cagaagtgag tgacaggcag ctg                                            23

<210>  12
<211>  26
<212>  DNA
<213>  Artificial Sequence

<220>
<223>  sense Slfn2 -1766

<400>  12
cggctagctt ggtggctcct agcgrr                                         26

<210>  13
<211>  25
<212>  DNA
<213>  Artificial Sequence

<220>
<223>  sense Slfn2 -1265

<400>  13
cggctagcgg aactgaactg gagct                                          25

<210>  14
<211>  30
<212>  DNA
<213>  Artificial Sequence

<220>
<223>  anti-sense Slfn 2 +198

<400>  14
ccctcgagcc atgtcagcag ttggtaaagc                                     30